(19) Europäisches Patentamt
European Patent Office
Office européen des brevets

(11) EP 3 351 117 A1

(12) # EUROPEAN PATENT APPLICATION
published in accordance with Art. 153(4) EPC

(43) Date of publication:
**25.07.2018 Bulletin 2018/30**

(21) Application number: **16846665.4**

(22) Date of filing: **16.09.2016**

(51) Int Cl.:
***A23L 33/105*** *(2016.01)* ***A23L 5/41*** *(2016.01)*
***A61K 8/97*** *(2017.01)* ***A61K 36/28*** *(2006.01)*
***A61Q 13/00*** *(2006.01)* ***C09B 67/00*** *(2006.01)*
***C11B 5/00*** *(2006.01)* ***C11B 9/00*** *(2006.01)*
***C11B 11/00*** *(2006.01)* ***C12N 5/04*** *(2006.01)*

(86) International application number:
**PCT/JP2016/077559**

(87) International publication number:
**WO 2017/047794 (23.03.2017 Gazette 2017/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **17.09.2015 JP 2015183988**

(71) Applicant: **San-Ei Gen F.F.I., INC.**
**Toyonaka-shi, Osaka 561-8588 (JP)**

(72) Inventors:
- **UCHIDA Koji**
**Toyonaka-shi**
**Osaka 561-8588 (JP)**
- **SAKATANI Keisuke**
**Toyonaka-shi**
**Osaka 561-8588 (JP)**

(74) Representative: **Vossius & Partner**
**Patentanwälte Rechtsanwälte mbB**
**Siebertstrasse 3**
**81675 München (DE)**

(54) **EXTRACT FROM SEEDS OF PLANT GENUS HELIANTHUS, AND METHOD FOR PRODUCING SAME**

(57) An object of the present invention is to provide a composition which has a function of imparting stability to natural colorants and flavoring compounds and in which the occurrence of degradation smell is suppressed. The present invention provides a *Helianthus* seed extract having a feature (A) as follows: (A) a content of 1-octen-3-one of an acid-sugar solution when stored under white fluorescent light of 10,000 lux at 30°C for 7 days is 0.1 ppb or less, wherein the acid-sugar solution contains the *Helianthus* seed extract in an amount of 0.0075 mass% in terms of a solid content and is adjusted to have 10° Brix by high fructose corn syrup and to have a pH being 3.0.

*Fig.1*

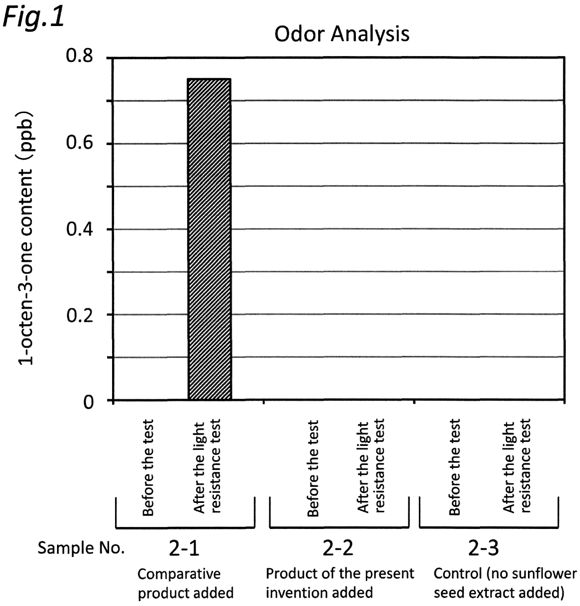




Printed by Jouve, 75001 PARIS (FR)

## Description

### Technical Field

The present application claims priority to Japanese Patent Application No. 2015-183988, filed with the Japan Patent Office, the entire contents of which are incorporated herein by reference.

The present invention relates to a *Helianthus* seed extract that has a function of imparting stability to natural colorants and flavoring compounds and in which the occurrence of degradation smell is remarkably suppressed.

### Background Art

1. Color Fading of Natural Colorants

Natural colorants fade rapidly due to their oxidation in air. L-ascorbic acid, alpha-tocophenol, rutin and the like are conventionally known as antioxidants. However, the effects of L-ascorbic acid are not sufficient, and moreover, alpha-tocophenol, rutin and the like are not easily dissolved in water and thus are not suitable for the purpose of stabilizing colorants in foods and beverages.

A sunflower seed extract containing chlorogenic acids and the like as its main components is reported as an antioxidant that can be used for adding stability to natural colorants (Patent Document 1). However, sunflower seed extract has strong odor derived from the components of the seed materials themselves, and its application as an antioxidant is very limited. Moreover, in addition to the strong odor derived from the components of the seed materials, the sunflower seed extract has a problem in that the offensive odor is further increased as time lapses, causing to generate degradation smell. Especially, the "smell caused by light degradation", which is generated due to light irradiation, is a serious problem. Even when there is no odor at the time of production, offensive odors are generated and intensified as the product is exposed to light during storage or the like.

Since the generation mechanism of the degradation smell in sunflower seed extract is yet to be clarified and the odor generation from precursor substances cannot be suppressed, sunflower seed extract has not been widely used as a stabilizer for natural colorants in the field of food and beverage and the like. Moreover, it is not conventionally known that sunflower seed extract exhibits extremely excellent effects of imparting stability to natural colorants to protect them against color fading and changing caused by exposure to heat.

Meanwhile, as a method for obtaining chlorogenic acid from sunflower seed extract in a high yield, Patent Document 2 suggests a technique in which chlorogenic acids are recovered from a solid generated by bringing into contact the extract solution of sunflower seeds and the aluminum salt. However, with the technique disclosed in Patent Document 2, it is impossible to reduce or eliminate the odor components of the sunflower seed extract and to suppress the odor caused by light degradation.

Also, Patent Document 3 suggests a technique for producing chlorogenic acid-containing compositions with reduced calorie by bringing into contact the chlorogenic acids and saccharides derived from oil extraction residues of sunflower seeds, yeasts and solvent. However, with the technique disclosed in Patent Document 3, the odor or fermentation smell peculiar to yeast remains, and moreover, it is impossible to reduce or eliminate the odor components of the sunflower seed extract and to suppress the smell caused by light degradation.

2. Degradation of Natural and Artificial Flavoring Compounds

Similar to natural colorants, natural and artificial flavoring compounds have a problem in that their flavor is lost or degradation smell is generated due to decomposition or structural change caused by heating or the passage of time. In particular, while citral, which is a lemon flavoring, has a lemon-like fresh flavor and aroma and thus is highly demanded in many fields, there is a problem in that the flavor is lost and degradation smell is generated due to the decomposition of citral.

As a conventional decomposition inhibitor of citral, the extract obtained by extracting fermented tea leaves with water, ethanol or their mixture is reported to have a function of suppressing the occurrence of degradation smell (Patent Document 4). However, it is admitted that its effect in suppressing the generation of the p-cresol, which is the main component causing the degradation smell of citral, is not sufficient.

### Citation List

#### Patent Documents

Patent Document 1: Japanese Patent No. 3247990
Patent Document 2: Japanese Patent Application Publication No. 2015-51932
Patent Document 3: Japanese Patent Application Publication No. 2015-91223
Patent Document 4: Japanese Patent No. 4185317

Summary of Invention

Technical Problem

The present invention was achieved in light of the aforementioned circumstances of the conventional techniques, and it is an object thereof to provide a composition which has a function of imparting stability to natural colorants and flavoring compounds and in which the occurrence of degradation smell is suppressed.

Solution to Problem

As a result of intensive study conducted to solve the aforementioned problems, the inventors of the present invention have developed a method for producing a *Helianthus* seed extract characterized by the following steps: (step 1) performing aqueous extraction or aqueous alcohol extraction on the *Helianthus* seeds; (step 2) recovering an extract solution by performing solid-liquid separation after the extraction; and (step 3) performing heat treatment on the extract solution at 60°C or higher under an acidic condition.
Testing the properties of the *Helianthus* seed extract obtained by the method described above, the inventors of the present invention discovered that it was a composition in which the degradation smell was remarkably reduced, and furthermore, not only the substances causing the degradation smell but also the precursors thereof are reduced, removed or inactivated.
In addition, by examining the light resistance in the visible light region and the heat resistance to evaluate the stability of natural colorants, for which purpose sunflower seed extract had not been used, it was found that this composition had extremely excellent light resistance and heat resistance. Moreover, it was found that the composition obtained by this production method could impart stability to natural colorants even with a small titer, compared with the sunflower seed extract obtained by a conventional method of Patent Document 1.
The inventors of the present invention also found that the *Helianthus* seed extract obtained by this method had a function of imparting stability to flavoring compounds. Especially, the inventors discovered that it could impart excellent heat resistance to citral, a lemon flavoring compound, and the generation of the p-cresol, the main component causing the degradation smell of citral, was remarkably suppressed.
The present invention specifically relates to aspects of the invention described below.

[1] A *Helianthus* seed extract having a feature (A) as follows:

(A) a content of 1-octen-3-one of an acid-sugar solution when stored under white fluorescent light of 10,000 lux at 30°C for 7 days is 0.1 ppb or less, wherein the acid-sugar solution contains the *Helianthus* seed extract in an amount of 0.0075 mass% in terms of a solid content and is adjusted to have 10° Brix by high fructose corn syrup and to have a pH being 3.0.

[2] The *Helianthus* seed extract according to aspect 1, further having a feature (B) as follows:

(B) when an acid-sugar solution that contains the *Helianthus* seed extract in an amount of 0.0075 mass% in terms of a solid content and is adjusted to have 10° Brix by high fructose corn syrup and to have a pH being 3.0 is prepared,

(b-1) a content of methional of the acid-sugar solution is 0.025 ppb or less; and
(b-2) a content of methional of the acid-sugar solution when stored under white fluorescent light of 10,000 lux at 30°C for 7 days is 0.03 ppb or less.

[3] The *Helianthus* seed extract according to aspect 1 or 2, further having a feature (C) as follows:

(C) a retention rate of anthocyanin pigments of an acid-sugar solution when stored in a dark place at 50°C for 10 days is 60% or more compared with before storage, wherein the acid-sugar solution contains purple sweet potato color at a content of 0.03 mass% when calculated in terms of color value ($E^{10\%}_{1cm}$) 80, contains the *Helianthus* seed extract in an amount of 0.0041 mass% in terms of a solid content and is adjusted to have 10° Brix by high fructose corn syrup and to have a pH being 3.0.

[4] The *Helianthus* seed extract according to any one of aspects 1 to 3, further having a feature (D) as follows:

(D) a retention rate of anthocyanin pigments of an acid-sugar solution when stored under white LED light of 10,000 lux at 10°C for 14 days is 60% or more compared with before storage, wherein the acid-sugar solution contains purple sweet potato color at a content of 0.03 mass% when calculated in terms of color value ($E^{10\%}_{1cm}$) 80, contains the *Helianthus* seed extract in an amount of 0.0041 mass% in terms of a solid content and is adjusted to have 10° Brix by high fructose corn syrup and to have a pH being 3.0.

[5] The *Helianthus* seed extract according to any one of aspects 1 to 4, further having a feature (E) as follows:

(E) a content of p-cresol of an acid-sugar solution when stored in a dark place at 50°C for 7 days is 20 ppb or less, wherein the acid-sugar solution contains citral in an amount of 0.01 mass%, contains the *Helianthus* seed extract in an amount of 0.0075 mass% in terms of a solid content and is adjusted to have 10° Brix by high fructose corn syrup and to have a pH being 3.0.

[6] The *Helianthus* seed extract according to any one of aspects 1 to 5, further having a feature (F) as follows:

(F) when an aqueous solution containing the *Helianthus* seed extract in an amount of 0.75 mass% in terms of a solid content is prepared using McIlvaine buffer of pH 3.0, a turbidity ($OD_{700}$ value) of the aqueous solution is 0.2 or less.

[7] The *Helianthus* seed extract according to aspect 6, having the features (A) to (F).
[8] A production method for a *Helianthus* seed extract, including:

(step 1) performing aqueous extraction or aqueous alcohol extraction on the *Helianthus* seeds;
(step 2) recovering an extract solution by performing solid-liquid separation after the extraction; and
(step 3) performing heat treatment on the extract solution at 60°C or higher under an acidic condition.

[9] The production method according to aspect 8,
wherein a hydrogen ion concentration of the extract solution after the aqueous extraction or the aqueous alcohol extraction is $3.16 \times 10^{-8}$ to $1 \times 10^{-6}$ mol/L, and the heat treatment recited in the step 3 is performed under a condition in which, when the hydrogen ion concentration of the extract solution after the aqueous extraction or the aqueous alcohol extraction is taken as 1, a hydrogen ion concentration ratio is set to $1\times10^{1}$ to $3.16\times10^{5}$.
[10] The production method according to aspect 8 or 9,
wherein the *Helianthus* seeds are seeds of plants belonging to *Helianthus annuus*, a seed shell content therein adjusted to 20% or less.
[11] A *Helianthus* seed extract obtained by the production method according to any one of aspects 8 to 10.
[12] A method for reducing degradation smell of the *Helianthus* seed extract produced, wherein the steps 1 to 3 according to any one of aspects 8 to 10 are performed in a production process of the *Helianthus* seed extract.
[13] An agent for preventing color fading of natural colorants, including the *Helianthus* seed extract according to any one of aspects 1 to 7 and 11.
[14] A pigment preparation including the *Helianthus* seed extract according to any one of aspects 1 to 7 and 11.
[15] An agent for suppressing degradation smell of flavoring compounds, including the *Helianthus* seed extract according to any one of aspects 1 to 7 and 11.
[16] A flavoring preparation including the *Helianthus* seed extract according to any one of aspects 1 to 7 and 11.
[17] A food, a beverage, a cosmetic product, a medicine, a quasi-drug, a personal hygiene product, or feed, including the *Helianthus* seed extract according to any one of aspects 1 to 7 and 11.
[18] A method for preventing color fading of natural colorants, including a step in which the *Helianthus* seed extract according to any one of aspects 1 to 7 and 11 is contained.
[19] A method for suppressing degradation smell of flavoring compounds, including a step in which the *Helianthus* seed extract according to any one of aspects 1 to 7 and 11 is contained.
[20] A production method for an agent for preventing color fading of natural colorants, an agent for suppressing degradation smell of flavoring compounds, a pigment preparation, a flavoring preparation, a food, a beverage, a cosmetic product, a medicine, a quasi-drug, a personal hygiene product, or feed, the method including a step in which the *Helianthus* seed extract obtained by the production method according to any one of aspects 8 to 10 is contained.

Advantageous Effects of the Invention

With the present invention, it is possible to provide a composition which has a function of imparting stability to natural colorants and flavoring compounds and in which the occurrence of degradation smell is suppressed. As a result, the *Helianthus* seed extract according to the present invention can be utilized in a wide spectrum of fields for purposes which could not be fulfilled by sunflower seed extract obtained by conventional techniques.

Brief Description of Drawings

FIG. 1 shows the 1-octen-3-one contents measured by GC/MS analysis in the stability test of the sunflower seed extract in Example 2.
FIG. 2 shows the methional contents measured by GC/MS analysis in the stability test of the sunflower seed extract in Example 2.
FIG. 3 shows the pigment retention rates measured in the light resistance test in the experiment on the function of imparting stability to natural colorants in Example 5.
FIG. 4 shows the pigment retention rates measured in the heat resistance test in the experiment on the function of imparting stability to natural colorants in Example 5.
FIG. 5 shows the color difference values (ΔE values) measured in the light resistance test in the experiment on the function of imparting stability to natural colorants in Example 6.
FIG. 6 shows the color difference values (ΔE values) measured in the heat resistance test in the experiment on the function of imparting stability to natural colorants in Example 6.
FIG. 7 shows the pigment retention rates measured in the test of light resistance against LED light irradiation in the experiment on the function of imparting stability to natural colorants in Example 7.
FIG. 8 shows the color difference values (ΔE values) measured in the test of light resistance against LED light irradiation in the experiment on the function of imparting stability to natural colorants in Example 7.
FIG. 9 shows the pigment retention rates measured in the light resistance test and the heat resistance test in the experiment on the function of imparting stability to natural colorants in carbonated drinks in Example 8.
FIG. 10 shows the color difference values (ΔE values) measured in the light resistance test and the heat resistance test in the experiment on the function of imparting stability to natural colorants in carbonated drinks in Example 8.
FIG. 11 shows the pigment retention rates measured in the light resistance test and the heat resistance test in the experiment on the function of imparting stability to natural colorants in alcoholic drinks in Example 9.
FIG. 12 shows the color difference values (ΔE values) measured in the light resistance test and the heat resistance test in the experiment on the function of imparting stability to natural colorants in alcoholic drinks in Example 9.
FIG. 13 shows the pigment retention rates measured in the light resistance test and the heat resistance test in the experiment on the function of imparting stability to natural colorants in Example 10.
FIG. 14 shows the color difference values (ΔE values) measured in the light resistance test and the heat resistance test in the experiment on the function of imparting stability to natural colorants in Example 10.
FIG. 15 shows the p-cresol contents measured by GC/MS analysis in the experiment on the function of imparting stability to flavoring compounds in Example 11.
FIG. 16 shows the p-methylacetophenone contents measured by GC/MS analysis in the experiment on the function of imparting stability to flavoring compounds in Example 11.
FIG. 17 shows the result of the sensory evaluation of the odor characteristics conducted in the light resistance test in the stability test of the sunflower seed extract in Example 14.
FIG. 18 shows the pigment retention rates measured in the light resistance test in the experiment on the function of imparting stability to natural colorants in Example 15.

Description of Embodiments

Hereinafter, embodiments of the present invention will be described in detail.

[Description of Light and Temperature Conditions Employed in Stability Test]

The light intensity of "10,000 lux" employed in the stability test for evaluating light resistance in the present invention is 5 to 10 times higher than the illuminance of the fluorescent or LED light used in an ordinary retail store and the like.
Meanwhile, the temperature of "50°C" employed in the stability test for evaluating heat resistance in the present invention is a temperature at which chemical reaction proceeds about 8 times faster compared with storage at a general room temperature of 20°C.

1. *Helianthus* Seed Extract

The present invention relates to a *Helianthus* seed extract that has a function of imparting stability to natural colorants and flavoring compounds and in which the occurrence of degradation smell is remarkably suppressed.
As a result, the *Helianthus* seed extract according to the present invention can be utilized in a wide spectrum of fields for purposes which could not be fulfilled by conventional sunflower seed extracts. The *Helianthus* seed extract according to the present invention does not preclude the inclusion of features other than those described below as long as they do not interfere with the effects realized by the technical characteristics of the present invention. Also, except for its essential technical features, the technical scope of the present invention is not limited to a mode in which all of the following features are included.

[Characteristics Related to Quality of *Helianthus* Seed Extract]

The *Helianthus* seed extract according to the present invention is an extract that exhibits excellent characteristics due to its compositional features, and particularly, is a composition in which the occurrence of degradation smell with the passage of time, which could not be overcome by conventional techniques, is greatly reduced.

Ability to Suppress Degradation Smell

The *Helianthus* seed extract according to the present invention is a composition in which the occurrence of degradation smell is greatly reduced compared with the sunflower seed extract obtained by a conventional production method or crude extraction only. Here, the term "degradation smell" refers to the smell caused by light degradation or thermal degradation, which is generated as the components of the composition change with the passage of time. The occurrence of the smell caused by light degradation is particularly problematic in sunflower seed extract.
The term "smell caused by light degradation" refers to a phenomenon in which offensive odors are generated as the sunflower seed extract is exposed to light irradiation and are enhanced with the passage of time. Moreover, in sunflower seed extract, even when there is no smell at the time of production, there is a tendency that offensive odors are generated and enhanced with the passage of time. This phenomenon is thought to occur as the light causes the decomposition or reaction of the components contained in the sunflower seed extract, resulting in the generation of odor components.
Meanwhile, the term "smell caused by thermal degradation" refers to a phenomenon in which the decomposition or reaction of the components contained in the sunflower seed extract is accelerated by exposure to high temperature, causing offensive odors to be generated and enhanced.
The *Helianthus* seed extract according to the present invention is a composition in which the occurrence of the smell caused by thermal degradation is suppressed, and in addition, particularly, the occurrence of the smell caused by light degradation is greatly reduced.
The ability to suppress the degradation smell is achieved by the production method according to the present invention as described below. That is, it is realized by removing or inactivating not only the substances causing the degradation smell but also the precursors thereof.
In contrast, when purification using a general adsorption resin or the like is employed, it is not always possible to obtain a composition in which the occurrence of degradation smell with the passage of time can be reduced, even if there may be some effects in reducing offensive odors or degradation smells.
The ability to suppress the degradation smell of the *Helianthus* seed extract according to the present invention can be evaluated and specified using the content of 1-octen-3-one contained in the extract as an index.
Here, "1-octen-3-one" is a compound having a mushroom-like odor. This compound is the main odor component generated and increased with the passage of time in the *Helianthus* seed extract obtained by a conventional technique or crude extraction. The generation of this odor component is increased especially by light irradiation.
In the present invention, using as an index the generation of 1-octen-3-one, which is the main component of the degradation smell, makes it possible to give a relative evaluation of the generation of other components causing the degradation smell. Accordingly, in the present invention, by measuring the generation of 1-octen-3-one, it becomes possible to judge whether the extract is a composition in which the occurrence of degradation smell, particularly, the smell caused by light degradation, is suppressed.
Concretely, in the *Helianthus* seed extract according to the present invention, the 1-octen-3-one content is lower than a predetermined level in a case where it is measured by the method as described in (A) below.
That is, the *Helianthus* seed extract according to the present invention has a feature as follows:

(A) A content of 1-octen-3-one of an acid-sugar solution when stored under white fluorescent light of 10,000 lux at 30°C for 7 days is 0.1 ppb or less, preferably 0.05 ppb or less, and more preferably 0.005 ppb or less, wherein the acid-sugar solution contains the *Helianthus* seed extract in an amount of 0.0075 mass% in terms of a solid content and is adjusted to have 10° Brix by high fructose corn syrup and to have a pH being 3.0.

These values of the index can be achieved only with a component composition in which the components securing the function of imparting stability to the natural colorant or flavoring compound are maintained, and furthermore, substances causing degradation smell are removed from the composition.

Meanwhile, it is not preferable if the value exceeds 0.1 ppb, because the smell exceeds the threshold of human perception and can be confirmed even by sensory evaluation.

Further, in addition to the evaluation of the 1-octen-3-one content as described above, it is preferable to evaluate and specify the ability to suppress the degradation smell of the *Helianthus* seed extract according to the present invention by using the content of methional contained in the composition as an index.

Here, "methional" is a compound having a potato-like odor. This compound is one of the odor components that are generated and increased with the passage of time in sunflower seed extract obtained by a conventional technique or crude extraction. This odor component is generated and increased especially by light irradiation.

Accordingly, in the present invention, in addition to the evaluation of the 1-octen-3-one content as described above, it is preferable to judge by measuring the generation of methional whether the extract is a composition in which the occurrence of the degradation smell, particularly, the smell caused by light degradation, is suppressed.

Concretely, in the *Helianthus* seed extract according to the present invention, the methional content is lower than a predetermined level in a case where it is measured by the method as described in (B) and (b-2) below.

That is, the *Helianthus* seed extract according to the present invention has a feature as follows:

(B) When an acid-sugar solution that contains the *Helianthus* seed extract in an amount of 0.0075 mass% in terms of a solid content and is adjusted to have 10° Brix by high fructose corn syrup and to have a pH being 3.0 is prepared,

(b-2) a content of methional of the acid-sugar solution when stored under white fluorescent light of 10,000 lux at 30°C for 7 days is 0.03 ppb or less, preferably 0.02 ppb or less, more preferably 0.01 ppb or less, even more preferably 0.008 ppb or less, and even more preferably 0.007 ppb or less.

These values of the index can be achieved only with a component composition in which the components securing the function of imparting stability to natural colorants or flavoring compounds are maintained, and furthermore, substances causing degradation smell are removed from the composition.

<u>Function to Suppress Offensive Odor Derived from Raw Materials</u>

The *Helianthus* seed extract according to the present invention is a compound in which the offensive odor derived from the raw materials is greatly reduced compared with the *Helianthus* seed extract obtained by a conventional production method or by crude extraction only.

This ability to suppress offensive odor is achieved by the production method according to the present invention as described below. That is, it is realized by removing or inactivating the substances causing the offensive odor.

In the *Helianthus* seed extract according to the present invention, it is possible to evaluate and specify the characteristics in which the offensive odor derived from the raw materials (that is, the offensive odor inherent in the extract immediately after production) is suppressed by using the methional content as an index.

In the present invention, using as an index the content of methional, which is the main odor component derived from the raw material, makes it possible to give a relative evaluation of the content of other odor components. Accordingly, in the present invention, by measuring the methional content, it becomes possible to judge whether the extract is a composition in which the offensive odor derived from the raw materials is suppressed.

Concretely, in the *Helianthus* seed extract according to the present invention, the methional content becomes lower than a predetermined level in a case where it is measured by the method as described in (B) and (b-1) below.

That is, the *Helianthus* seed extract according to the present invention has a feature as follows:

(B) When an acid-sugar solution that contains the *Helianthus* seed extract in an amount of 0.0075 mass% in terms of a solid content and is adjusted to have 10° Brix by high fructose corn syrup and to have a pH being 3.0 is prepared,

(b-1) a content of methional of the acid-sugar solution is 0.025 ppb or less, preferably 0.02 ppb or less, more preferably 0.01 ppb or less, even more preferably 0.008 ppb or less, and even more preferably 0.006 ppb or less.

These values of the index can be achieved only with a component composition in which the components securing the function of imparting stability to natural colorants or flavoring compounds are maintained, and furthermore, substances causing offensive odor are removed from the composition.

Function to Suppress Turbidity

The *Helianthus* seed extract according to the present invention is a compound in which the occurrence of turbidity is greatly reduced compared with the sunflower seed extract obtained by a conventional production method or by crude extraction only. That is, the *Helianthus* seed extract according to the present invention is expected to serve as a composition which can be beneficially used in new applications, including beverages, acidic foods, acidic beverages, and the like, for which the sunflower seed extract obtained by conventional production methods could hardly be used.
The function to suppress turbidity performed by the *Helianthus* seed extract according to the present invention is exhibited sufficiently in a neutral solution, and in particular, this function is exhibited remarkably in an acidic solution. In contrast, the occurrence of turbidity in an acidic solution is severe when the sunflower seed extract obtained by a conventional production method or by crude extraction only is employed.
Here, an acidic solution indicates a solution having a pH of less than 7, and it preferably has a pH of 6 or less, and more preferably 5 or less. The lower limit is not particularly limited, but can be a pH of 1 or more, and preferably 2 or more, for example.
The characteristics of the *Helianthus* seed extract according to the present invention in which the occurrence of turbidity is suppressed can be evaluated and specified using the turbidity ($OD_{700}$ value) as an index.
Concretely, in the *Helianthus* seed extract according to the present invention, the turbidity ($OD_{700}$ value) is lower than a predetermined level in a case where it is measured by the method as described in (F) below.
That is, the *Helianthus* seed extract according to the present invention has a feature as follows:

(F) When an aqueous solution containing the *Helianthus* seed extract in an amount of 0.75 mass% in terms of a solid content is prepared using McIlvaine buffer of pH 3.0, a turbidity ($OD_{700}$ value) of the aqueous solution is 0.2 or less, preferably 0.18 or less, more preferably 0.16 or less, even more preferably 0.15 or less, and even more preferably 0.14 or less.

Meanwhile, when the value is higher than the predetermined level, especially when the turbidity ($OD_{700}$ value) exceeds 0.2, the turbidity becomes outside the allowable range in terms of quality and thus is not preferable.
The term "McIlvaine buffer" as used herein refers to a buffer solution prepared by using citric acid and phosphate ($Na_2HPO_4$) and is also known as the citric acid buffer.

[Function of Imparting Stability to Natural Colorants]

The *Helianthus* seed extract according to the present invention is a composition that has a function of imparting stability to natural colorants due to its compositional characteristics.
Here, the term "the function of imparting stability to natural colorants" as used herein refers to a function to stabilize the compound structure of the natural colorant, thereby suppressing its decomposition or structural change, and to prevent the color from fading or changing.
Specifically, when a composition containing the *Helianthus* seed extract according to the present invention and a natural colorant is prepared, functions such as the improvement of the pigment retention rate and the prevention of color change are exhibited.
For example, it is possible to prepare a colorant composition in which the characteristics of its color are hardly lost even after heat treatment (e.g. production, processing, or cooking) or the passage of time (e.g. storage).
The *Helianthus* seed extract according to the present invention is a composition that has a function of imparting extensive and long-term stability to natural colorants in terms of both light resistance and heat resistance.
Here, the function of imparting light resistance exhibited by the *Helianthus* seed extract according to the present invention refers to a function to maintain the stability of natural colorants even after a long-term irradiation in the wavelength range from ultraviolet to the entire visible region.
Meanwhile, the function of imparting heat resistance exhibited by the *Helianthus* seed extract according to the present invention refers to a function to maintain the stability of natural colorants even after a long-term storage under a high temperature of about 50°C. The inventors of the present invention are the first ones to reveal that a *Helianthus* seed extract has a function of imparting heat resistance to natural colorants, and this fact has not been known in the art.
Examples of natural colorants for which the *Helianthus* seed extract according to the present invention can preferably exhibit the function of imparting stability are anthocyanin pigments and gardenia pigments. That is, the *Helianthus* seed extract according to the present invention is a compound that is preferably involved in the stabilization of the molecular structure of anthocyanin pigments and gardenia pigments. Particularly, among anthocyanin pigments, the composition is preferably involved in the stabilization of the structure of acylated anthocyanins.

Anthocyanin Pigments

Examples of colorants derived from natural materials for which the *Helianthus* seed extract according to the present invention can preferably exhibit the function of imparting stability are colorant compositions containing anthocyanin pigments as their pigment components. Examples thereof are red radish color, red cabbage color, purple sweet potato color, grape juice color, grape skin color, colored potato color, purple corn color, *Clitoria ternatea* color, black soybean color, elderberry color, cranberry color, gooseberry color, salmonberry color, strawberry color, cherry color, dark sweet cherry color, thimbleberry color, European dewberry color, hibiscus color, black huckleberry color, black currant color, black berry color, red currant color, loganberry color, plum color, blueberry color, boysenberry color, whortleberry color, mulberry color, morello cherry color, raspberry color, perilla color, red rice color, and purple carrot color.
More preferable examples are red radish color, red cabbage color, purple sweet potato color, and purple carrot color, which are colorant compositions containing acylated anthocyanins as their main pigment component.
The term "anthocyanin pigment" as used herein refers to a colorant composition containing anthocyanins as its main pigment component. In the present invention, colorant compositions containing pigments other than anthocyanins, other compounds or the like are also acceptable as long as the function of imparting stability to natural colorants performed by the *Helianthus* seed extract according to the present invention is secured.
Here, specific examples of the forms of the anthocyanin pigments are such forms of colorant compositions as pigment preparations and pigment extracts, and moreover, colorant compositions such as fruit juice and vegetable juice are also included herein.
The term "anthocyanin" as used herein refers to a pigment compound which exhibits colors ranging from red, purple to blue and which is a glycoside in which a sugar chain or chains (e.g. glucose, galactose, and rhamnose) are bound to anthocyanidin. In addition to the sugar chain, an organic acid or acids (e.g. sinapic acid, caffeic acid, succinic acid, and malonic acid) can also be bound to form an acylated anthocyanin.
An anthocyanidin consists of three ring structures, which are the A-ring, the B-ring and the C-ring, and is classified mainly into six anthocyanidins (pelargonidin, cyanidin, delphinidin, peonidin, petunidin and malvidin) based on the number of the hydroxyl groups (-OH) and the methoxy groups ($-OCH_3$) attached to the B-ring. The blueness tends to increase as the number of the hydroxyl groups increase: pelargonidin, which has one hydroxyl group, exhibits reddish orange color; cyanidin, which has two hydroxyl groups, exhibits reddish purple to purple color; and delphinidin, which has three hydroxyl groups, exhibits bluish purple color. The color becomes reddish when the hydroxyl groups in the B-ring are replaced with the methoxy groups.

Gardenia Pigments

Examples of colorants derived from natural materials for which the *Helianthus* seed extract according to the present invention can preferably exhibit the function of imparting stability are colorant compositions containing gardenia pigments as their pigment components.
The term "gardenia pigment" as used herein refers to a colorant composition containing the pigment component obtained from the fruit of *Gardenia augusta* or *Gardenia jasminoides* as its main component. Specific examples thereof are gardenia red pigment, gardenia blue pigment and gardenia yellow pigment.
Here the gardenia red pigment is explained generally as a pigment obtained by adding beta-glucosidases to a mixture of ester hydrolysates of iridoid glycosides obtained from the gardenia fruit and protein decomposition products. The gardenia red pigment as used herein is not limited by this definition and includes a wide range of colorant compositions containing pigment compounds generated by the reaction between the aglycones of iridoid compounds derived from the gardenia fruit having a carboxyl group at position 4 of the iridoid skeleton and amino group-containing compositions.
The gardenia blue pigment is explained generally as a pigment obtained by adding beta-glucosidases to a mixture of iridoid glycosides obtained from the gardenia fruit and protein decomposition products. The gardenia blue pigment as used herein is not limited by this definition and includes a wide range of colorant compositions containing pigment compounds generated by the reaction between the aglycones of iridoid compounds derived from the gardenia fruit having a methyl ester group at position 4 of the iridoid skeleton and amino group-containing compositions.
The gardenia yellow pigment is explained generally as a pigment having crocin and crocetin, which are carotenoid compounds obtained from the gardenia fruit, as its main components. The gardenia yellow pigment as used herein includes a wide range of colorant compositions containing such pigment compounds.
In the present invention, colorant compositions into which pigments other than gardenia pigments, other compounds or the like are mixed are also permissible as long as the function of imparting stability to natural colorants performed by the *Helianthus* seed extract according to the present invention is secured.

Here, specific examples of the forms of the gardenia pigments are such forms of colorant compositions as pigment preparations and pigment extracts.

Examples of colorant compositions derived from natural materials for which the *Helianthus* seed extract according to the present invention can preferably exhibit the function of imparting stability are gardenia red pigments, gardenia blue pigments and gardenia yellow pigments. More preferable examples are gardenia red pigments and gardenia blue pigments. Even more preferable examples are gardenia red pigments.

Index of the Function of Imparting Stability

The function of imparting stability to natural colorants performed by the *Helianthus* seed extract according to the present invention can be evaluated and specified by using as indices the pigment retention rate and the measured values regarding color change obtained after conducting a stability test on the heat resistance of the anthocyanin pigment derived from purple sweet potatoes.

Here, the anthocyanin pigment derived from purple sweet potatoes is employed as the evaluation standard because the harvest time is almost the same and the composition is relatively uniform for each variety.

Function of Imparting Heat Resistance

Concretely, in the *Helianthus* seed extract according to the present invention, the pigment retention rate after a heat resistance test is higher than a predetermined level in a case where it is measured by the method as described in (C) (c-1) below.

That is, the *Helianthus* seed extract according to the present invention has a feature as follows:

(C) (c-1) A retention rate of anthocyanin pigments of an acid-sugar solution when stored in a dark place at 50°C for 10 days is 60% or more, preferably 70% or more, more preferably 75% or more, even more preferably 80% or more, even more preferably 85% or more, and even more preferably 90% or more compared with before storage, wherein the acid-sugar solution contains purple sweet potato color at a content of 0.03 mass% when calculated in terms of color value ($E^{10\%}_{1cm}$) 80, contains the *Helianthus* seed extract in an amount of 0.0041 mass% in terms of a solid content and is adjusted to have 10° Brix by high fructose corn syrup and to have a pH being 3.0. Meanwhile, it is not preferable if the value is lower than the predetermined level, because the function of imparting stability is insufficient.

In the *Helianthus* seed extract according to the present invention, it is preferable that the difference of the pigment retention rate between presence and absence of the *Helianthus* seed extract is higher than a predetermined level as described in (C) (c-2) below.

That is, the *Helianthus* seed extract according to the present invention has a feature as follows:

(C) (c-2) Following Formula (1) is satisfied when an acid solution is stored in a dark place at 50°C for 10 days, wherein the acid-sugar solution contains purple sweet potato color at a content of 0.03 mass% when calculated in terms of color value ($E^{10\%}_{1cm}$) 80, contains the *Helianthus* seed extract in an amount of 0.0041 mass% in terms of a solid content and is adjusted to have 10° Brix by high fructose corn syrup and to have a pH being 3.0:

$$\text{Formula (1):} \quad X_1 - X_0 \geq 10$$

where $X_1$ is the pigment retention rate (%) after the storage of the acid-sugar solution containing the *Helianthus* seed extract, and $X_0$ is the pigment retention rate (%) after the storage of the acid-sugar solution that does not contain the *Helianthus* seed extract.

It is preferable that an example of the value on the right side of Formula (1) is 10, and it is preferably 13, more preferably 15, even more preferably 20, even more preferably 25, and even more preferably 30.

In the *Helianthus* seed extract according to the present invention, it is preferable that the color difference (ΔE value) (color change) between before and after the heat resistance test is lower than a predetermined level in a case where it is measured by the method as described in (C) (c-3) below.

That is, the *Helianthus* seed extract according to the present invention has a feature as follows:

(C) (c-3) When an acid-sugar solution is stored in a dark place at 50°C for 10 days, a color difference (ΔE value) between the acid-sugar solutions before and after the storage is 8 or less, preferably 6 or less, more preferably 5 or less, even more preferably 4 or less, even more preferably 3.5 or less, and even more preferably 3 or less, wherein the acid-sugar solution contains purple sweet potato color at a content of 0.03 mass% when calculated in terms of color value ($E^{10\%}_{1cm}$) 80, contains the *Helianthus* seed extract in an amount of 0.0041 mass% in terms of a solid content and is adjusted to have 10° Brix by high fructose corn syrup and to have a pH being 3.0. Meanwhile, it is not preferable if the value is higher than the predetermined level, because the function of imparting stability becomes insufficient.

Function of Imparting Light Resistance

The function of imparting stability to natural colorants performed by the *Helianthus* seed extract according to the present invention can also be evaluated and specified by using as indices the pigment retention rate and the measured values regarding color change obtained after a stability test on light resistance.

In the *Helianthus* seed extract according to the present invention, it is preferable that the pigment retention rate after the light resistance test is higher than a predetermined level in a case where it is measured by the method as described in (D) (d-1) below.

That is, the *Helianthus* seed extract according to the present invention has a feature as follows:

(D) (d-1) A retention rate of anthocyanin pigments of an acid-sugar solution when stored under white fluorescent light of 10,000 lux at 30°C for 10 days is 50% or more, preferably 60% or more, more preferably 70% or more, even more preferably 75% or more, even more preferably 80% or more, and even more preferably 85% or more compared with before storage, wherein the acid-sugar solution contains purple sweet potato color at a content of 0.03 mass% when calculated in terms of color value ($E^{10\%}_{1cm}$) 80, contains the *Helianthus* seed extract in an amount of 0.0041 mass% in terms of a solid content and is adjusted to have 10° Brix by high fructose corn syrup and to have a pH being 3.0. Meanwhile, it is not preferable if the value is lower than the predetermined level, because the function of imparting stability becomes insufficient.

In the *Helianthus* seed extract according to the present invention, it is preferable that the difference of the pigment retention rate between presence and absence of the *Helianthus* seed extract is higher than a predetermined level as described in (D) (d-2) below.

That is, the *Helianthus* seed extract according to the present invention has a feature as follows:

(D) (d-2) Following Formula (2) is satisfied when an acid-sugar solution is stored under white fluorescent light of 10,000 lux at 30°C for 10 days, wherein the acid-sugar solution contains purple sweet potato color at a content of 0.03 mass% when calculated in terms of color value ($E^{10\%}_{1cm}$) 80, contains the *Helianthus* seed extract in an amount of 0.0041 mass% in terms of a solid content and is adjusted to have 10° Brix by high fructose corn syrup and to have a pH being 3.0:

$$\text{Formula (2):} \quad Y_1 - Y_0 \geq 10$$

where $Y_1$ is the pigment retention rate (%) after the storage of the acid-sugar solution containing the *Helianthus* seed extract, and Yo is the pigment retention rate (%) after the storage of the acid-sugar solution that does not contain the *Helianthus* seed extract.

It is preferable that an example of the value on the right side of Formula (2) is 10, and it is preferably 15, more preferably 20, even more preferably 25, even more preferably 30, even more preferably 35 and even more preferably 40.

In the *Helianthus* seed extract according to the present invention, it is preferable that the color difference (ΔE value) (color change) between before and after the light resistance test is lower than a predetermined level in a case where it is measured by the method as described in (D) (d-3) below.

That is, the *Helianthus* seed extract according to the present invention has a feature as follows:

(D) (d-3) When an acid-sugar solution is stored under white fluorescent light of 10,000 lux at 30°C for 10 days, a color difference (ΔE value) between the acid-sugar solutions before and after the storage is 10 or less, preferably 8 or less, more preferably 6 or less, even more preferably 5 or less, even more preferably 4 or less, and even more preferably 3 or less, wherein the acid-sugar solution contains purple sweet potato color at a content of 0.03 mass% when calculated in terms of color value ($E^{10\%}_{1cm}$) 80, contains the *Helianthus* seed extract in an amount of 0.0041 mass% in terms of a solid content and is adjusted to have 10° Brix by high fructose corn syrup and to have a pH being 3.0. Meanwhile, it is not preferable if the value is higher than the predetermined level, because the function of imparting stability becomes insufficient.

Function of Imparting Stability against White LED Light Irradiation

The *Helianthus* seed extract according to the present invention is a compound that enables natural colorants to have sufficient light resistance and to maintain colors considerably even under the white LED light irradiation, which has strong degradation effects on natural colorants.

Here, the term "LED" refers to lighting equipment using a light-emitting diode, which is characterized by its low electric energy consumption and long life, and an example of an LED used for lighting equipment includes a white LED. However, compared with the white fluorescent light of the same illuminance, the white LED light has stronger degradation effects on natural colorants due to its wavelength characteristics.

The function of imparting stability to natural colorants performed by the *Helianthus* seed extract according to the present invention can be evaluated and specified by using as indices the pigment retention rate and the measured values regarding color change obtained after conducting a stability test under the irradiation of white LED light.

Concretely, it is preferable that, in the *Helianthus* seed extract according to the present invention, the pigment retention rate after a light resistance test is higher than a predetermined level in a case where it is measured by the method as described in (D) (d-4) below.

That is, the *Helianthus* seed extract according to the present invention has a feature as follows:

(D) (d-4) A retention rate of anthocyanin pigments of an acid-sugar solution when stored under white LED light of 10,000 lux at 10°C for 14 days is 60% or more, preferably 65% or more, and more preferably 70% or more compared with before storage, wherein the acid-sugar solution contains purple sweet potato color at a content of 0.03 mass% when calculated in terms of color value ($E^{10\%}_{1cm}$) 80, contains the *Helianthus* seed extract in an amount of 0.0041 mass% in terms of a solid content and is adjusted to have 10° Brix by high fructose corn syrup and to have a pH being 3.0. Meanwhile, it is not preferable if the value is lower than the predetermined level, because the function of imparting stability becomes insufficient.

In the *Helianthus* seed extract according to the present invention, it is preferable that the difference of the pigment retention rate between presence and absence of the *Helianthus* seed extract is higher than a predetermined level as described in (D) (d-5) below.

That is, the *Helianthus* seed extract according to the present invention has a feature as follows:

(D) (d-5) Following Formula (3) is satisfied when an acid-sugar solution is stored under white LED light of 10,000 lux at 10°C for 14 days, wherein the acid-sugar solution contains purple sweet potato color at a content of 0.03 mass% when calculated in terms of color value ($E^{10\%}_{1cm}$) 80, contains the *Helianthus* seed extract in an amount of 0.0041 mass% in terms of a solid content and is adjusted to have 10° Brix by high fructose corn syrup and to have a pH being 3.0:

$$\text{Formula (3):} \quad Z_1 - Z_0 \geq 10$$

where $Z_1$ is the pigment retention rate (%) after the storage of the acid-sugar solution containing the *Helianthus* seed extract, and $Z_0$ is the pigment retention rate (%) after the storage of the acid-sugar solution that does not contain the *Helianthus* seed extract.

It is preferable that an example of the value on the right side of Formula (3) is 10, and it is preferably 13, more preferably 15, and even more preferably 17.

In the *Helianthus* seed extract according to the present invention, it is preferable that the color difference ($\Delta E$ value) (color change) between before and after the light resistance test is lower than a predetermined level in a case where it is measured by the method as described in (D) (d-6) below.

That is, the *Helianthus* seed extract according to the present invention has a feature as follows:

(D) (d-6) When an acid-sugar solution is stored under white LED light of 10,000 lux at 10°C for 14 days, a color difference ($\Delta E$ value) between the acid-sugar solutions before and after the storage is 10 or less, preferably 9 or less, more preferably 8 or less, and even more preferably 7 or less, wherein the acid-sugar solution contains purple sweet potato color at a content of 0.03 mass% when calculated in terms of color value ($E^{10\%}_{1cm}$) 80, contains the *Helianthus* seed extract in an amount of 0.0041 mass% in terms of a solid content and is adjusted to have 10° Brix by high fructose corn syrup and to have a pH being 3.0. Meanwhile, it is not preferable if the value is higher than the predetermined level, because the function of imparting stability becomes insufficient.

Terms for Colorants

The term "purple sweet potato color" as used herein refers to an anthocyanin pigment composition obtained from the extract of the sweet potato (*Ipomoea batatas*). It is a colorant composition which contains cyanidin acyl glucosides and peonidin acyl glucosides as its main components and whose maximum absorption wavelength in citric acid buffer (pH 3.0) is 515-535 nm.
The term "pigment retention rate" as used herein refers to the value calculated by Formula (4) below based on the absorbance at the maximum absorption wavelength of each colorant measured before and after the stability test.

[Numerical Formula 1]

$$\text{Pigment retention rate (\%)} = \frac{\text{Absorbance at the maximum absorption wavelength after the stability test}}{\text{Absorbance at the maximum absorption wavelength before the stability test}} \times 100 \quad \cdots \text{Formula (4)}$$

The term "maximum absorption wavelength" ($\lambda$max) of a pigment as used herein refers to a light wavelength (nm) at which the absorption in the visible light region is maximum. The term "absorbance" as used herein refers to a value indicating the degree of light absorption by a substance. For example, the absorbance ($A_\lambda$) at the maximum absorption wavelength ($\lambda$max) can be calculated by Formula (5) below. In this formula, A represents the absorbance, $\lambda$ represents the maximum absorption wavelength, $A_\lambda$ represents the absorbance at the maximum absorption wavelength, I represents the incident light intensity, and $I_0$ represents the transmitted light intensity.

[Numerical Formula 2]

$$A_\lambda = -\log_{10}(I / I_0) \quad \cdots \text{Formula (5)}$$

The term "color value" as used herein refers to the "color value $E^{10\%}{}_{1cm}$", and "color value $E^{10\%}{}_{1cm}$" is a value calculated by measuring the absorbance (A) of a solution containing 10 mass% of a colorant composition at the maximum absorption wavelength ($\lambda$max) in the visible light region by using a measurement cell with the optical path length of 1 cm. This value is also expressed as "color value (10%E)". In detail, the value can be calculated following the method described in the Eighth Edition of the Specifications and Standards for Food Additives (published by the Ministry of Health, Labour and Welfare of Japan).
The term "in terms of color value" as used herein means to calculate the content of the pigment (colorant composition) as a content for a certain unit of color value. For example, "in terms of color value 60" means to convert the content of the pigment (colorant composition) to a content at color value 60.
The term "Hunter Lab colorimetric system (Lab system)" as used herein refers to a colorimetric system forming a color solid composed of an orthogonal coordinate system consisting of the a axis and the b axis representing chromaticity and the L axis vertical to these axes. Here, the "L value" is a numerical value expressing brightness. The color is white when L = 100 and is black when L = 0. The "a value" is a numerical value indicating redness to greenness of the color. The redness becomes stronger as the a value increases in the positive direction, and the greenness becomes stronger as the a value increases in the negative direction. The "b value" is a numerical value indicating yellowness to blueness of the color. The yellowness becomes stronger as the b value increases in the positive direction, and the blueness becomes stronger as the b value increases in the negative direction.
The term "color difference ($\Delta$E)" as used herein refers to the value calculated by Formula (6) below to express the distance of two colors. The expressions "L", "a" and "b" indicate those in the Hunter Lab colorimetric system.

[Numerical Formula 3]

$$\Delta E = \sqrt{(L_1 - L_2)^2 + (a_1 - a_2)^2 + (b_1 - b_2)^2} \quad \cdots \text{Formula (6)}$$

The "HUE value" as used herein refers to a value indicating a hue expressed by a symbol and a numerical value. Specifically, it is a value indicating a hue obtained by converting the angle formed by the straight line between the origin and the plot (a value, b value) in the orthogonal coordinate system of the a axis and the b axis in the Hunter Lab colorimetric system to the hue expression in the Munsell hue circle.

[Function of Imparting Stability to Flavoring Compounds]

The *Helianthus* seed extract according to the present invention is a composition that has a function of imparting stability to flavoring compounds due to its compositional characteristics.
Here, the term "the function of imparting stability to flavoring compounds" as used herein refers to a function to stabilize the compound structure of the flavoring compound, thereby suppressing its decomposition or structural change, and to prevent the loss of flavor or the occurrence of offensive odor.
Specifically, when a composition containing the *Helianthus* seed extract according to the present invention and flavoring compounds is prepared, functions such as the improvement of the retention rate of the flavoring compounds and the prevention of the generation of odor compounds are exhibited. For example, it is possible to prepare a flavoring composition in which its flavor characteristics derived from the flavorant are not easily lost and the occurrence of offensive odor is suppressed even after heat treatment (e.g. production, processing, or cooking) or the passage of time (e.g. storage).
As a conventional technique in the art, it is reported that the "water and/or ethanol extract of fermented tea leaves" is effective as a decomposition inhibitor of citral, which is a flavoring compound (Patent Document 4). However, it is not admitted that this extract has a sufficient effect in suppressing the generation of the p-cresol, which is the main component causing the degradation smell of citral.
The *Helianthus* seed extract according to the present invention is a composition that has a function of imparting extensive and long-term stability, specifically heat resistance, to flavoring compounds. Here, the function of imparting heat resistance exhibited by the *Helianthus* seed extract according to the present invention refers to a function to maintain flavoring compounds in a stable state even after a long-term storage under a high temperature of about 50°C.
The kind of flavoring compounds for which the *Helianthus* seed extract according to the present invention can exhibit the function of imparting stability is not particularly limited, and both natural and artificial flavorings are included. Preferable examples of flavoring compounds are flavoring compounds belonging to monoterpenes. That is, the *Helianthus* seed extract according to the present invention is a composition that is preferably involved in the stabilization of the molecular structure of monoterpenes.
Here, specific examples of monoterpenes are citral (a general term for geranial and neral), limonene, pinene, sabinene, and myrcene. In particular, citral is preferable.
Examples of flavoring compositions for which the *Helianthus* seed extract according to the present invention can preferably exhibit the function of imparting stability are flavoring compositions containing citral as their flavoring component. Specific examples thereof are lemon flavorings, orange flavorings, lime flavorings, ginger flavorings, yuzu (*Citrus junos*) flavorings, and grapefruit flavorings.

Index of the Function of Imparting Stability

The function of imparting stability to flavoring compounds performed by the *Helianthus* seed extract according to the present invention can be evaluated and specified by using as indices the measured values regarding odor components obtained after conducting a stability test on heat resistance.
Specifically, in the *Helianthus* seed extract according to the present invention, the function of imparting stability can be evaluated and specified by using the p-cresol content after a heat resistance test as an index in a case where measurement is performed by the method as described in (E) (e-1) below. Here, "p-cresol" is a compound having a disinfectant-like odor. This compound is a main odor component generated and increased with the passage of time due to the decomposition and the like of citral. The generation of this odor component is increased especially by storage under a high temperature.

Accordingly, in the present invention, by measuring the p-cresol content, it is possible to judge whether the extract has the function of imparting stability to flavoring compounds or not.

That is, the *Helianthus* seed extract according to the present invention has a feature as follows:

(E) (e-1) A content of p-cresol of an acid-sugar solution when stored in a dark place at 50°C for 7 days is 20 ppb or less, preferably 17.5 ppb or less, and more preferably 15 ppb or less, wherein the acid-sugar solution contains citral in an amount of 0.01 mass%, contains the *Helianthus* seed extract in an amount of 0.0075 mass% in terms of a solid content and is adjusted to have 10° Brix by high fructose corn syrup and to have a pH being 3.0.

It is not preferable if the value is higher than the predetermined level, because the function of imparting stability becomes insufficient. Meanwhile, this excellent effect of reducing the generation of p-cresol cannot be achieved by a conventional technique in which the water and/or ethanol extract of fermented tea leaves is used as an active ingredient (Patent Document 4).

Further, in addition to the evaluation of the p-cresol content as described above, it is preferable to evaluate and specify the ability of the *Helianthus* seed extract according to the present invention to suppress the degradation smell by using as an index the p-methylacetophenone content after a heat resistance test. Specifically, it can be evaluated and specified by using the p-methylacetophenone content after a heat resistance test as an index in a case where measurement is performed by the method as described in (E) (e-2) below.

Here, "p-methylacetophenone" is a compound having an almond-like odor. Just like p-cresol, this compound is a main odor component generated and increased with the passage of time due to the decomposition of citral. The generation of this odor component is increased especially by storage under a high temperature.

Accordingly, in the present invention, in addition to the evaluation of the p-cresol content as described above, it is preferable to judge whether the extract has the function of imparting stability to flavoring compounds by measuring the generation of p-methylacetophenone.

That is, the *Helianthus* seed extract according to the present invention has a feature as follows:

(E) (e-2) A content of p-methylacetophenone of an acid-sugar solution when stored in a dark place at 50°C for 7 days is 1,600 ppb or less, preferably 1,500 ppb or less, more preferably 1,350 ppb or less, and even more preferably 1,200 ppb or less, wherein the acid-sugar solution contains citral in an amount of 0.01 mass%, contains the *Helianthus* seed extract in an amount of 0.0075 mass% in terms of a solid content and is adjusted to have 10° Brix by high fructose corn syrup and to have a pH being 3.0. It is not preferable if the value is higher than the predetermined level, because the function of imparting stability becomes insufficient.

The features described in (A) to (F) above are realized by the compositional features of the compounds constituting the *Helianthus* seed extract according to the present invention and are features expressing these compositional features.

As a preferred mode according to the present invention, it is particularly preferable that the *Helianthus* seed extract according to the present invention is a composition having the features described in (A) to (F) above. Here, in the present invention, the composition having the features described in (A) to (F) above can be explained as a composition having all the features described in (A) to (F) above, and what is meant here is that the composition includes a necessary part of each of feature (A) to feature (F). In this regard, the invention is not limited to a composition that includes the entire feature described in each of (A) to (F) above as the best mode for each feature.

## 2. Method for Producing *Helianthus* Seed Extract

The *Helianthus* seed extract according to the present invention having the features as described in Chapter 1 above can be prepared by the following steps. Here, the production method according to the present invention does not preclude the inclusion of steps other than those described below as long as they do not practically interfere with the effects realized by the technical features of the present invention. Also, the technical scope of the present invention is not limited to a mode including all the following steps, as long as its essential steps are included.

The method for producing an extract from *Helianthus* seeds according to the present invention pertains to a method characterized by including the following steps: (step 1) A step of performing aqueous extraction or aqueous alcohol extraction on the *Helianthus* seeds; (step 2) A step of recovering the extract solution by performing solid-liquid separation after the extraction; and (step 3) A step of performing heat treatment on the extract solution at 60°C or higher under an acidic condition. The steps 1, 2 and 3 are performed in this order in the production method according to the present invention. Meanwhile, the production method according to the present invention does not preclude the inclusion of other steps performed between, before and/or after each step.

Raw Material

"*Helianthus* seeds" which are the raw materials used in the production method according to the present invention refer to the seeds of the plants belonging to the genus *Helianthus* of the family Asteraceae. In the present invention, it is particularly preferable to use the seeds of the plants belonging to *Helianthus annuus*. Here, *Helianthus annuus* is the scientific name for the sunflower. In the present invention, it is possible to use the seeds of any varieties belonging to *Helianthus annuus* as raw materials.

As the *Helianthus* seeds applicable in the present invention, the seeds can be used as they are, however, it is preferable to employ a material obtained by crushing, pulverizing, grinding, mashing or powdering the seed coat. That is, it is preferable to use a processed material of the above-identified seeds. In particular, it is preferable to use oil cakes, which are residues of oil expression.

As the raw material employed in the present invention, it is particularly preferable to use a material in which seed shells are removed. In particular, the seed shell content is preferably 20% or less, and more preferably 10% or less. That is, as the raw material used in the present invention, it is preferable to employ a processed material of seeds in which a process of removing seed shells is conducted and whose seed shell content is adjusted to 20% or less, and more preferably 10% or less.

For the *Helianthus* seeds, which are employed as the raw material in the present invention, it is preferable that the seed shell content is low, because there is a tendency that the lower the seed shell content is, the more the function of imparting stability to natural colorants and flavoring compounds improves. In this regard, the conventional technique of sunflower seed extraction in Patent Document 1 only discloses a technical idea of preferably utilizing seed shells.

Extraction Step

In the production method according to the present invention, it is preferable to carry out extraction on the *Helianthus* seeds mentioned above in a way that enables the extract to contain a high amount of components having functions of imparting stability and that can reduce the components causing offensive odor and degradation smell. Specifically, in the production method according to the present invention, aqueous extraction or aqueous alcohol extraction is performed on the *Helianthus* seeds mentioned above.

When extraction is performed on the plant, which is the raw material, there is no limitation on the shape of the raw material, and it can be directly subjected to extraction with a solvent. In order to increase the surface area and improve the extraction efficiency, it is preferable to process (e.g. crushing) the raw material. Meanwhile, if desired, the extraction process can also be performed on the residue obtained after the extraction in this step, preferably the residue of the first extraction.

It is preferable to use water or aqueous alcohol as the extraction solvent. Examples of the alcohol used in the aqueous alcohol are lower alcohols with 1 to 4 carbon atoms. Specific examples thereof are methanol, ethanol, propanol, isopropyl alcohol, and butanol. A preferable example is ethanol.

Water or aqueous alcohol is particularly preferable as solvents. For the aqueous alcohol, it is preferable that the alcohol content is 50% (v/v) or less, preferably 40% (v/v) or less, more preferably 30% (v/v) or less, and even more preferably 20% (v/v) or less. The lower limit of the alcohol content is not particularly limited, and an example thereof is 1% (v/v) or more.

The amount of the solvent used relative to that of the raw material is not particularly limited, and an example thereof is 0.1 to 1,000 parts by mass of the solvent relative to 1 part by mass of the raw material. In particular, it is preferable to use the solvent of 0.5 to 100 parts by mass, and preferably 1 to 50 parts by mass, relative to 1 part by mass of the raw material.

The extraction operation can be performed by an ordinary method, and there is no limitation on it. Soaking extraction or static extraction can be employed, and it is possible to improve the extraction efficiency by performing such operations as stirring, suspension, shaking, vibration, or ultrasonication, for example.

The temperature condition for extraction in the present invention is preferably lower than 80°C. It is preferably 75°C or lower, more preferably 70°C or lower, even more preferably 65°C or lower, and even more preferably 60°C or lower. In the present invention, setting the extraction temperature lower than a predetermined value enables the extract to contain a high amount of components having functions of imparting stability and can reduce the components causing offensive odor and degradation smell. It is not preferable if the extraction temperature is too high, because offensive odor and degradation smell are added to the extract obtained.

The lower limit of the extraction temperature is not particularly limited as long as extraction can be performed, and considering the extraction efficiency, it is preferably 10°C or higher, more preferably 20°C or higher, even more preferably 25°C or higher, even more preferably 30°C or higher, and even more preferably 35°C or higher.

Meanwhile, the extraction time is not particularly limited, and its example is 1 minute to 72 hours. It is preferably 10 minutes to 48 hours, and more preferably 30 minutes to 24 hours.

Solid-Liquid Separation Step

In the production method according to the present invention, solid-liquid separation is performed to remove the residues of raw materials and to recover the extract solution before conducting heat treatment on the extract solution mentioned above. In the present invention, it is not preferable if the solid-liquid separation step is not performed, because offensive odor and degradation smell are added to the extract obtained in the following heat treatment step.

The solid-liquid separation can be performed by an ordinary method. For example, filtration, suction filtration, coprecipitation, or centrifugation can be employed to remove the solid or insoluble matter. It is also preferable to use a filter aid (e.g. diatomaceous earth) when filtration is performed. The solid-liquid separation step can also be performed a plurality of times if desired.

Heating Step

In the production method according to the present invention, the extract solution obtained after the above-mentioned solid-liquid separation is subjected to heat treatment. In the present invention, it becomes possible to remarkably reduce or eliminate the components causing offensive odor and degradation smell by performing the heat treatment after removing the residues of raw materials. In addition, even if the heat treatment is performed, the amount of components having functions of imparting stability is hardly reduced and the extract's functions regarding stability are maintained. Meanwhile, the heating step can also be performed a plurality of times if desired.

In one mode of the production method according to the present invention, the heating step can be performed as a part of or an alternative to the sterilization treatment. Also, in another mode of the production method according to the present invention, the heating step can be performed as a part of or an alternative to the concentration treatment.

In the present invention, the heat treatment needs to be performed at a temperature of 60°C or higher. It is preferably 65°C or higher, more preferably 70°C or higher, even more preferably 75°C or higher, and even more preferably 80°C or higher. The upper limit can be set to the boiling point of the aqueous solution and is preferably 100°C or lower for an aqueous solution under a normal pressure. For an aqueous solution under a pressurized condition, it is preferably 150°C or lower, more preferably 140°C or lower, even more preferably 130°C or lower, and even more preferably 120°C or lower.

Specifically, it is preferable that the temperature is 60 to 150°C, preferably 65 to 140°C, more preferably 70 to 130°C, even more preferably 75 to 100°C, and even more preferably 80 to 95°C.

It is not preferable if the temperature is too lower than the predetermined value, because the components in the extract causing offensive odor and degradation smell are not sufficiently removed or deactivated. Also, it is not preferable if the temperature is too higher than the predetermined value, because the effects on the components having functions of imparting stability are concerned.

The heating time is a factor determined by the temperature condition and can be determined properly, and its example is 1 minute to 72 hours. It is preferably 10 minutes to 48 hours, and more preferably 30 minutes to 24 hours, for example.

It is not preferable if the heating time is too shorter than the predetermined value, because the components in the extract causing offensive odor and degradation smell are not sufficiently removed or deactivated. Also, it is not preferable if the temperature is too longer than the predetermined value, because the effects on the components having functions of imparting stability are concerned.

The heat treatment in the present invention is preferably carried out under an acidic condition. In the heat treatment, it becomes possible to remarkably reduce or eliminate the components causing offensive odor and degradation smell by adjusting the extract solution after the above-mentioned solid-liquid separation to an acidic condition.

Means for adjustment to an acidic condition include the addition of an inorganic and/or organic acid, for example. Examples of the inorganic acid are hydrochloric acid, sulfuric acid, nitric acid, and phosphoric acid, and examples of the organic acid are citric acid, acetic acid, malic acid, and lactic acid.

The amount of the inorganic or organic acid added to the extraction solvent is not particularly limited, and it is preferable to appropriately adjust it within a range of around 0.001 to 10 mass%, preferably 0.01 to 7.5 mass%, and more preferably 0.025 to 5 mass% with respect to the extraction solvent. Though it is preferable to conduct the adjustment to an acidic condition prior to the heat treatment, it can also be carried out during the heat treatment.

In the adjustment to an acidic condition in the heat treatment in the present invention, it is preferable to adjust the hydrogen ion concentration of the extract solution after the solid-liquid separation by setting the hydrogen ion concentration ratio to a predetermined value. The "hydrogen ion concentration ratio" as used herein is expressed as the ratio of the hydrogen ion concentration of the solution to be subjected to the heat treatment relative to the hydrogen ion concentration of the extract solution after the aqueous extraction or aqueous alcohol extraction mentioned above.

Here, the extract solution obtained by performing the extraction step as described in the paragraphs above becomes a solution with a hydrogen ion concentration of $3.16 \times 10^{-8}$ to $1 \times 10^{-6}$ mol/L.

As the hydrogen ion concentration suitable for the heat treatment in the present invention, it is preferable that the hydrogen ion concentration ratio is set to $1 \times 10^1$ to $3.16 \times 10^5$, preferably $3.16 \times 10^1$ to $1 \times 10^5$, and more preferably $1 \times 10^2$ to $3.16 \times 10^4$ when the hydrogen ion concentration of the extract solution after the aqueous extraction or aqueous alcohol extraction is taken as 1.

Also, as the hydrogen ion concentration suitable for the heat treatment in the present invention, it is preferable that the hydrogen ion concentration ratio is set to $2.24 \times 10^1$ to $2.24 \times 10^4$, preferably $7.08 \times 10^1$ to $7.08 \times 10^3$, and more preferably $2.24 \times 10^2$ to $2.24 \times 10^3$ when the average hydrogen ion concentration of the extract solution after the aqueous extraction or aqueous alcohol extraction, which is $4.47 \times 10^{-7}$ mol/L, is taken as 1.

In the present invention, it is not preferable if the above-mentioned hydrogen ion concentration ratio falls out of a predetermined range, because it becomes difficult to reduce or eliminate the components causing offensive odor and degradation smell.

Meanwhile, it is preferable that the hydrogen ion concentration of the extract solution after the adjustment of the hydrogen ion concentration ratio is $1 \times 10^{-5}$ to $1 \times 10^{-2}$ mol/L, preferably $3.16 \times 10^{-5}$ to $3.16 \times 10^{-3}$ mol/L, and more preferably $1 \times 10^{-4}$ to $1 \times 10^{-3}$ mol/L.

Solid-Liquid Separation and Refinement Step

In the production method according to the present invention, it is desirable to remove the residues and recover the extract solution by conducting solid-liquid separation after the heat treatment mentioned above. In the present invention, the solid-liquid separation after the heat treatment enables to further reduce the turbidity of the extract obtained and thus is preferable.

The solid-liquid separation can be performed by an ordinary method. For example, filtration, suction filtration, coprecipitation, or centrifugation can be employed to remove the solid or insoluble matter. Meanwhile, it is also preferable to use a filter aid (e.g. diatomaceous earth) if filtration is performed. The solid-liquid separation step can also be performed a plurality of times if desired.

In the production method according to the present invention, it is also preferable to remove the impurities in the extract by conducting refinement after the heat treatment or the solid-liquid separation after the heat treatment as described above. An ordinary method can be employed in the refinement process as long as it does not cause the loss of the components having functions of imparting stability.

For example, the refinement can be conducted by adsorption treatment using silica gel, porous ceramics, styrenic or aromatic synthetic resins, or the like. It can also be conducted by ion exchange treatment using cationic or anionic resins. It can also be conducted by membrane separation treatment using membrane filters, ultrafiltration membranes, reverse osmosis membranes, electrodialysis membranes, functional polymer membranes, or the like.

Other Steps

In the production method according to the present invention, a *Helianthus* seed extract (composition) of desired qualities and/or forms can be produced as the extract solution mentioned above is further subjected to refinement treatment, concentration treatment, pH adjustment, dilution treatment, drying treatment, sterilization treatment, or the like depending on the purpose of use. Moreover, enzyme treatment with proteases or the like, microorganism treatment, or the like can also be performed depending on the purpose of use. These steps can be carried out not only at the final stage after the pH adjustment step but also after any of the steps described above as needed. Desired steps can also be performed in combination. It is also possible to conduct a desired step a plurality of times.

In the production method according to the present invention, operations such as dilution, concentration, and drying can be performed by ordinary methods. The refinement treatment can be conducted by the same method as described in the paragraphs above.

In the production method according to the present invention, the pH adjustment operation can be performed by an ordinary method. In the present invention, when the extract solution obtained does not have a near neutral pH, it is preferable to adjust it to near neutral.

In the production method according to the present invention, the sterilization treatment can also be performed by an ordinary method. Examples of the sterilization method are heat treatment, high pressure treatment, high-pressure heat treatment, sterile filtration, ultraviolet irradiation, and chemical treatment with disinfectants. It is preferable to conduct the sterilization treatment using the heat treatment or the high-pressure heat treatment.

Examples of the temperature condition for heat sterilization are 60°C or higher, preferably 70°C or higher, and more preferably 80°C or higher. The upper limit is not particularly limited as long as the components having functions of imparting stability are not negatively affected, and its examples are 140°C or lower under a pressurized condition, and 100°C or lower and preferably 95°C or lower under a normal pressure.

[Products]

The present invention includes the *Helianthus* seed extract obtained by the production method described above. That is, the present invention includes the *Helianthus* seed extract produced by conducting the above-mentioned steps 1 to 3 as necessary steps. Meanwhile, it does not preclude the inclusion of the *Helianthus* seed extract produced by methods including steps other than those described above. Also, it is not limited to a mode including all the steps mentioned above, as long as the necessary steps are included.

[Method for Reducing Degradation Smell of *Helianthus* Seed Extract]

In the present invention, by conducting the steps 1 to 3 mentioned above in the production process of the *Helianthus* seed extract, it becomes possible to reduce the occurrence of offensive odor, and moreover, the occurrence of degradation smell with the passage of time in the *Helianthus* seed extract produced.

That is, in the present invention, it becomes possible to provide a method for reducing degradation smell of the *Helianthus* seed extract produced, wherein the steps 1 to 3 mentioned above are performed in the process for producing the *Helianthus* seed extract.

The steps 1 to 3 in the production method for the *Helianthus* seed extract described above can be employed as the steps 1 to 3 in the method for reducing degradation smell. As the processes other than these steps, those in the production method for the *Helianthus* seed extract described above can also be employed.

3. Application

The *Helianthus* seed extract according to the present invention is a composition that exhibits excellent properties relating to odor characteristics. That is, it is a composition in which the offensive odor derived from raw materials and the occurrence of degradation smell with the passage of time are remarkably suppressed. Also, the *Helianthus* seed extract according to the present invention is a composition that exhibits excellent effects in the function of imparting stability to natural colorants and flavoring compounds.

As a result, the *Helianthus* seed extract according to the present invention can be utilized in a wide spectrum of fields for purposes which could not be fulfilled by *Helianthus* seed extracts obtained by conventional techniques.

[Application for the Prevention of Color Fading of Natural Colorants]

The *Helianthus* seed extract according to the present invention can be utilized as an agent for preventing color fading of natural colorants, an agent for suppressing color fading of natural colorants, an agent for stabilizing natural colorants, an agent for protecting natural colorants, an agent for imparting heat resistance to natural colorants, an agent for imparting light resistance to natural colorants, or the like. Also, the *Helianthus* seed extract according to the present invention can be used in methods for realizing these applications. The present invention includes methods for producing these agents.

Here, examples of natural colorants are anthocyanin pigments and gardenia pigments, which are natural colorants described in Chapter 1 above. Preferable examples thereof are anthocyanin pigments and gardenia red pigments, preferably anthocyanin pigments, and more preferably acylated anthocyanin pigments.

The form of the aforementioned agents such as those for preventing color fading of natural colorants in the present invention is not particularly limited, and examples thereof are a liquid form, a paste form, a gelatinous form, a semi-solid form, and a solid form (e.g. a powdery form and a granular form). A particularly preferable example thereof is a powdery form.

Meanwhile, the ratio of the *Helianthus* seed extract contained in the aforementioned agents such as those for preventing color fading of natural colorants according to the present invention is not particularly limited if it is determined taking into consideration the final concentration at the time when the agent is used, and for example, it is 1 mass% or more, preferably 5 mass% or more, and more preferably 10 mass% or more in terms of the solid content of the *Helianthus* seed extract. Also, the upper limit of the ratio of the *Helianthus* seed extract contained in the aforementioned agents such as those for preventing color fading of natural colorants according to the present invention is not particularly limited, and for example, it is 100 mass% or less, preferably 95 mass% or less, and more preferably 90 mass% or less in terms of the solid content of the *Helianthus* seed extract.

In addition, other components can be contained in the aforementioned agents such as those for preventing color fading of natural colorants, as long as the function of imparting stability to natural colorants performed by the *Helianthus* seed extract according to the present invention is not damaged.

The *Helianthus* seed extract according to the present invention can be utilized in a form of a pigment preparation by being mixed with a natural colorant in advance. Such a pigment preparation becomes a pigment preparation in which the color fading of the natural colorant contained therein is prevented or suppressed.

An example of the pigment preparation is an anthocyanin pigment preparation. Specific examples are pigment preparations of red radish color, red cabbage color, purple sweet potato color, grape juice color, grape skin color, colored potato color, purple corn color, *Clitoria ternatea* color, black soybean color, elderberry color, cranberry color, gooseberry color, salmonberry color, strawberry color, cherry color, dark sweet cherry color, thimbleberry color, European dewberry color, hibiscus color, black huckleberry color, black currant color, black berry color, red currant color, loganberry color, plum color, blueberry color, boysenberry color, whortleberry color, mulberry color, morello cherry color, raspberry color, perilla color, red rice color, and purple carrot color.

Also, an example of the pigment preparation is a gardenia pigment preparation. Specific examples are pigment preparations of gardenia red pigment, gardenia blue pigment and gardenia yellow pigment.

The ratio of the *Helianthus* seed extract contained in the pigment preparation according to the present invention can be adjusted depending on the type and purpose of use of the pigment preparation and is not particularly limited as long as the function of imparting stability to natural colorants is exhibited. For example, a pigment preparation of color value 60 can contain the *Helianthus* seed extract in an amount of 1 to 95 mass%, preferably 5 to 90 mass%, and more preferably 10 to 85 mass% in terms of the solid content.

The pigment preparations according to the present invention can not only be used for conventional purposes as colorants using pigments but also in a wide spectrum of fields for purposes which could not be fulfilled by conventional natural colorants, because the light and heat resistance of the colorant is improved, and the occurrence of odor is suppressed. Specifically, the pigment preparations according to the present invention (i.e. the pigment preparations containing the *Helianthus* seed extract according to the present invention) can be suitably used as natural colorants used in such products as foods, beverages, quasi-drugs, medicines, cosmetic products, personal hygiene products, and feed.

The ratio of the *Helianthus* seed extract according to the present invention contained in various products such as foods, beverages and the like can be adjusted depending on the type and purpose of use of the product as long as the function of imparting stability to natural colorants is exhibited.

For example, a product can contain 0.001 to 10 mass%, preferably 0.003 to 1 mass%, and more preferably 0.005 to 0.1 mass% of *Helianthus* seed extract in terms of the solid content.

Method for Preventing Color Fading of Natural Colorants

The present invention includes various methods for fulfilling the purpose of imparting stability to natural colorants. More specifically, the present invention includes various methods such as a method for preventing color fading of natural colorants, a method for suppressing color fading of natural colorants, a method for stabilizing natural colorants, a method for protecting natural colorants, a method for imparting heat resistance to natural colorants, and a method for imparting light resistance to natural colorants, the methods including a step in which the aforementioned *Helianthus* seed extract is contained in the pigment preparations or the products.

The description in paragraphs above can be referred to upon determining the content of the *Helianthus* seed extract in these various methods.

[Application for Suppressing Degradation Smell of Flavoring Compounds]

The *Helianthus* seed extract according to the present invention can be used as an agent for suppressing degradation smell of flavoring compounds, an agent for preventing degradation smell of flavoring compounds, an agent for stabilizing flavoring compounds, an agent for protecting flavoring compounds, an agent for imparting heat resistance to flavoring compounds, an agent for imparting light resistance to flavoring compounds, or the like. Especially, among these applications, the *Helianthus* seed extract according to the present invention can be suitably used for the purposes relating to the imparting of heat resistance.

Also, the *Helianthus* seed extract according to the present invention can be used in methods for achieving these applications. The present invention encompasses the production methods of these agents.

The examples of flavoring compounds include monoterpenes, which are flavoring compounds referred to in Chapter 1 above. Specific examples thereof are citral (a general term for geranial and neral), limonene, pinene, sabinene, and myrcene.

The form of the aforementioned agents such as those for suppressing degradation smell of flavoring compounds in the present invention is not particularly limited, and examples thereof are a liquid form, a paste form, a gelatinous form, a semi-solid form, and a solid form (e.g. a powdery form and a granular form).

The ratio of the *Helianthus* seed extract contained in the aforementioned agents such as those for suppressing degradation smell of flavoring compounds according to the present invention is not particularly limited if it is determined taking into consideration the final concentration at the time when the agent is used, and for example, it is 1 mass% or more, preferably 5 mass% or more, and more preferably 10 mass% or more in terms of the solid content of the *Helianthus* seed extract. Also, the upper limit of the ratio of the *Helianthus* seed extract contained in the aforementioned agents such as those for suppressing degradation smell of flavoring compounds according to the present invention is not particularly limited, and for example, it is 100 mass% or less, preferably 95 mass% or less, and more preferably 90 mass% or less in terms of the solid content of the *Helianthus* seed extract.

In addition, other components can be contained in the aforementioned agents such as those for suppressing degradation smell of flavoring compounds, as long as the function of imparting stability to flavoring compounds performed by the *Helianthus* seed extract according to the present invention is not damaged.

The *Helianthus* seed extract according to the present invention can be utilized in a form of a flavoring preparation by being mixed with flavoring compounds in advance. Such a flavoring preparation becomes a flavoring preparation in which the degradation of flavoring compounds contained therein is prevented or suppressed.

Examples of the flavoring preparation are citrus and ginger flavoring preparations. Specific examples thereof are lemon flavoring preparations, orange flavoring preparations, lime flavoring preparations, ginger flavoring preparations, yuzu (*Citrus junos*) flavoring preparations, and grapefruit flavoring preparations.

The ratio of the *Helianthus* seed extract contained in the flavoring preparation according to the present invention can be adjusted depending on the type and purpose of use of the flavoring preparation and is not particularly limited as long as the function of imparting stability to flavoring compounds is exhibited, and for example, a flavoring preparation can contain the *Helianthus* seed extract in an amount of 1 to 95 mass%, preferably 5 to 90 mass%, and more preferably 10 to 85 mass% in terms of the solid content.

The flavoring preparations according to the present invention can not only be used for conventional uses of flavorants but also in a wide spectrum of fields for purposes which could not be fulfilled by conventional flavorants, because the heat resistance of the flavoring compounds is improved, and the occurrence of odor is suppressed. Specifically, the flavoring preparations according to the present invention (i.e. the flavoring preparations containing the *Helianthus* seed extract according to the present invention) can be suitably used as flavorants used in such products as foods, beverages, quasi-drugs, medicines, cosmetic products, personal hygiene products, and feed.

The ratio of the *Helianthus* seed extract according to the present invention contained in various products such as foods, beverages and the like can be adjusted depending on the type and purpose of use of the product as long as the function of imparting stability to flavoring compounds is exhibited. For example, a product can contain the *Helianthus* seed extract in an amount of 0.001 to 10 mass%, preferably 0.003 to 1 mass%, and more preferably 0.005 to 0.1 mass% in terms of the solid content.

Method for Suppressing Degradation Smell of Flavoring Compounds

The present invention includes various methods for fulfilling the purpose of imparting stability to flavoring compounds. More specifically, the present invention includes various methods such as a method for suppressing degradation smell of flavoring compounds, a method for preventing degradation smell of flavoring compounds, a method for stabilizing flavoring compounds, a method for protecting flavoring compounds, a method for imparting heat resistance to flavoring compounds, and a method for imparting light resistance to flavoring compounds, the methods including a step in which the aforementioned *Helianthus* seed extract is contained in the flavoring preparations or the products.

The description in paragraphs above can be referred to upon determining the content of the *Helianthus* seed extract in the various methods.

[Uses for Flavoring Preparations]

The *Helianthus* seed extract according to the present invention is a composition that exhibits excellent properties relating to odor characteristics. Also, since the *Helianthus* seed extract is an extract derived from a sunflower, which is a flavoring substance, it can be used as a flavoring preparation even if it is not mixed with other flavoring compounds. That is, it can be used as a flavoring preparation whose flavoring component is the *Helianthus* seed extract itself.

As the form of this flavoring preparation, it is possible to employ the same forms employed in the above-mentioned agents containing flavorants.

The flavoring preparations according to the present invention can be used in a wide spectrum of fields for purposes for which conventional *Helianthus* seed extract could not be used as flavorants, because the occurrence of offensive odor and degradation smell is suppressed. Specifically, the flavoring preparations according to the present invention (i.e. the flavoring preparations containing the *Helianthus* seed extract according to the present invention as their flavoring component) can be suitably used as flavorants used in such products as foods, beverages, quasi-drugs, medicines, cosmetic products, personal hygiene products, and feed.

The ratio of the *Helianthus* seed extract contained in the flavoring preparation according to the present invention can be adjusted depending on the purpose of use of the flavoring preparation and is not particularly limited, and for example, it is 1 mass% or more, preferably 5 mass% or more, and more preferably 10 mass% or more in terms of the solid content of the *Helianthus* seed extract. Also, the upper limit of the ratio of the *Helianthus* seed extract contained in the flavoring preparation according to the present invention is not particularly limited, and for example, it is 100 mass% or less, preferably 95 mass% or less, and more preferably 90 mass% or less in terms of the solid content of the *Helianthus* seed extract.

In addition, other components can be contained in the flavoring preparation, as long as the flavor and aroma of the *Helianthus* seed extract according to the present invention are not negatively affected.

The ratio of the *Helianthus* seed extract according to the present invention contained in various products such as foods, beverages and the like can be adjusted depending on the type and purpose of use of the product. For example, a product can contain the *Helianthus* seed extract in an amount of 0.001 to 10 mass%, preferably 0.003 to 1 mass%, and more preferably 0.005 to 0.1 mass% in terms of the solid content.

[Examples of Products]

Hereinafter, the products for which the *Helianthus* seed extract according to the present invention can be used will be shown with examples. However, the products for which the *Helianthus* seed extract according to the present invention can be used are not limited to these examples.

The examples of "foods" and "beverages" are beverages, frozen or chilled desserts, desserts, sugar confectionery (e.g. candies, gummies and marshmallows), gum, chocolate, confectionery (e.g. cookies), bread, processed agricultural products (e.g. pickles), processed meat products, processed marine products, dairy products, noodles, seasonings, jams, sauces, and alcoholic drinks.

The examples of "cosmetic products" are skin lotions, lipsticks, sunscreen cosmetics, and makeup cosmetics.

The examples of "medicines" are various tablets, capsules, liquid medicines, lozenges, and mouthwashes.

The examples of "quasi-drugs" are nutritional supplements, various supplements, dentifrice, mouth freshener, deodorants, hair growth tonics, anti hair loss tonics, and skin moisturizers.

The examples of "personal hygiene products" are soaps, detergents, shampoos, conditioners, hair treatments, dentifrice, and bath additives.

The examples of "feed" are various pet foods such as cat foods and dog foods, and feed for aquarium and farmed fish.

Among the examples above, products that are expected to be displayed in the store or be stored under a high temperature are especially preferable as the products utilizing the *Helianthus* seed extract according to the present invention. Examples thereof are beverages (fruit juice drinks, non-fruit drinks, carbonated drinks, soured milk beverages, non-alcoholic drinks, and the like), alcoholic drinks, candies, gum, jelly, syrup, jams, and pickles.

Also, the *Helianthus* seed extract according to the present invention can be suitably used in products such as carbonated drinks and alcoholic drinks, in which the color fading of natural colorants proceeds rapidly and the effects of odor tend to be problematic.

[Various Production Methods]

The present invention includes the methods for producing various agents and products. More specifically, the present invention includes the production methods for agents for preventing color fading of natural colorants, agents for suppressing degradation smell of flavoring compounds, pigment preparations, flavoring preparations, foods, beverages, cosmetic products, medicines, quasi-drugs, personal hygiene products, feed, and the like, the methods including a step in which the aforementioned *Helianthus* seed extract is contained in the agents, the preparations, or the products. These production methods include a step in which the *Helianthus* seed extract obtained by the production method mentioned above is contained in the agents, the preparations, or the products.

The description regarding various agents and products in paragraphs above can be referred to upon determining the content of the *Helianthus* seed extract in these production methods. Also, conventional methods for producing various agents and products can be employed in the production processes of these production methods as long as the aforementioned *Helianthus* seed extract is added to the agents, the preparations, or the products.

Examples

Hereinafter, the present invention will be described by use of examples, but the scope of the present invention is not limited by the examples.

Example 1: "Production of *Helianthus* Seed Extract"

Sunflower seed extract, which is a *Helianthus* seed extract, was prepared using sunflower oil cakes as the raw material.

Sunflower seed oil cakes (with seed shell content of less than 10%) were pulverized and extracted with aqueous alcohol (20% ethanol solution) in an amount of 20 times of the weight of the raw material. The extraction was performed by stirring at 60°C for 5 hours. After the extraction, diatomaceous earth, used as a filter aid, was added at a ratio of 1 part by mass of diatomaceous earth with respect to the mass of the solution and mixed. The mixed solution was subjected to suction filtration using a filter (No. 2 Filter, φ150mm, Advantec Toyo Kaisha, Ltd.) on which a layer of the diatomaceous earth was formed in advance, and the filtrate was collected.

The hydrogen ion concentration of the filtrate obtained was adjusted by adding sulfuric acid to the filtrate so that its hydrogen ion concentration ratio in which the hydrogen ion concentration immediately after the extraction ($4.47 \times 10^{-7}$ mol/L) was taken as 1 would be $2.24 \times 10^3$, and the solution was subjected to heat treatment at 80°C for 30 minutes. Then, suction filtration was conducted using the filter aid as described above, the filtrate was collected, and the pH was adjusted to neutral.

Vacuum concentration was conducted using an evaporator to prepare a concentrated solution, which was then sterilized by heat treatment at 80°C for 10 minutes. Then, a sunflower seed extract (Sample Product 1) which was concentrated to the solid content of 15 mass% was obtained.

Another sunflower seed extract (Sample Product 2) was prepared in the same manner as described above except that the extraction was carried out with water in an amount of 10 times of the weight of the raw material at 60°C for 5 hours.

As a conventional product for comparison, a sunflower seed extract was prepared following the method described in Patent Document 1. In this method, crushed sunflower seeds (without removing the seed shells) were extracted with water 7 times the weight of the crushed sunflower seeds at 80-85°C for 5 hours. After conducting suction filtration, a concentrated solution was prepared using an evaporator, and a sunflower seed extract (Comparative Product 1) which was concentrated to the solid content of 15 mass% was obtained.

Example 2: "Stability Test on *Helianthus* Seed Extract (Odor Analysis)"

Evaluation was carried out on the occurrence of odor with the passage of time in the sunflower seed extracts which were prepared in the example above as *Helianthus* seed extracts. GC/MS analysis of the compounds causing degradation smell was employed as the evaluation method.

(1) Preparation of the Beverage

An acid-sugar solution that contains the sunflower seed extract in an amount of 0.0075 mass% in terms of the solid content, is 10° Brix (adjusted with 13.3 mass% of 75° Brix high fructose corn syrup) and has pH3.0 (adjusted with citric acid anhydrous (0.2 mass%) and trisodium citrate dihydrate) was prepared. The solution was heated up to 93°C for sterilization and hot packed into a 200 mL plastic bottle. The plastic bottle beverage obtained was used as the test liquid.

Sample Product 2 and Comparative Product 1 were used as the sunflower seed extracts. A test liquid with no sunflower seed extract added was also prepared as a control.

(2) Stability Test

(i) Light Resistance Test

The test liquids in plastic bottles were irradiated with white fluorescent light of 10,000 lux at 30°C for 7 days in a fluorescent light irradiation device. A CLH-301 cultivation chamber (Tomy Seiko Co., Ltd.) was used as the fluorescent light irradiation device.

(ii) Heat Resistance Test

The test liquids in plastic bottles were stored in a dark place under a high temperature of 50°C for 7 days.

(iii) GC/MS Analysis

The occurrence of smell caused by light degradation was evaluated by conducting GS/MS analysis of the 1-octen-3-one content and the methional content. A gas chromatograph mass spectrometer (Agilent 5975 inert GC/MS System, Agilent Technologies, Inc.) was employed for GC/MS analysis. The results are shown in Tables 1 and 2 and FIGS. 1 and 2.

The results showed that, in the beverage with the comparative product of sunflower seed extract added (Sample 2-1), the content of 1-octen-3-one, which is an odor component having a mushroom-like smell derived from sunflower seed extract, greatly increased up to 0.75 ppb when stored under light irradiation. Since the 1-octen-3-one content before the light irradiation was below the limit of detection, it was shown that 1-octen-3-one was a compound generated with the passage of time due to the light irradiation.

It was also shown that this beverage already contained 0.031 ppb of methional, an odor component having potato-like smell, even before the stability test, and its content increased further due to the light irradiation.

In contrast, in the beverage with a sample product of the sunflower seed extract according to the present invention added (Sample 2-2), the generation of 1-octen-3-one was not detected, with its content below the limit of detection, even after the strong light irradiation as described above. Moreover, the generation of 1-octen-3-one due to the storage under a high temperature was not detected, with its content below the limit of detection. The presence of 1-octen-3-one was not confirmed even before the stability test (immediately after the production).

It was also shown that the methional content in this beverage (Sample 2-2) was greatly reduced to less than approximately 1/15 of that in the beverage with the comparative product added (Sample 2-1) even when measured immediately after the preparation of the beverage (before the stability test). Moreover, the methional content increased only slightly even after the strong light irradiation as described above.

These results show that the *Helianthus* seed extract according to the present invention is a composition in which the occurrence of smell caused by light degradation is greatly suppressed compared with the sunflower seed extract prepared by a conventional production method. It is also shown that the offensive odor occurring immediately after the production, which derives from the raw material, is greatly reduced in this composition.

Table 1

| Samples | 1-octen-3-one content (ppb) | |
|---|---|---|
| | Before the stability test | After the light resistance test at 10,000 lux for 7 days |
| Sample 2-1 (comparative product added) | N.D. | 0.75 |
| Sample 2-2 (product of the present invention added) | N.D. | N.D. |
| Sample 2-3 (control: no sunflower seed extract added) | N.D. | N.D. |

Table 2

| Samples | Methional content (ppb) | |
|---|---|---|
| | Before the stability test | After the light resistance test at 10,000 lux for 7 days |
| Sample 2-1 (comparative product added) | 0.0309 | 0.0438 |
| Sample 2-2 (product of the present invention added) | 0.0019 | 0.0056 |
| Sample 2-3 (control: no sunflower seed extract added) | N.D. | N.D. |

Example 3: "Stability Test on the *Helianthus* Seed Extract (Sensory Evaluation)"

Evaluation of flavor change with time was conducted on the sunflower seed extracts which were prepared in the example above as *Helianthus* seed extracts. Sensory evaluation by panelists was employed as the evaluation method.

(1) Preparation of the Beverage

Plastic bottle beverages were prepared as the test liquids in the same manner as the method described in Example 2 (1).

(2) Stability Test

(i) Light Resistance Test

The test liquids in plastic bottles were irradiated with white fluorescent light of 10,000 lux at 30°C for 14 days in a fluorescent light irradiation device. A CLH-301 cultivation chamber (Tomy Seiko Co., Ltd.) was used as the fluorescent light irradiation device.

(ii) Sensory Evaluation

Blind sensory evaluation on the flavor of the beverages was carried out by five panelists who were professionally engaged in flavor evaluation. The intensity of the smell of the beverage was evaluated at six levels (0 < 1 < 2 < 3 < 4 < 5), where 0 indicates no smell, and 5 indicates strong smell. The results are shown in Table 3.

The results showed that, in the beverage with the comparative product of sunflower seed extract added (Sample 3-1), the occurrence of offensive odor was confirmed immediately after the sterilization in the preparation process of the beverage, and a mushroom-like smell increased due to the storage under light irradiation. In contrast, in the beverage with the sample product of the sunflower seed extract according to the present invention added (Sample 3-2), the occurrence of offensive odor was not confirmed even after the strong light irradiation as described above. Moreover, the occurrence of offensive odor was not confirmed even before the stability test (immediately after the production).

It was confirmed even by the results of the sensory evaluation that the *Helianthus* seed extract according to the present invention is a composition in which the occurrence of smell caused by light degradation is greatly suppressed compared with the sunflower seed extract prepared by a conventional production method. It was also shown that the offensive odor occurring immediately after the production was greatly reduced in this composition.

Table 3

| Samples | Sensory evaluation | |
|---|---|---|
| | Before the stability test | After the light resistance test at 10,000 lux for 14 days |
| Sample 3-1 (comparative product added) | 2.2 | 5.0 |
| Sample 3-2 (product of the present invention added) | 0 | 0 |
| Sample 3-3 (control: no sunflower seed extract added) | 0 | 0 |

Example 4: "Quality Test of the *Helianthus* Seed Extract (Turbidity Measurement)"

Quality evaluation was conducted on the turbidity of the sunflower seed extracts which were prepared in the example above as *Helianthus* seed extracts. The measurement of OD value at a fixed wavelength of 700 nm was employed as the evaluation method.

Solutions containing the sunflower seed extract in an amount of 0.75 mass% in terms of the solid content were prepared using McIlvaine buffer of pH 3.0 or pH 7.0. Sample Product 2 and Comparative Product 1 were used as the sunflower seed extracts.

The OD value at a fixed wavelength of 700 nm was measured for each test liquid prepared using a spectrophotometer (V-560, JASCO Corporation; the optical path length of the measurement cell: 1 cm). The results are shown in Table 4.

The results showed that, in the test liquids with the sample product of the sunflower seed extract according to the present invention added (Samples 4-2 and 4-4), the turbidity was greatly reduced compared with the test liquids with the comparative product added (Samples 4-1 and 4-3). This effect was enough exhibited in neutral solutions (pH 7.0), and especially remarkable in acidic solutions (pH 3.0).

Specifically, the turbidity ($OD_{700}$ value) of the test liquid of pH 3.0 containing the sample product of the present invention (Sample 4-4) was greatly reduced to 0.1167 (approximately 45% of Sample 4-3). This value is much lower than the $OD_{700}$ value of 0.2, which serves as a standard value indicating the occurrence of turbidity in this measurement system.

These results show that the *Helianthus* seed extract according to the present invention is a composition in which the occurrence of turbidity is greatly reduced compared with the sunflower seed extract prepared by a conventional production method.

In particular, it is shown that the *Helianthus* seed extract according to the present invention is a composition in which the occurrence of turbidity under acidic conditions is greatly reduced, while the occurrence of turbidity under acidic conditions is remarkable in the sunflower seed extract prepared by a conventional production method.

Table 4

| Samples | Turbidity ($OD_{700}$ value) |
|---|---|
| Sample 4-1, pH7.0 (comparative product added) | 0.1850 |
| Sample 4-2, pH7.0 (product of the present invention added) | 0.1285 |
| Sample 4-3, pH3.0 (comparative product added) | 0.2558 |
| Sample 4-4, pH3.0 (product of the present invention added) | 0.1167 |

Example 5: "Experiment on the Function of Imparting Stability to Natural Colorants (Pigment Retention Rate)"

Evaluation of the function of imparting stability to natural colorants was conducted on the sunflower seed extract which was prepared in the example above as a *Helianthus* seed extract. The measurement of the retention rate of the anthocyanin pigment, a natural colorant, was employed as the evaluation method.

(1) Preparation of the Beverage

Acid-sugar solutions that contain the sunflower seed extract in an amount of 0.0041 mass% in terms of the solid content, are 10° Brix (adjusted with 13.3 mass% of 75° Brix high fructose corn syrup), and have pH3.0 (adjusted with citric acid anhydrous (0.2 mass%) and trisodium citrate dihydrate), each containing one of the anthocyanin pigments shown in Table 5 at a content of 0.03 mass% when calculated in terms of color value ($E^{10\%}_{1cm}$) 80, were prepared. The solutions were heated up to 93°C for sterilization and hot packed into 200 mL plastic bottles. The plastic bottle beverages obtained were used as the test liquids.

Sample Product 1 was used as the sunflower seed extract. The pigment preparations or colorant compositions manufactured by San-Ei Gen F.F.I., Inc. were used as the anthocyanin pigments. Test liquids with no sunflower seed extract added were also prepared as controls.

Table 5

| Colorant | Sample | Sunflower seed extract |
|---|---|---|
| Red radish color | Sample 5-1 (control: no sunflower seed extract added) | - |
| | Sample 5-2 (product of the present invention added) | Sample Product 1 |
| Red cabbage color | Sample 5-3 (control: no sunflower seed extract added) | - |
| | Sample 5-4 (product of the present invention added) | Sample Product 1 |
| Purple sweet potato color | Sample 5-5 (control: no sunflower seed extract added) | - |
| | Sample 5-6 (product of the present invention added) | Sample Product 1 |
| Purple carrot color | Sample 5-7 (control: no sunflower seed extract added) | - |
| | Sample 5-8 (product of the present invention added) | Sample Product 1 |

(2) Stability Test

(i) Light Resistance Test

The test liquids in plastic bottles were irradiated with white fluorescent light of 10,000 lux at 30°C for 10 days in a fluorescent light irradiation device. A CLH-301 cultivation chamber (Tomy Seiko Co., Ltd.) was used as the fluorescent light irradiation device.

(ii) Heat Resistance Test

The test liquids in plastic bottles were stored in a dark place under a high temperature of 50°C for 10 days.

(iii) Calculation of the Pigment Retention Rate

Color of the transmitted light at the measurement wavelength of 380 to 780 nm was measured for samples before and after the stability test using a spectrophotometer (V-560, JASCO Corporation; the optical path length of the measurement cell: 1 cm), and the pigment retention rates of anthocyanin pigments were calculated by Formula (4) described above. The results are shown in Tables 6 and 7 and FIGS. 3 and 4.

The results showed that, in the beverages with no sunflower seed extract added (Samples 5-1, 5-3, 5-5 and 5-7), which were the controls, the pigment retention rate fell drastically due to storage under light irradiation or a high temperature.

In contrast, in the beverages with the sample product of the sunflower seed extract according to the present invention added (Samples 5-2, 5-4, 5-6 and 5-8), the pigment retention rate remained high even after the strong light irradiation or the storage under a high temperature as described above.

These results show that, in terms of stability of natural colorants, the *Helianthus* seed extract according to the present invention is a composition that has a function of imparting both light resistance and heat resistance. The fact that a sunflower seed extract has a function of imparting heat resistance to natural colorants is a new discovery accomplished for the first time in this Example.

Table 6

| Colorant | Sample | Pigment retention rate (%) | |
|---|---|---|---|
| | | After the light resistance test at 10,000 lux for 5 days | After the light resistance test at 10,000 lux for 10 days |
| Red radish color | Sample5-1 (control: no sunflower seed extract added) | 73.4 | 36.8 |
| | Sample5-2 (product of the present invention added) | 89.3 | 70.3 |
| Red cabbage color | Sample5-3 (control: no sunflower seed extract added) | 73.2 | 41.8 |
| | Sample5-4 (product of the present invention added) | 96.7 | 75.9 |
| Purple sweet potato color | Sample5-5 (control: no sunflower seed extract added) | 64.1 | 45.1 |
| | Sample5-6 (product of the present invention added) | 98.3 | 89.3 |
| Purple carrot color | Sample5-7 (control: no sunflower seed extract added) | 54.2 | 26.8 |
| | Sample5-8 (product of the present invention added) | 86.4 | 75.5 |

Table 7

| Colorant | Sample | Pigment retention rate (%) | |
|---|---|---|---|
| | | After the heat resistance test at 50°C for 5 days | After the heat resistance test at 50°C for 10 days |
| Red radish color | Sample5-1 (control: no sunflower seed extract added) | 51.1 | 21.6 |
| | Sample5-2 (product of the present invention added) | 83.7 | 71.2 |

(continued)

| Colorant | Sample | Pigment retention rate (%) | |
|---|---|---|---|
| | | After the heat resistance test at 50°C for 5 days | After the heat resistance test at 50°C for 10 days |
| Red cabbage color | Sample5-3 (control: no sunflower seed extract added) | 65.6 | 38.6 |
| | Sample5-4 (product of the present invention added) | 90.7 | 80.3 |
| Purple sweet potato color | Sample5-5 (control: no sunflower seed extract added) | 75.4 | 57.9 |
| | Sample5-6 (product of the present invention added) | 95.6 | 93.2 |
| Purple carrot color | Sample5-7 (control: no sunflower seed extract added) | 59.0 | 37.0 |
| | Sample5-8 (product of the present invention added) | 82.8 | 71.1 |

Example 6: "Experiment on the Function of Imparting Stability to Natural Colorants (Color Change)"

Evaluation of the function of imparting stability to natural colorants was conducted on the sunflower seed extract which was prepared in the example above as a *Helianthus* seed extract. Evaluation of color change of the anthocyanin pigment, a natural colorant, was employed as the evaluation method.

(1) Preparation of the Beverage

Plastic bottle beverages were prepared as the test liquids in the same manner as the method described in Example 5 (1).

(2) Stability Test

(i) Light Resistance Test

The test liquids in plastic bottles were irradiated with white fluorescent light of 10,000 lux at 30°C for 10 days in a fluorescent light irradiation device. A CLH-301 cultivation chamber (Tomy Seiko Co., Ltd.) was used as the fluorescent light irradiation device.

(ii) Heat Resistance Test

The test liquids in plastic bottles were stored in a dark place under a high temperature of 50°C for 10 days.

(iii) Evaluation of Colors

Color of the transmitted light at the measurement wavelength of 380 to 780 nm was measured for the test liquids prepared using a spectrophotometer (V-560, JASCO Corporation; the optical path length of the measurement cell: 1 cm), and the tristimulus values of the Hunter Lab colorimetric system (L, a and b values) were measured. Based on the values obtained, the $\Delta E$ value (Formula (6) mentioned above) was calculated as the color difference between the test liquids before and after the stability test. The results are shown in Tables 8 and 9 and FIGS. 5 and 6.

The results showed that, in the beverages with no sunflower seed extract added (Samples 6-1, 6-3, 6-5 and 6-7), which were the controls, the $\Delta E$ values were high, indicating severe color changes, due to storage under light irradiation or a high temperature.

In contrast, in the beverages with the sample product of the sunflower seed extract according to the present invention added (Samples 6-2, 6-4, 6-6 and 6-8), the $\Delta E$ values remained low, indicating that color change was reduced compared with the controls, even after the strong light irradiation or the storage under a high temperature as described above.

These results confirmed the function of imparting stability to natural colorants exhibited by the *Helianthus* seed extract according to the present invention even from the viewpoint of color change.

Table 8

| Colorant | Sample | ΔE value between before and after the stability test | |
|---|---|---|---|
| | | After the light resistance test at 10,000 lux for 5 days | After the light resistance test at 10,000 lux for 10 days |
| Red radish color | Sample 6-1 (control: no sunflower seed extract added) | 4.4 | 11.4 |
| | Sample 6-2 (product of the present invention added) | 1.7 | 5.2 |
| Red cabbage color | Sample 6-3 (control: no sunflower seed extract added) | 5.8 | 13.2 |
| | Sample 6-4 (product of the present invention added) | 0.8 | 5.4 |
| Purple sweet potato color | Sample 6-5 (control: no sunflower seed extract added) | 8.3 | 12.9 |
| | Sample 6-6 (product of the present invention added) | 0.6 | 2.7 |
| Purple carrot color | Sample 6-7 (control: no sunflower seed extract added) | 10.1 | 16.5 |
| | Sample 6-8 (product of the present invention added) | 2.8 | 5.3 |

Table 9

| Colorant | Sample | ΔE value between before and after the stability test | |
|---|---|---|---|
| | | After the heat resistance test at 50°C for 5 days | After the heat resistance test at 50°C for 10 days |
| Red radish color | Sample 6-1 (control: no sunflower seed extract added) | 8.6 | 14.7 |
| | Sample 6-2 (product of the present invention added) | 3.0 | 5.5 |
| Red cabbage color | Sample 6-3 (control: no sunflower seed extract added) | 7.3 | 13.9 |
| | Sample 6-4 (product of the present invention added) | 1.9 | 4.4 |
| Purple sweet potato color | Sample 6-5 (control: no sunflower seed extract added) | 5.7 | 10.1 |
| | Sample 6-6 (product of the present invention added) | 1.5 | 2.3 |
| Purple carrot color | Sample 6-7 (control: no sunflower seed extract added) | 9.0 | 14.1 |
| | Sample 6-8 (product of the present invention added) | 3.7 | 6.5 |

Example 7: Evaluation of the Function of Imparting Stability to Natural Colorants against LED Light Irradiation

Evaluation of the function of imparting stability to natural colorants against LED light irradiation was conducted on the sunflower seed extract which was prepared in the example above as a *Helianthus* seed extract. The pigment retention rate and color change were measured for the evaluation.

(1) Preparation of the Beverage

An acid-sugar solution that contains the sunflower seed extract in an amount of 0.0041 mass% in terms of the solid content, is 10° Brix (adjusted with 13.3 mass% of 75° Brix high fructose corn syrup), has pH3.0 (adjusted with citric acid anhydrous (0.2 mass%) and trisodium citrate dihydrate) and contains the purple sweet potato color at a content of 0.03 mass% when calculated in terms of color value ($E^{10\%}{}_{1cm}$) 80 was prepared. The solution was heated up to 93°C for sterilization and hot packed into a 200 mL plastic bottle. The plastic bottle beverage obtained was used as the test liquid.
Sample Product 2 was used as the sunflower seed extract. The pigment preparation manufactured by San-Ei Gen F.F.I., Inc. was used as the purple sweet potato color. A test liquid with no sunflower seed extract added was also prepared as a control.

(2) Stability Test

(i) Light Resistance Test

The test liquids in plastic bottles were irradiated with white LED light of 10,000 lux at 10°C for 14 days in an LED light irradiation device. A growth chamber (MLR-351H, SANYO Electric Co., Ltd.) was used as the LED light irradiation device.

(ii) Calculation of the Pigment Retention Rate

The pigment retention rate of the sample after the stability test relative to the sample before the stability test was calculated in the same manner as described in Example 5 (2). The results are shown in Table 10 and FIG. 7.

(iii) Evaluation of Colors

In the same manner as described in Example 6 (2), the tristimulus values of the Hunter Lab colorimetric system (L, a and b values) were measured for the test liquids prepared, and the ΔE value, which is the color difference between the test liquids before and after the stability test, was calculated. The results are shown in Table 11 and FIG. 8.
The results showed that, in the beverage with no sunflower seed extract added (Sample 7-1), which was the control, the pigment retention rate fell drastically due to storage under LED light irradiation. The ΔE value was also high, indicating a severe color change.
In contrast, in the beverage with the sample product of the sunflower seed extract according to the present invention added (Sample7-2), the pigment retention rate remained high even after the strong LED light irradiation as described above. Moreover, the ΔE value was low, indicating that the extent of color change was reduced compared with the control.
These results show that the *Helianthus* seed extract according to the present invention is a composition that can impart sufficient light resistance to natural colorants and can realize excellent retention of colors even after a long-term irradiation of white LED light.
Examples 5 and 6 described above showed that the *Helianthus* seed extract (Sample Product 1) produced by using aqueous alcohol as the extraction solvent had a function of imparting stability to natural colorants. Meanwhile, the results of Example 7 confirmed that the extract (Sample Product 2) produced by using water as the extraction solvent also had a function of imparting stability to natural colorants.

Table 10

| Colorant | Sample | Pigment retention rate (%) | |
|---|---|---|---|
| | | After the light resistance test at 10,000 lux for 7 days | After the light resistance test at 10,000 lux for 14 days |
| Purple sweet potato color | Sample 7-1 (control: no sunflower seed extract added) | 66.5 | 54.3 |
| | Sample 7-2 (product of the present invention added) | 82.1 | 72.5 |

Table 11

| Colorant | Sample | ΔE value between before and after the stability test | |
|---|---|---|---|
| | | After the light resistance test at 10,000 lux for 7 days | After the light resistance test at 10,000 lux for 14 days |
| Purple sweet potato color | Sample 7-1 (control: no sunflower seed extract added) | 7.6 | 10.5 |
| | Sample 7-2 (product of the present invention added) | 4.2 | 6.4 |

Example 8: Evaluation of the Function of Imparting Stability to Natural Colorants in Carbonated Drinks

Evaluation of the function of imparting stability to natural colorants was conducted on a carbonated drink containing the sunflower seed extract which was prepared in the example above as a *Helianthus* seed extract. The pigment retention rate and color change were measured for the evaluation.

(1) Preparation of the Beverage

A syrup was prepared so that the final concentrations and properties of the carbonated drink would be as follows. That is, each component was added so that, in the carbonated drink to be prepared, the sunflower seed extract content would be 0.0075 mass% in terms of the solid content, Brix would be 10° (adjusted with 13.3 mass% of 75° Brix high fructose corn syrup), the pH would be 3.0 (adjusted with citric acid anhydrous (0.2 mass%) and trisodium citrate dihydrate), and the content of the purple sweet potato color would be 0.03 mass% when calculated in terms of color value ($E^{10\%}_{1cm}$) 80. Then, the syrup obtained was heated up to 93°C for sterilization, and 40 parts of the syrup and 60 parts of carbonated water were mixed. The mixture was packed into a 280 mL plastic bottle, and the plastic bottle beverage obtained was used as the test liquid.

Sample Product 2 was used as the sunflower seed extract. The pigment preparation manufactured by San-Ei Gen F.F.I., Inc. was used as the purple sweet potato color. A test liquid with no sunflower seed extract added was also prepared as a control.

(2) Stability Test

(i) Light Resistance Test

The test liquids in plastic bottles were irradiated with white fluorescent light of 10,000 lux at 10°C for 14 days in a fluorescent light irradiation device. A CLH-301 cultivation chamber (Tomy Seiko Co., Ltd.) was used as the fluorescent light irradiation device.

(ii) Heat Resistance Test

The test liquids in plastic bottles were stored in a dark place under a high temperature of 50°C for 14 days.

(iii) Calculation of the Pigment Retention Rate

The pigment retention rate of the sample after the stability test relative to the sample before the stability test was calculated in the same manner as described in Example 5 (2). The results are shown in Tables 12 and 13 and FIG. 9.

(iv) Evaluation of Colors

In the same manner as described in Example 6 (2), the tristimulus values of the Hunter Lab colorimetric system (L, a and b values) were measured for the test liquids prepared, and the ΔE value, which is the color difference between the test liquids before and after the stability test, was calculated. The results are shown in Tables 14 and 15 and FIG. 10.

The results showed that, in the carbonated drink with no sunflower seed extract added (Sample 8-1), which was the control, the pigment retention rate fell drastically due to storage under light irradiation or a high temperature. The ΔE value was also high, indicating a severe color change.

In contrast, in the carbonated drink with the sample product of the sunflower seed extract according to the present invention added (Sample 8-2), the pigment retention rate remained high even after the strong light irradiation or the storage under a high temperature as described above. Moreover, the ΔE value was low, indicating that the extent of color change was reduced compared with the control.

These results show that the *Helianthus* seed extract according to the present invention is a composition that can impart sufficient stability to natural colorants and can realize excellent retention of colors even when added to a carbonated drink, in which natural colorants tend to fade easily.

Table 12

| Colorant | Sample | Pigment retention rate (%) | |
|---|---|---|---|
| | | After the light resistance test at 10,000 lux for 7 days | After the light resistance test at 10,000 lux for 14 days |
| Purple sweet potato color | Sample 8-1 (control: no sunflower seed extract added) | 72.1 | 51.4 |
| | Sample 8-2 (product of the present invention added) | 98.3 | 85.0 |

Table 13

| Colorant | Sample | Pigment retention rate (%) | |
|---|---|---|---|
| | | After the heat resistance test at 50°C for 7 days | After the heat resistance test at 50°C for 14 days |
| Purple sweet potato color | Sample 8-1 (control: no sunflower seed extract added) | 52.7 | 26.4 |
| | Sample 8-2 (product of the present invention added) | 89.9 | 76.7 |

Table 14

| Colorant | Sample | ΔE value between before and after the stability test | |
|---|---|---|---|
| | | After the light resistance test at 10,000 lux for 7 days | After the light resistance test at 10,000 lux for 14 days |
| Purple sweet potato color | Sample 8-1 (control: no sunflower seed extract added) | 6.5 | 10.8 |
| | Sample 8-2 (product of the present invention added) | 2.0 | 3.8 |

Table 15

| Colorant | Sample | ΔE value between before and after the stability test | |
|---|---|---|---|
| | | After the heat resistance test at 50°C for 7 days | After the heat resistance test at 50°C for 14 days |
| Purple sweet potato color | Sample 8-1 (control: no sunflower seed extract added) | 10.3 | 16.5 |
| | Sample 8-2 (product of the present invention added) | 2.6 | 5.4 |

Example 9: Evaluation of the Function of Imparting Stability to Natural Colorants in Alcoholic Drinks

Evaluation of the function of imparting stability to natural colorants was conducted on an alcoholic drink containing the sunflower seed extract which was prepared in the example above as a *Helianthus* seed extract. The pigment retention rate and color change were measured for the evaluation.

(1) Preparation of the Beverage

An alcoholic solution that contains 0.0041 mass% of sunflower seed extract in terms of the solid content, is 10° Brix (adjusted with 13.3 mass% of 75° Brix high fructose corn syrup), has pH3.0 (adjusted with citric acid anhydrous (0.2 mass%) and trisodium citrate dihydrate), contains 5% ethanol (v/v) and contains the purple sweet potato color at a content of 0.03 mass% when calculated in terms of color value ($E^{10\%}_{1cm}$) 80 was prepared. The solution was packed into a 200 mL glass bottle and subjected to heat sterilization at 70°C for 10 minutes. The glass bottle beverage obtained was used as the test liquid.

Sample Product 2 was used as the sunflower seed extract. The pigment preparation manufactured by San-Ei Gen F.F.I., Inc. was used as the purple sweet potato color. A test liquid with no sunflower seed extract added was also prepared as a control.

(2) Stability Test

(i) Light Resistance Test

Test liquids in the glass bottles were irradiated with white fluorescent light of 10,000 lux at 10°C for 10 days in a fluorescent light irradiation device. A CLH-301 cultivation chamber (Tomy Seiko Co., Ltd.) was used as the fluorescent light irradiation device.

(ii) Heat Resistance Test

Test liquids in the glass bottles were stored in a dark place under a high temperature of 50°C for 10 days.

(iii) Calculation of the Pigment Retention Rate

The pigment retention rate of the sample after the stability test relative to the sample before the stability test was calculated in the same manner as described in Example 5 (2). The results are shown in Tables 16 and 17 and FIG. 11.

(iv) Evaluation of Colors

In the same manner as described in Example 6 (2), the tristimulus values of the Hunter Lab colorimetric system (L, a and b values) were measured for the test liquids prepared, and the ΔE value, which is the color difference between the test liquids before and after the stability test, was calculated. The results are shown in Tables 18 and 19 and FIG. 12.

The results showed that, in the alcoholic drink with no sunflower seed extract added (Sample 9-1), which was the control, the pigment retention rate fell drastically due to storage under light irradiation or a high temperature. The ΔE value was also high, indicating a severe color change.

In contrast, in the carbonated drink with the sample product of the sunflower seed extract according to the present invention added (Sample 9-2), the pigment retention rate remained high even after the strong light irradiation or the storage under a high temperature as described above. Moreover, the ΔE value was low, indicating that the extent of color change was reduced compared with the control.

These results show that the *Helianthus* seed extract according to the present invention is a composition that can impart sufficient stability to natural colorants and can realize excellent retention of colors even when added to an alcoholic drink, in which natural colorants tend to fade easily.

Table 16

| Colorant | Sample | Pigment retention rate (%) | |
|---|---|---|---|
| | | After the light resistance test at 10,000 lux for 5 days | After the light resistance test at 10,000 lux for 10 days |
| Purple sweet potato color | Sample 9-1 (control: no sunflower seed extract added) | 89.4 | 66.8 |
| | Sample 9-2 (product of the present invention added) | 100.4 | 82.9 |

Table 17

| Colorant | Sample | Pigment retention rate (%) | |
|---|---|---|---|
| | | After the heat resistance test at 50°C for 5 days | After the heat resistance test at 50°C for 10 days |
| Purple sweet potato color | Sample 9-1 (control: no sunflower seed extract added) | 64.9 | 38.8 |
| | Sample 9-2 (product of the present invention added) | 89.6 | 76.1 |

Table 18

| Colorant | Sample | ΔE value between before and after the stability test | |
|---|---|---|---|
| | | After the light resistance test at 10,000 lux for 5 days | After the light resistance test at 10,000 lux for 10 days |
| Purple sweet potato color | Sample 9-1 (control: no sunflower seed extract added) | 2.5 | 7.9 |
| | Sample 9-2 (product of the present invention added) | 0.3 | 3.9 |

Table 19

| Colorant | Sample | ΔE value between before and after the stability test | |
|---|---|---|---|
| | | After the heat resistance test at 50°C for 5 days | After the heat resistance test at 50°C for 10 days |
| Purple sweet potato color | Sample 9-1 (control: no sunflower seed extract added) | 8.7 | 16.0 |
| | Sample 9-2 (product of the present invention added) | 2.6 | 5.7 |

Example 10: "Experiment on the Function of Imparting Stability to Natural Colorants (Gardenia Pigment)"

Evaluation of the function of imparting stability to the gardenia pigment, a natural colorant, was conducted on the sunflower seed extract which was prepared in the example above as a *Helianthus* seed extract. The pigment retention rate and color change were measured for the evaluation.

(1) Preparation of the Beverage

An acid-sugar solution that contains sunflower seed extract in an amount of 0.0041 mass% in terms of the solid content, is 10° Brix (adjusted with 13.3 mass% of 75° Brix high fructose corn syrup), has pH3.5 (adjusted with citric acid anhydrous (0.2 mass%) and trisodium citrate dihydrate) and contains the gardenia pigment at a content of 0.045 mass% when calculated in terms of color value ($E^{10\%}_{1cm}$) 50.5 was prepared. The solution was heated up to 93°C for sterilization and hot packed into a 200 mL plastic bottle. The plastic bottle beverage obtained was used as the test liquid.

Sample Product 2 was used as the sunflower seed extract. The pigment preparation manufactured by San-Ei Gen F.F.I., Inc. was used as the gardenia pigment. A test liquid with no sunflower seed extract added was also prepared as a control.

(2) Stability Test

(i) Light Resistance Test

The test liquids in plastic bottles were irradiated with white fluorescent light of 10,000 lux at 30°C for 6 days in a fluorescent light irradiation device. A CLH-301 cultivation chamber (Tomy Seiko Co., Ltd.) was used as the fluorescent light irradiation device.

(ii) Heat Resistance Test

The test liquids in plastic bottles were stored in a dark place under a high temperature of 50°C for 10 days.

(iii) Calculation of the Pigment Retention Rate

The pigment retention rate of the sample after the stability test relative to the sample before the stability test was calculated in the same manner as described in Example 5 (2). The results are shown in Tables 20 and 21 and FIG. 13.

(iv) Evaluation of Colors

In the same manner as described in Example 6 (2), the tristimulus values of the Hunter Lab colorimetric system (L, a and b values) were measured for the test liquids prepared, and the ΔE value, which is the color difference between the test liquids before and after the stability test, was calculated. The results are shown in Tables 22 and 23 and FIG. 14.

The results showed that, in the beverage with no sunflower seed extract added (Sample 10-1), which was the control, the pigment retention rate fell drastically due to storage under light irradiation or a high temperature. The ΔE value was also high, indicating a severe color change.

In contrast, in the beverage with the sample product of the sunflower seed extract according to the present invention added (Sample 10-2), the pigment retention rate remained high even after the strong light irradiation or the storage under a high temperature as described above. Moreover, the ΔE value was low, indicating that the extent of color change was reduced compared with the control.

These results show that, in terms of stability of the gardenia pigment, which is a natural colorant, the *Helianthus* seed extract according to the present invention is a composition that has a function of imparting both light resistance and heat resistance. Especially, the results indicate advantageous effects on the function of imparting stability against heat.

Table 20

| Colorant | Sample | Pigment retention rate (%) | |
|---|---|---|---|
| | | After the light resistance test at 10,000 lux for 3 days | After the light resistance test at 10,000 lux for 6 days |
| Gardenia red pigment | Sample 10-1 (control: no sunflower seed extract added) | 54.5 | 42.0 |
| | Sample 10-2 (product of the present invention added) | 63.9 | 52.8 |

Table 21

| Colorant | Sample | Pigment retention rate (%) | |
|---|---|---|---|
| | | After the heat resistance test at 50°C for 5 days | After the heat resistance test at 50°C for 10 days |
| Gardenia red pigment | Sample 10-1 (control: no sunflower seed extract added) | 64.6 | 58.2 |
| | Sample 10-2 (product of the present invention added) | 84.7 | 84.0 |

Table 22

| Colorant | Sample | ΔE value between before and after the stability test | |
|---|---|---|---|
| | | After the light resistance test at 10,000 lux for 3 days | After the light resistance test at 10,000 lux for 6 days |
| Gardenia red pigment | Sample 10-1 (control: no sunflower seed extract added) | 11.0 | 14.2 |
| | Sample 10-2 (product of the present invention added) | 9.1 | 11.9 |

Table 23

| Colorant | Sample | ΔE value between before and after the stability test | |
|---|---|---|---|
| | | After the heat resistance test at 50°C for 5 days | After the heat resistance test at 50°C for 10 days |
| Gardenia red pigment | Sample 10-1 (control: no sunflower seed extract added) | 9.3 | 11.6 |
| | Sample 10-2 (product of the present invention added) | 4.6 | 4.2 |

Example 11: "Experiment on the Function of Imparting Stability to Flavoring Compounds (Odor Analysis)"

Evaluation of the function of imparting stability to flavoring compounds was conducted on the sunflower seed extract which was prepared in the example above as a *Helianthus* seed extract. GC/MS analysis of the compounds causing degradation smell of the citral, a flavoring compound, was employed as the evaluation method.

(1) Preparation of the Beverage

An acid-sugar solution that contains sunflower seed extract in an amount of 0.0075 mass% in terms of the solid content, is 10° Brix (adjusted with 13.3 mass% of 75° Brix high fructose corn syrup), has pH3.0 (adjusted with citric acid anhydrous (0.2 mass%) and trisodium citrate dihydrate) and contains citral in an amount of 0.01 mass% was prepared. The solution was heated up to 93°C for sterilization and hot packed into a 200 mL plastic bottle. The plastic bottle beverage obtained was used as the test liquid.

Sample Product 2 was used as the sunflower seed extract. A test liquid with no sunflower seed extract added was also prepared as a control.

(2) Stability Test

(i) Light Resistance Test

The test liquids in plastic bottles were irradiated with white fluorescent light of 10,000 lux at 10°C for 10 days in a fluorescent light irradiation device. A CLH-301 cultivation chamber (Tomy Seiko Co., Ltd.) was used as the fluorescent light irradiation device.

(ii) Heat Resistance Test

The test liquids in plastic bottles were stored in a dark place under a high temperature of 50°C for 7 days.

(iii) GC/MS Analysis

The suppression of occurrence of degradation smell of citral was evaluated by conducting GS/MS analysis of the p-cresol and p-methylacetophenone contents. A gas chromatograph mass spectrometer (Agilent 5975 inert GC/MS System, Agilent Technologies, Inc.) was employed for GC/MS analysis. The results are shown in Tables 24 and 25 and FIGS. 15 and 16.

The results showed that, in the beverage with no sunflower seed extract added (Sample 11-1), which was the control, the content of p-cresol, an odor component having a disinfectant-like odor derived from the citral, greatly increased up to 95.4 ppb due to storage under a high temperature. Also, the content of p-methylacetophenone, which is an odor component having an almond-like odor, significantly rose up to 1,900 ppb. Since the p-cresol and p-methylacetophenone contents were very low before the storage under a high temperature, it was indicated that these compounds were generated with the passage of time due to the storage under a high temperature.

In contrast, in the beverage with the sample product of the sunflower seed extract according to the present invention added (Sample 11-2), the p-cresol content after the storage under the high temperature condition mentioned above was only 7.4 ppb, indicating that the generation of p-cresol was greatly reduced to approximately 7.75 % of the control.

Here, when the p-cresol content in Sample 11-2 after the storage under a high temperature is converted to a value that can be compared with the conventional technique disclosed in Experimental Example 1 of Patent Document 4 (a heat resistance test at 50°C for 7 days, which is the same condition as the present example), the value is 7.75. In contrast, the minimum value of the p-cresol generation after the storage under a high temperature is 17.1 when the conventional product disclosed in Patent Document 4 is added. Therefore, it is indicated that the generation of p-cresol in the beverage with the sample product of the sunflower seed extract according to the present invention added is significantly reduced compared with the conventional product disclosed in Patent Document 4.

Also, in the beverage with the sample product of the sunflower seed extract according to the present invention added (Sample 11-2), the p-methylacetophenone content was 627 ppb. It was indicated that even the generation of p-methylacetophenone was reduced to approximately 33.0 % of the control (Sample 11-1).

These results show that the *Helianthus* seed extract according to the present invention is a composition that has a function of imparting to citral, a flavoring compound, stability as a compound. Especially, it is shown that, while the function to suppress p-cresol generation is insufficient in the conventional technique for suppressing the generation of degradation smell derived from the citral disclosed in Patent Document 4, the *Helianthus* seed extract according to the present invention is a composition that can greatly reduce the generation of p-cresol.

Table 24

| Sample | p-Cresol content (ppb) | | |
|---|---|---|---|
| | Before the stability test | After the light resistance test at 10,000 lux for 10 days | After the heat resistance test at 50°C for 7 days |
| Sample 11-1 (control: no sunflower seed extract added) | 1.3 | 1.1 | 95.4 |
| Sample 11-2 (product of the present invention added) | 1.5 | 1.3 | 7.4 |

Table 25

| Sample | p-Methylacetophenone content (ppb) | | |
|---|---|---|---|
| | Before the stability test | After the light resistance test at 10,000 lux for 10 days | After the heat resistance test at 50°C for 7 days |
| Sample 11-1 (control: no sunflower seed extract added) | 11.9 | 27.6 | 1900 |
| Sample 11-2 (product of the present invention added) | 3.9 | 10.2 | 627 |

Example 12: "Experiment on the Function of Imparting Stability to Flavoring Compounds (Sensory Evaluation)"

Evaluation of the function of imparting stability to flavoring compounds was conducted on the sunflower seed extract which was prepared in the example above as a *Helianthus* seed extract. Sensory evaluation by panelists was employed as the evaluation method.

(1) Preparation of the Beverage

Plastic bottle beverages were prepared as the test liquids in the same manner as described in Example 11 (1).

(2) Stability Test

(i) Heat Resistance Test

The test liquids in plastic bottles were stored in a dark place under a high temperature of 50°C for 6 days.

(ii) Sensory Evaluation

Blind sensory evaluation on the flavor of the beverages was carried out by eight panelists who were professionally engaged in flavor evaluation. The flavor of the beverage was evaluated at six levels (5 > 4 > 3 > 2 > 1 > 0) based on the goodness of the flavor, where 5 indicates the favorable flavor immediately after the production of the beverage with no sunflower seed extract added. Concretely, the evaluation standard is as follows: 5 (The same as the flavor of the beverage with no sunflower seed extract added (Sample 12-1) immediately after the production), 4 (Slightly different), 3 (Different), 2 (Significantly different), 1 (The same level as the flavor of the beverage with no sunflower seed extract added (Sample 12-1) after the heat resistance test), and 0 (Worse than the flavor of the beverage with no sunflower seed extract added (Sample 12-1) after the heat resistance test). The results are shown in Table 26.

In the beverage with no sunflower seed extract added (Sample 12-1), which was the control, the disinfectant-like odor increased due to storage under a high temperature. In contrast, in the beverages with the sample product of the sunflower seed extract according to the present invention added (Sample 12-2), the occurrence of offensive odor was greatly reduced even after the storage under a high temperature as described above.

It is confirmed even by the results of the sensory evaluation that the *Helianthus* seed extract according to the present invention is a composition that has a function of imparting to citral, which is a flavoring compound, stability as a compound.

Table 26

| Sample | Sensory evaluation | |
|---|---|---|
| | Before the stability test | After the heat resistance test at 50°C for 6 days |
| Sample 12-1 (control: no sunflower seed extract added) | 5 (standard) | 1 (standard) |
| Sample 12-2 (product of the present invention added) | 5.0 | 3.5 |

Example 13: "Experiment on the Function of Imparting Stability to Flavoring Compounds in Carbonated Drinks (Sensory Evaluation)"

Evaluation of the function of imparting stability to flavoring compounds was conducted on a carbonated drink containing the sunflower seed extract which was prepared in the example above as a *Helianthus* seed extract. Sensory evaluation was employed as the evaluation method.

(1) Preparation of a Lemon-Flavored Carbonated Drink

A syrup was prepared so that the final concentrations and properties of the carbonated drink would be as follows. That is, the syrup was prepared so that, in the carbonated drink to be prepared, the sunflower seed extract concentration would be 0.0075 mass% in terms of the solid content, Brix would be 10° (adjusted with 13.3 mass% of 75° Brix high fructose corn syrup), the pH would be 3.0 (adjusted with citric acid anhydrous (0.2 mass%) and trisodium citrate dihydrate), the content of the emulsified flavoring would be 0.02 mass%, the content of the carotene pigment preparation would be 0.00012 mass% in terms of the beta-carotene content, and the content of the lemon flavor would be 0.002 mass% in terms of the citral content. Then, the syrup obtained was heated up to 93°C for sterilization, and 40 parts of the syrup and 60 parts of carbonated water were mixed. The mixture was packed into a 280 mL plastic bottle, and the plastic bottle beverage obtained was used as the test liquid.

Sample Product 1 was used as the sunflower seed extract. The emulsified flavoring, the carotene pigment preparation and the lemon flavor manufactured by San-Ei Gen F.F.I., Inc. were used. A test liquid with no sunflower seed extract added was also prepared as a control.

(2) Stability Test

(i) Light Resistance Test

The test liquids in plastic bottles were irradiated with white fluorescent light of 10,000 lux at 10°C for 10 days in a fluorescent light irradiation device. A CLH-301 cultivation chamber (Tomy Seiko Co., Ltd.) was used as the fluorescent light irradiation device.

(ii) Heat Resistance Test

The test liquids in plastic bottles were stored in a dark place under a high temperature of 50°C for 14 days.

(iii) Sensory Evaluation

Blind sensory evaluation on the flavor of the lemon-flavored carbonated drinks was carried out by eight panelists who were professionally engaged in flavor evaluation. The flavor of the beverage was evaluated at 5 levels (5 > 4 > 3 > 2 > 1) based on the goodness of the flavor, where 5 indicates the favorable flavor immediately after the production of the beverage with no sunflower seed extract added. Concretely, the evaluation standard is as follows: 5 (The same as the flavor of the beverage with no sunflower seed extract added (Sample 13-1) immediately after the production), 4 (Slightly different), 3 (Different), 2 (Significantly different), and 1 (The same level as the flavor of the beverage with no sunflower seed extract added (Sample 13-1) after the stability test (the light resistance test or the heat resistance test)). The results are shown in Tables 27 and 28.

The results showed that, in the lemon-flavored carbonated drink with no sunflower seed extract added (Sample 13-1), which was the control, a disinfectant-like odor was generated, and severe deterioration of flavor was observed due to storage under light irradiation or a high temperature. In contrast, in the carbonated drink with the sample product of the sunflower seed extract according to the present invention added (Sample 13-2), the occurrence of offensive odor was greatly reduced, and the fresh aroma derived from the citral was maintained sufficiently even after the storage under light irradiation or a high temperature as described above.

These results show that the *Helianthus* seed extract according to the present invention is a composition that can impart sufficient stability to flavoring compounds even when added to a carbonated drink.

Here, Examples 11 and 12 described above showed that the *Helianthus* seed extract in which water was employed as the extraction solvent (Sample Product 2) had a function of imparting stability to flavoring compounds. Meanwhile, the results of the present example confirmed that the extract in which aqueous alcohol was employed as the extraction solvent (Sample Product 1) also had the function of imparting stability to flavoring compounds.

Table 27

| Sample | Sensory evaluation | |
|---|---|---|
| | Before the stability test | After the light resistance test at 10,000 lux for 10 days |
| Sample 13-1 (control: no sunflower seed extract added) | 5 (standard) | 1 (standard) |
| Sample 13-2 (product of the present invention added) | 5.0 | 3.5 |

Table 28

| Sample | Sensory evaluation | |
|---|---|---|
| | Before the stability test | After the heat resistance test at 50°C for 14 days |
| Sample 13-1 (control: no sunflower seed extract added) | 5 (standard) | 1 (standard) |
| Sample 13-2 (product of the present invention added) | 5.0 | 4.5 |

Example 14: "Influence of Heat Treatment After Extraction and Filtration on the Odor Characteristics of the *Helianthus* Seed Extract (Sensory Evaluation)"

Evaluation of the change of flavor with the passage of time was conducted on the *Helianthus* seed extracts produced under different conditions of heat treatment after the extraction and filtration in the production process.

(1) Production of the *Helianthus* Seed Extract

Sunflower seed oil cakes (with seed shell content of less than 10%) were pulverized and extracted with the extraction solvent shown in Tables 29 and 30 in an amount 20 times the weight of the raw material. The extraction was performed by stirring at 60°C for 5 hours. After the extraction, suction filtration was conducted in the same manner as described in Example 1, and the filtrate was collected.

The hydrogen ion concentration of the filtrate obtained was adjusted so that its hydrogen ion concentration ratio in which the hydrogen ion concentration immediately after the extraction ($3.16 \times 10^{-7}$ mol/L) was taken as 1 would be the values shown in Tables 29 and 30, and heat treatment was conducted for 30 minutes at the temperatures shown in Tables 29 and 30. Sulfuric acid was used for the adjustment of the hydrogen ion concentration. Then, the same treatments as described in Example 1 were performed, and sunflower seed extracts concentrated to the solid content of 15 mass% were obtained.

(2) Preparation of the Beverage

An acid-sugar solution that contains the sunflower seed extract in an amount of 0.0075 mass% in terms of the solid content, is 10° Brix (adjusted with 13.3 mass% of 75° Brix high fructose corn syrup), has pH3.0 (adjusted with citric acid anhydrous (0.2 mass%) and trisodium citrate dihydrate) and contains the purple sweet potato color at a content of 0.03 mass% when calculated in terms of color value ($E^{10\%}_{1cm}$) 80 was prepared. The solution was heated up to 93°C for sterilization and hot packed into a 200 mL plastic bottle. The plastic bottle beverages obtained were used as the test liquids.

The concentrated extracts prepared in (1) above and Comparative Product 1 prepared in Example 1 were used as the sunflower seed extracts. The pigment preparation manufactured by San-Ei Gen F.F.I., Inc. was used as the purple sweet potato color. A test liquid with no sunflower seed extract added was also prepared as a control.

(3) Stability Test

(i) Light Resistance Test

The test liquids in plastic bottles were irradiated with white fluorescent light of 10,000 lux at 30°C for 14 days in a fluorescent light irradiation device. A CLH-301 cultivation chamber (Tomy Seiko Co., Ltd.) was used as the fluorescent light irradiation device.

(ii) Sensory Evaluation

Blind sensory evaluation on the flavor of the beverages was carried out by five panelists who were professionally engaged in flavor evaluation. The strength (intensity) of the flavor of the beverage was evaluated at 6 levels ($0 < 1 < 2 < 3 < 4 < 5$), where 0 indicates no smell and 5 indicates strong smell. The results are shown in Tables 29 and 30 and FIG. 17.

The results showed that, in the beverage containing sunflower seed extract obtained by a conventional technique (Sample 14-c1: Comparative Product), a mushroom-like odor increased due to storage under light irradiation, and the occurrence of strong degradation smell was confirmed.

In contrast, the degradation smell after storage under light irradiation was reduced in the beverages containing sunflower seed extracts obtained by conducting heat treatment after extraction and filtration (Samples 14-1 to 14-6). Especially, the degradation smell was greatly reduced in the beverages containing sunflower seed extracts in which heat treatment after extraction was conducted at increased hydrogen ion concentrations (Samples 14-2, 14-3, 14-5 and 14-6). Furthermore, the degradation smell was reduced even more significantly in the beverages containing sunflower seed extracts obtained by conducting heat treatment under a condition in which, when the hydrogen ion concentration immediately after the extraction ($3.16 \times 10^{-7}$ mol/L) was taken as 1, the hydrogen ion concentration ratio was set to $3.16 \times 10^{3}$-fold (Samples 14-3 and 14-6), and the occurrence of degradation smell was suppressed to the same level as that in the beverage with no sunflower seed extract added (Sample 14-c2: control).

These results show that the occurrence of degradation smell with the passage of time can be reduced by further conducting heat treatment after the extraction and filtration in the production process of the *Helianthus* seed extract. It is also indicated that the effects to reduce the degradation smell are enhanced remarkably correlating with the increase in the hydrogen ion concentration at the time of heat treatment. Particularly, it is shown that, in the *Helianthus* seed extract obtained by greatly increasing the hydrogen ion concentration to approximately 3,160-fold relative to the extract solution, the occurrence of degradation smell is remarkably suppressed even after the severe test of storage under light irradiation.

These results show that the heat treatment after the extraction and filtration in the production process of the *Helianthus* seed extract improves the odor characteristics of the compositions composing the *Helianthus* seed extract. In particular, it is indicated that the odor characteristics can be improved remarkably by increasing the hydrogen ion concentration at which the heat treatment is conducted.

Moreover, it is shown that the effects to improve the odor properties can be exhibited regardless of whether the extraction solvent is water or aqueous alcohol.

Table 29

| Beverage | Production condition of the sunflower seed extract | | | Sensory evaluation (odor intensity) after the light resistance test |
|---|---|---|---|---|
| | Extraction condition | Heat treatment after filtration | | |
| | | Ratio of hydrogen ion concentration | Temperature | |
| Sample 14-c1 (Comparative product added) | Solvent: water 80-85°C | - | - | 5.0 |
| Sample 14-1 (Extract added) | Solvent: water 60°C | $1.00 \times 10^{0}$ times | 90°C | 2.8 |
| Sample 14-2 (Extract added) | Solvent: water 60°C | $3.16 \times 10^{2}$ times | 90°C | 1.2 |
| Sample 14-3 (Extract added) | Solvent: water 60°C | $3.16 \times 10^{3}$ times | 90°C | 0.5 |

(continued)

| Beverage | Production condition of the sunflower seed extract | | | Sensory evaluation (odor intensity) after the light resistance test |
|---|---|---|---|---|
| | Extraction condition | Heat treatment after filtration | | |
| | | Ratio of hydrogen ion concentration | Temperature | |
| Sample 14-c2 (control: no extract added) | - | - | - | 0.0 |

Table 30

| Beverage | Production condition of the sunflower seed extract | | | Sensory evaluation (odor intensity) after the light resistance test |
|---|---|---|---|---|
| | Extraction solvent | Heat treatment after filtration | | |
| | | Ratio of hydrogen ion concentration | Temperature | |
| Sample 14-c1 (Comparative product added) | Solvent: water 80-85°C | - | - | 5.0 |
| Sample 14-4 (Extract added) | Solvent: 20% EtOH 60°C | $1.00\times10^0$ times | 80°C | 2.2 |
| Sample 14-5 (Extract added) | Solvent: 20% EtOH 60°C | $3.16\times10^2$ times | 80°C | 1.1 |
| Sample 14-6 (Extract added) | Solvent: 20% EtOH 60°C | $3.16\times10^3$ times | 80°C | 0.5 |
| Sample 14-c2 (control: no extract added) | - | - | - | 0.0 |

Example 15: "Influence of Heat Treatment after Extraction and Filtration on the Function of Imparting Stability to Natural Colorants (Sensory Evaluation)"

Evaluation of the function of imparting stability to natural colorants was conducted on the *Helianthus* seed extract produced under different conditions of heat treatment after the extraction and filtration in the production process. Measurement of the pigment retention rate of the anthocyanin pigment, a natural colorant, was employed as the evaluation method.

(1) Preparation of the Beverage

Plastic bottle beverages were prepared as the test liquids in the same manner as described in Example 14 (2) using the sunflower seed extracts which were obtained by conducting heat treatment after extraction and filtration at higher hydrogen ion concentrations and exhibited favorable results in Example 14.

(2) Stability Test

(i) Light Resistance Test

The test liquids in plastic bottles were irradiated with white fluorescent light of 10,000 lux at 30°C for 14 days in a fluorescent light irradiation device. A CLH-301 cultivation chamber (Tomy Seiko Co., Ltd.) was used as the fluorescent light irradiation device.

(ii) Calculation of the Pigment Retention Rate

The pigment retention rate of the sample after the stability test relative to the sample before the stability test was calculated in the same manner as described in Example 5 (2). The results are shown in Tables 31 and 32 and FIG. 18.

The results showed that, in the beverage with no sunflower seed extract added (Sample 15-cl), which was the control, the pigment retention rate fell drastically due to storage under light irradiation. In contrast, in the beverages containing the sunflower seed extracts obtained by conducting heat treatment after extraction at higher hydrogen ion concentrations (Samples 15-1 to 15-4), the pigment retention rate remained high even after the strong light irradiation as described above.

It was also shown that the function of imparting stability to natural colorants could be exhibited regardless of whether the extraction solvent was water or aqueous alcohol.

These results show that the *Helianthus* seed extract obtained by conducting heat treatment after extraction and filtration in the production process of the *Helianthus* seed extract can become a composition having a function of imparting stability to natural colorants. It is indicated that this function is efficiently exhibited when the hydrogen ion concentration is increased to approximately 316- to 3,160-fold relative to the extract solution.

Table 31

| Beverage | Production condition of the sunflower seed extract | | | Pigment retention rate (%) after the light resistance test |
|---|---|---|---|---|
| | Extraction condition | Heat treatment after filtration | | |
| | | Ratio of hydrogen ion concentration | Temperature | |
| Sample 15-c1 (control: no extract added) | - | - | - | 38.8 |
| Sample 15-1 (Extract added) | Solvent: water | $3.16\times10^2$ times | 90°C | 70.7 |
| Sample 15-2 (Extract added) | Solvent: water 60°C | $3.16\times10^3$ times | 90°C | 78.3 |

Table 32

| Beverage | Production condition of the sunflower seed extract | | | Pigment retention rate (%) after the light resistance test |
|---|---|---|---|---|
| | Extraction condition | Heat treatment after filtration | | |
| | | Ratio of hydrogen ion concentration | Temperature | |
| Sample 15-c1 (control: no extract added) | - | - | - | 38.8 |
| Sample 15-3 (Extract added) | Solvent: 20% EtOH 60°C | $3.16\times10^2$ times | 80°C | 79.3 |
| Sample 15-4 (Extract added) | Solvent: 20% EtOH 60°C | $3.16\times10^3$ times | 80°C | 83.5 |

## Claims

1. A *Helianthus* seed extract having a feature (A) as follows:

   (A) a content of 1-octen-3-one of an acid-sugar solution when stored under white fluorescent light of 10,000 lux at 30°C for 7 days is 0.1 ppb or less, wherein the acid-sugar solution contains the *Helianthus* seed extract in an amount of 0.0075 mass% in terms of a solid content and is adjusted to have 10° Brix by high fructose corn syrup and to have a pH being 3.0.

2. The *Helianthus* seed extract according to claim 1, further having a feature (B) as follows:

(B) when an acid-sugar solution that contains the *Helianthus* seed extract in an amount of 0.0075 mass% in terms of a solid content and is adjusted to have 10° Brix by high fructose corn syrup and to have a pH being 3.0 is prepared,

(b-1) a content of methional of the acid-sugar solution is 0.025 ppb or less; and
(b-2) a content of methional of the acid-sugar solution when stored under white fluorescent light of 10,000 lux at 30°C for 7 days is 0.03 ppb or less.

3. The *Helianthus* seed extract according to claim 1 or 2, further having a feature (C) as follows:

(C) a retention rate of anthocyanin pigments of an acid-sugar solution when stored in a dark place at 50°C for 10 days is 60% or more compared with before storage, wherein the acid-sugar solution contains purple sweet potato color at a content of 0.03 mass% when calculated in terms of color value ($E^{10\%}{}_{1cm}$) 80, contains the *Helianthus* seed extract in an amount of 0.0041 mass% in terms of a solid content and is adjusted to have 10° Brix by high fructose corn syrup and to have a pH being 3.0.

4. The *Helianthus* seed extract according to any one of claims 1 to 3, further having a feature (D) as follows:

(D) a retention rate of anthocyanin pigments of an acid-sugar solution when stored under white LED light of 10,000 lux at 10°C for 14 days is 60% or more compared with before storage, wherein the acid-sugar solution contains purple sweet potato color at a content of 0.03 mass% when calculated in terms of color value ($E^{10\%}{}_{1cm}$) 80, contains the *Helianthus* seed extract in an amount of 0.0041 mass% in terms of a solid content and is adjusted to have 10° Brix by high fructose corn syrup and to have a pH being 3.0.

5. The *Helianthus* seed extract according to any one of claims 1 to 4, further having a feature (E) as follows:

(E) a content of p-cresol of an acid-sugar solution when stored in a dark place at 50°C for 7 days is 20 ppb or less, wherein the acid-sugar solution contains citral in an amount of 0.01 mass%, contains the *Helianthus* seed extract in an amount of 0.0075 mass% in terms of a solid content and is adjusted to have 10° Brix by high fructose corn syrup and to have a pH being 3.0.

6. The *Helianthus* seed extract according to any one of claims 1 to 5, further having a feature (F) as follows:

(F) when an aqueous solution containing the *Helianthus* seed extract in an amount of 0.75 mass% in terms of a solid content is prepared using McIlvaine buffer of pH 3.0, a turbidity ($OD_{700}$ value) of the aqueous solution is 0.2 or less.

7. The *Helianthus* seed extract according to claim 6, having the features (A) to (F).

8. A production method for a *Helianthus* seed extract, comprising:

(step 1) performing aqueous extraction or aqueous alcohol extraction on the *Helianthus* seeds;
(step 2) recovering an extract solution by performing solid-liquid separation after the extraction; and
(step 3) performing heat treatment on the extract solution at 60°C or higher under an acidic condition.

9. The production method according to claim 8,
wherein a hydrogen ion concentration of the extract solution after the aqueous extraction or the aqueous alcohol extraction is $3.16 \times 10^{-8}$ to $1 \times 10^{-6}$ mol/L, and the heat treatment recited in the step 3 is performed under a condition in which, when the hydrogen ion concentration of the extract solution after the aqueous extraction or the aqueous alcohol extraction is taken as 1, a hydrogen ion concentration ratio is set to $1\times10^1$ to $3.16\times10^5$.

10. The production method according to claim 8 or 9,
wherein the *Helianthus* seeds are seeds of plants belonging to *Helianthus annuus,* a seed shell content therein adjusted to 20% or less.

**11.** A *Helianthus* seed extract obtained by the production method according to any one of claims 8 to 10.

**12.** A method for reducing degradation smell of the *Helianthus* seed extract produced, wherein the steps 1 to 3 according to any one of claims 8 to 10 are performed in a production process of the *Helianthus* seed extract.

**13.** An agent for preventing color fading of natural colorants, comprising the *Helianthus* seed extract according to any one of claims 1 to 7 and 11.

**14.** A pigment preparation comprising the *Helianthus* seed extract according to any one of claims 1 to 7 and 11.

**15.** An agent for suppressing degradation smell of flavoring compounds, comprising the *Helianthus* seed extract according to any one of claims 1 to 7 and 11.

**16.** A flavoring preparation comprising the *Helianthus* seed extract according to any one of claims 1 to 7 and 11.

**17.** A food, a beverage, a cosmetic product, a medicine, a quasi-drug, a personal hygiene product, or feed, comprising the *Helianthus* seed extract according to any one of claims 1 to 7 and 11.

**18.** A method for preventing color fading of natural colorants, comprising a step in which the *Helianthus* seed extract according to any one of claims 1 to 7 and 11 is contained.

**19.** A method for suppressing degradation smell of flavoring compounds, comprising a step in which the *Helianthus* seed extract according to any one of claims 1 to 7 and 11 is contained.

**20.** A production method for an agent for preventing color fading of natural colorants, an agent for suppressing degradation smell of flavoring compounds, a pigment preparation, a flavoring preparation, a food, a beverage, a cosmetic product, a medicine, a quasi-drug, a personal hygiene product, or feed, the method comprising a step in which the *Helianthus* seed extract obtained by the production method according to any one of claims 8 to 10 is contained.

*Fig.1*

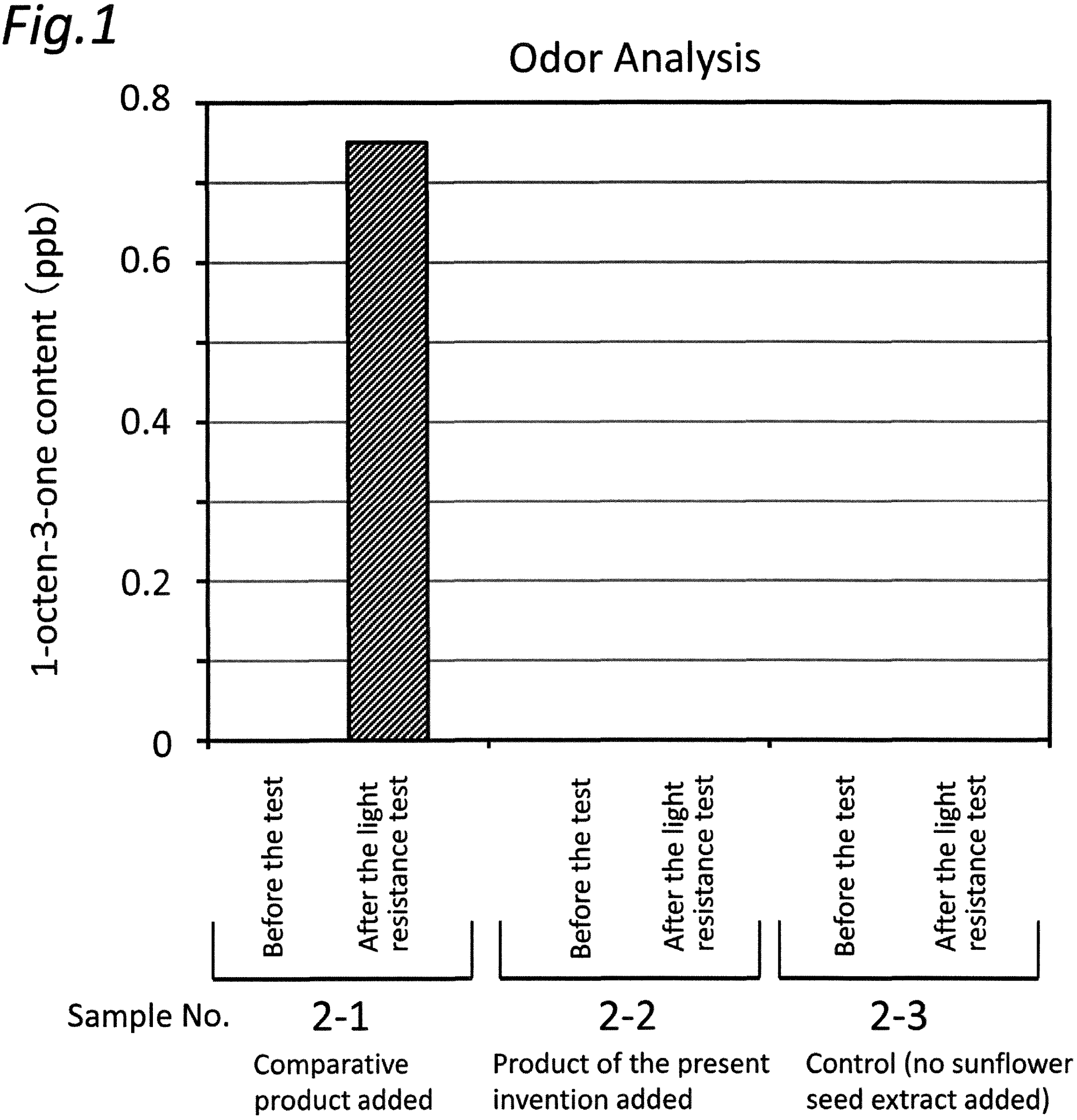
Odor Analysis
1-octen-3-one content (ppb)
0.8
0.6
0.4
0.2
0
Before the test
After the light resistance test
Before the test
After the light resistance test
Before the test
After the light resistance test
Sample No.
2-1
Comparative product added
2-2
Product of the present invention added
2-3
Control (no sunflower seed extract added)

Fig.2

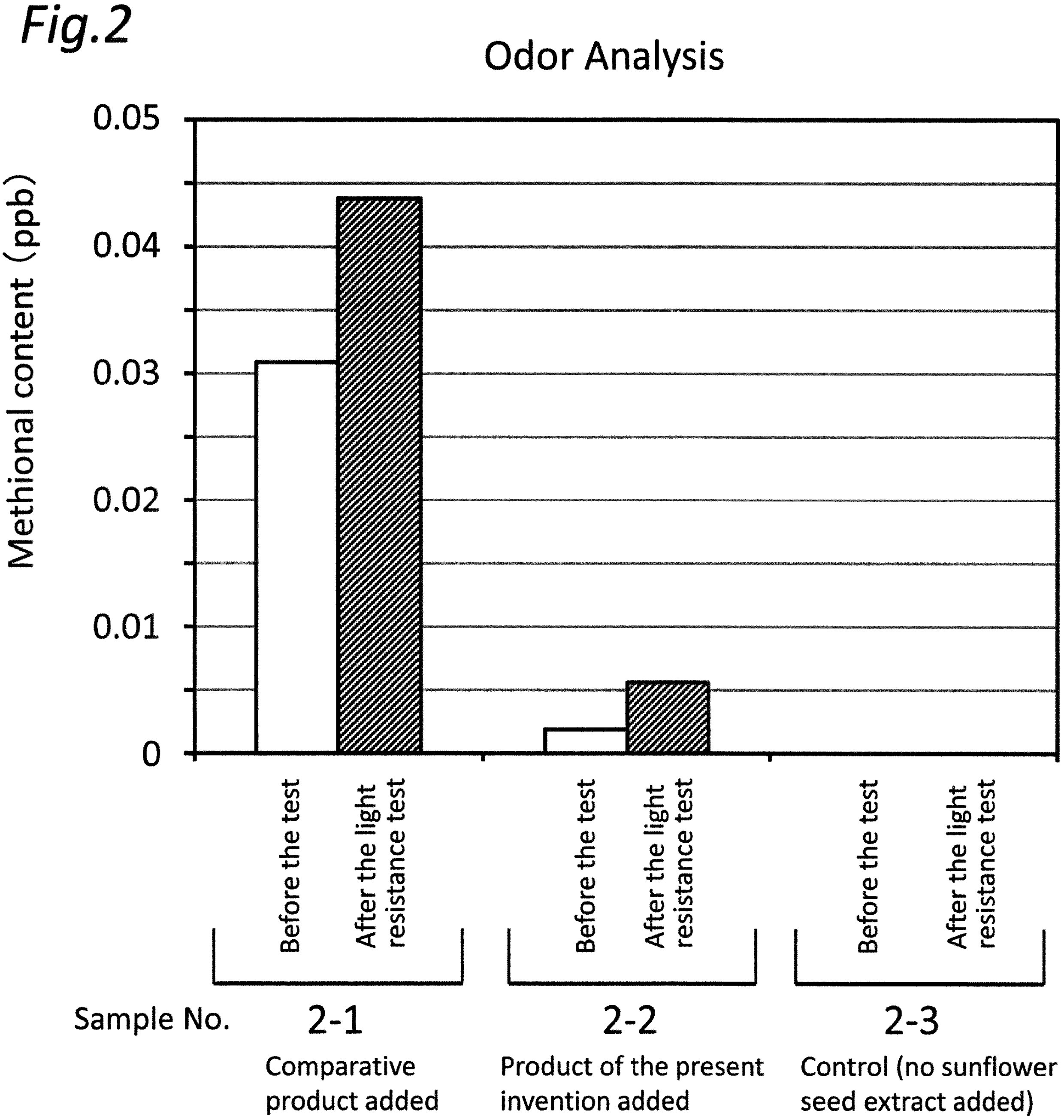
Odor Analysis
Methional content (ppb)
0.05
0.04
0.03
0.02
0.01
0
Before the test
After the light resistance test
Before the test
After the light resistance test
Before the test
After the light resistance test
Sample No.
2-1
Comparative product added
2-2
Product of the present invention added
2-3
Control (no sunflower seed extract added)

*Fig.3*

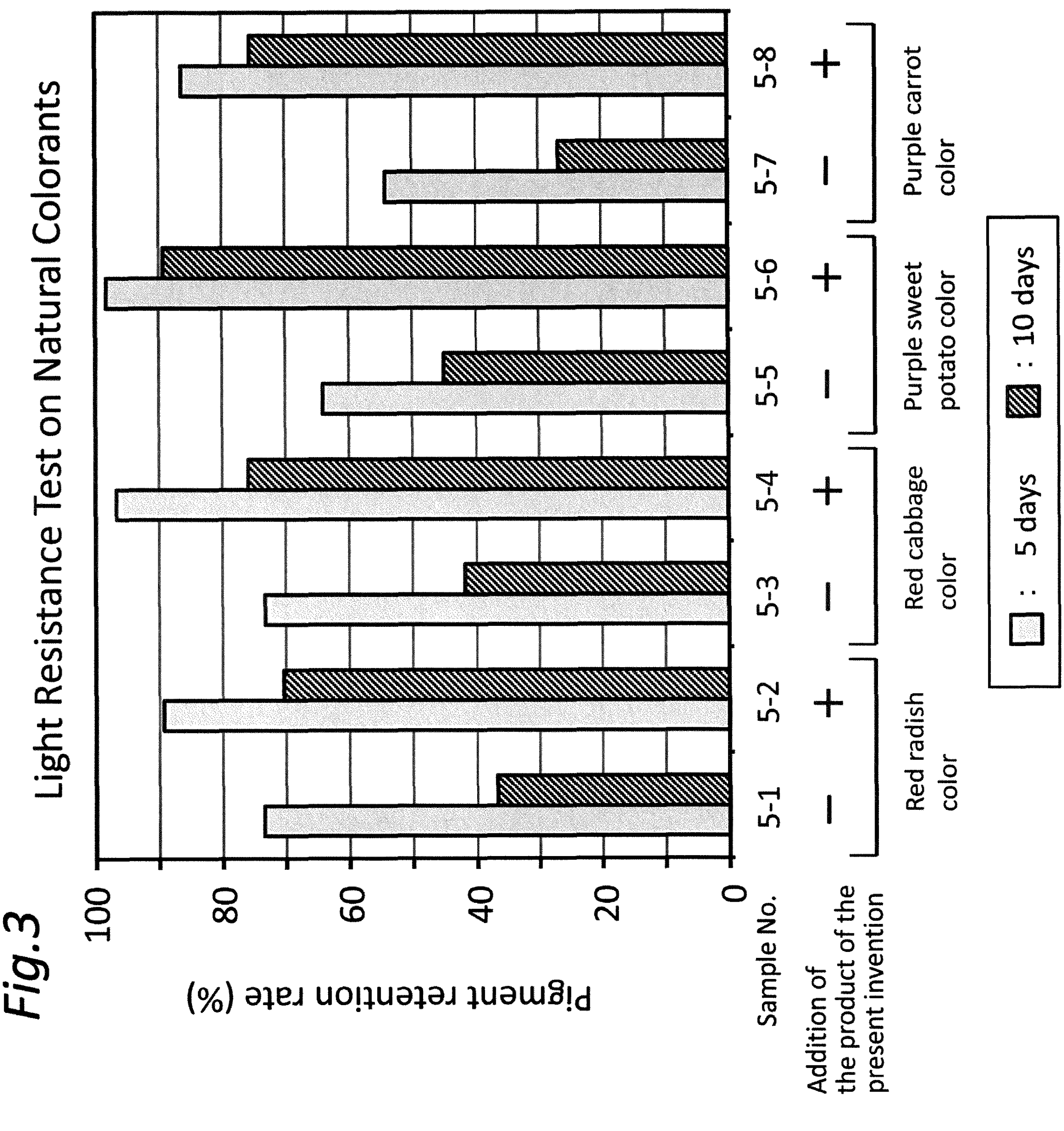
Light Resistance Test on Natural Colorants
Pigment retention rate (%)
100
80
60
40
20
0
Sample No.
5-1
5-2
5-3
5-4
5-5
5-6
5-7
5-8
Addition of the product of the present invention
−
+
−
+
−
+
−
+
Red radish color
Red cabbage color
Purple sweet potato color
Purple carrot color
: 5 days
: 10 days

*Fig.4*

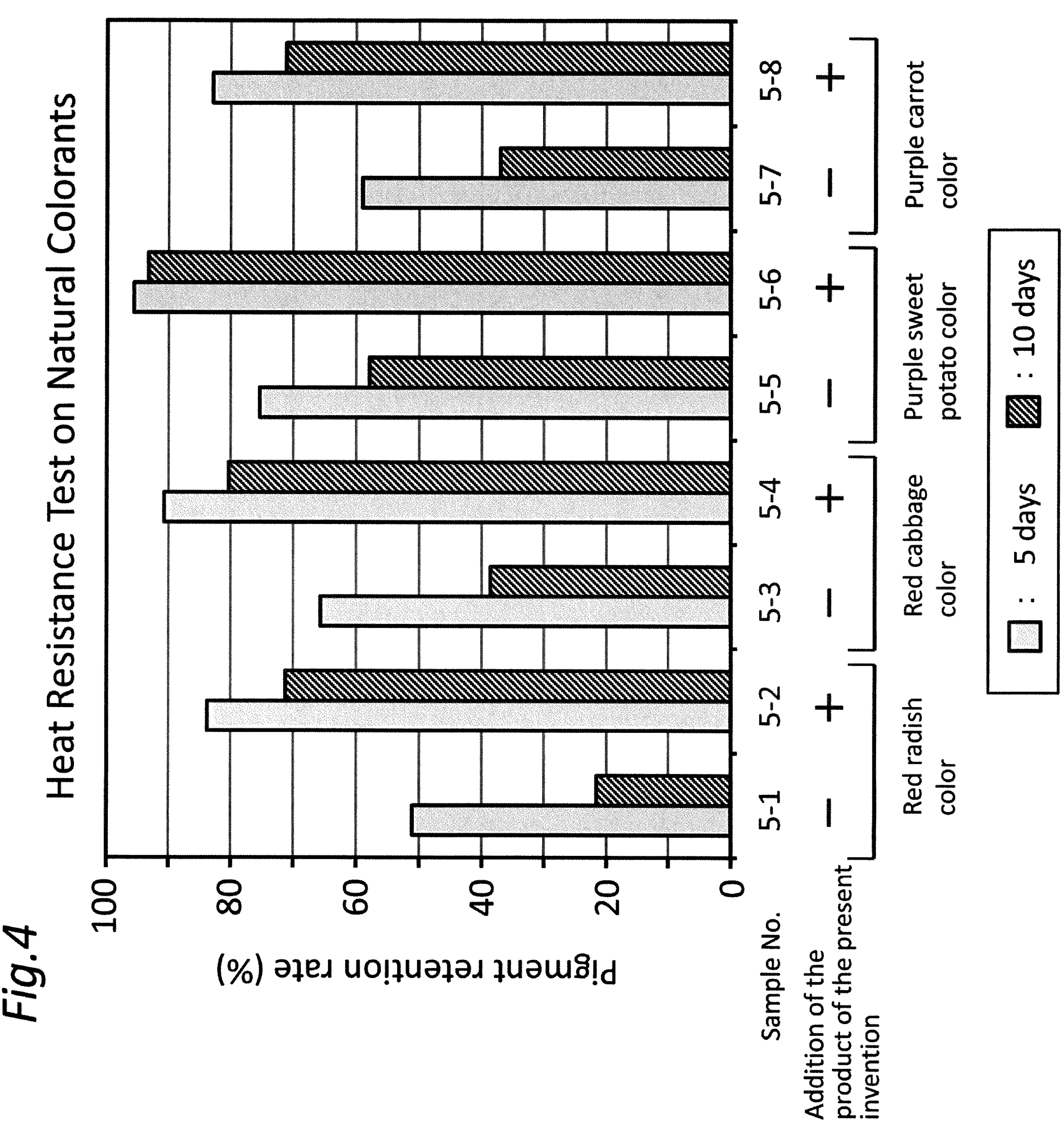
Heat Resistance Test on Natural Colorants
Pigment retention rate (%)
100
80
60
40
20
0
Sample No.
5-1
5-2
5-3
5-4
5-5
5-6
5-7
5-8
Addition of the product of the present invention
−
+
−
+
−
+
−
+
Red radish color
Red cabbage color
Purple sweet potato color
Purple carrot color
: 5 days
: 10 days

*Fig.5*

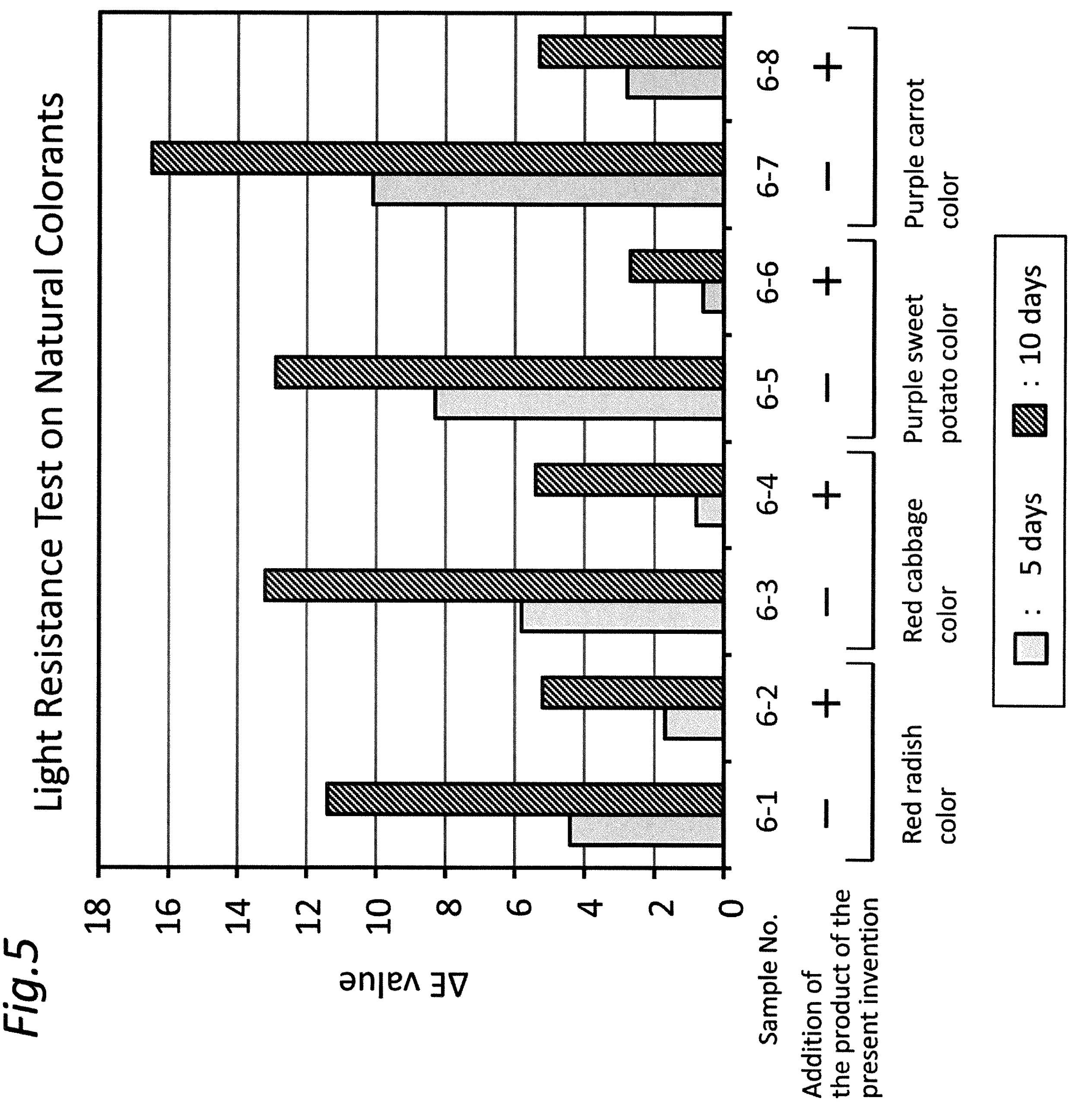

Light Resistance Test on Natural Colorants
ΔE value
18
16
14
12
10
8
6
4
2
0
Sample No.
6-1
6-2
6-3
6-4
6-5
6-6
6-7
6-8
Addition of the product of the present invention
−
+
−
+
−
+
−
+
Red radish color
Red cabbage color
Purple sweet potato color
Purple carrot color
: 5 days
: 10 days

*Fig.6*

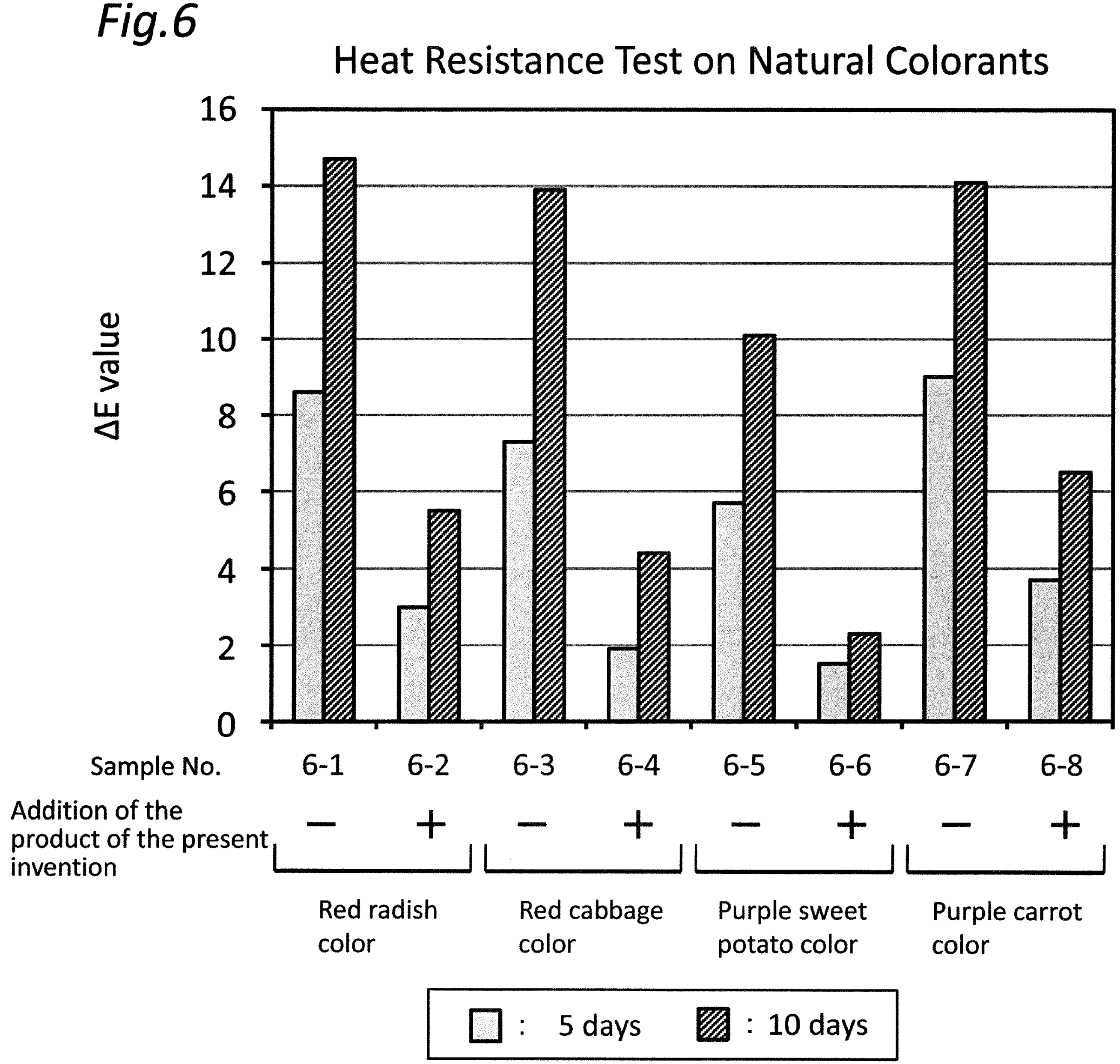
Heat Resistance Test on Natural Colorants
ΔE value
16
14
12
10
8
6
4
2
0
Sample No.
6-1
6-2
6-3
6-4
6-5
6-6
6-7
6-8
Addition of the product of the present invention
−
+
−
+
−
+
−
+
Red radish color
Red cabbage color
Purple sweet potato color
Purple carrot color
: 5 days
: 10 days

*Fig.7*

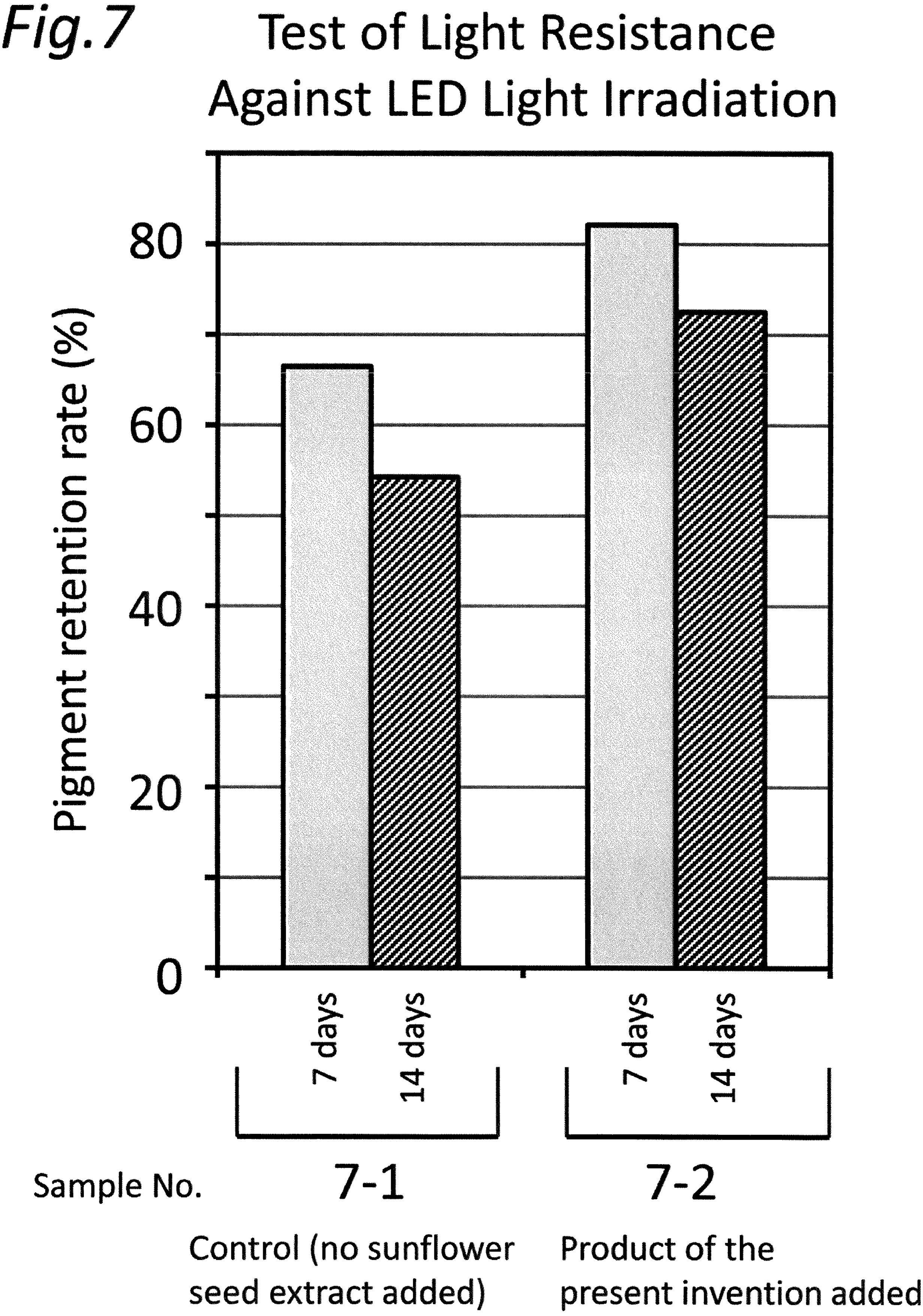
Test of Light Resistance
Against LED Light Irradiation
Pigment retention rate (%)
80
60
40
20
0
7 days
14 days
7 days
14 days
Sample No.
7-1
7-2
Control (no sunflower seed extract added)
Product of the present invention added

Fig.8

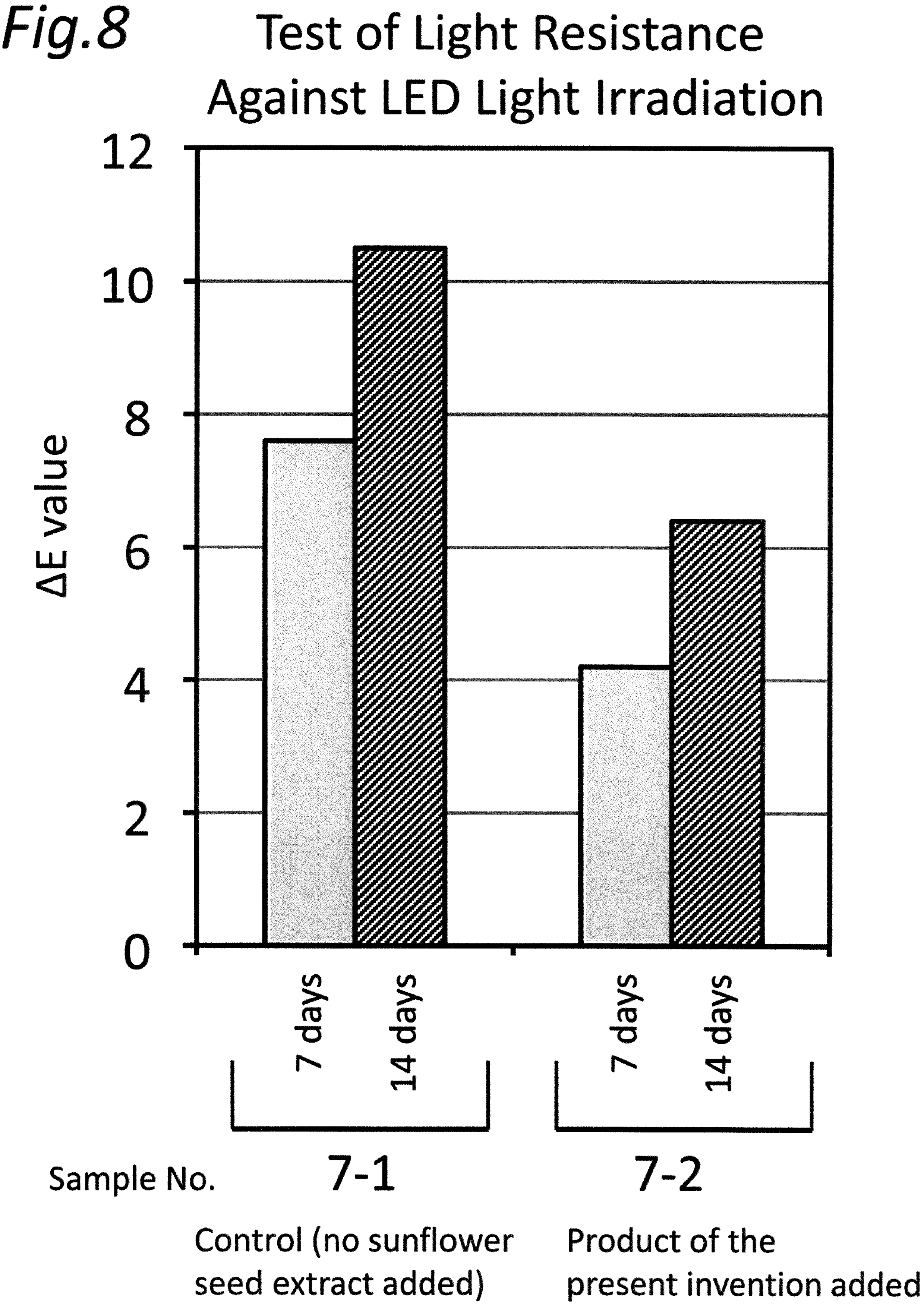
Test of Light Resistance
Against LED Light Irradiation
12
10
8
6
4
2
0
ΔE value
7 days
14 days
7 days
14 days
Sample No.
7-1
7-2
Control (no sunflower seed extract added)
Product of the present invention added

*Fig.9*

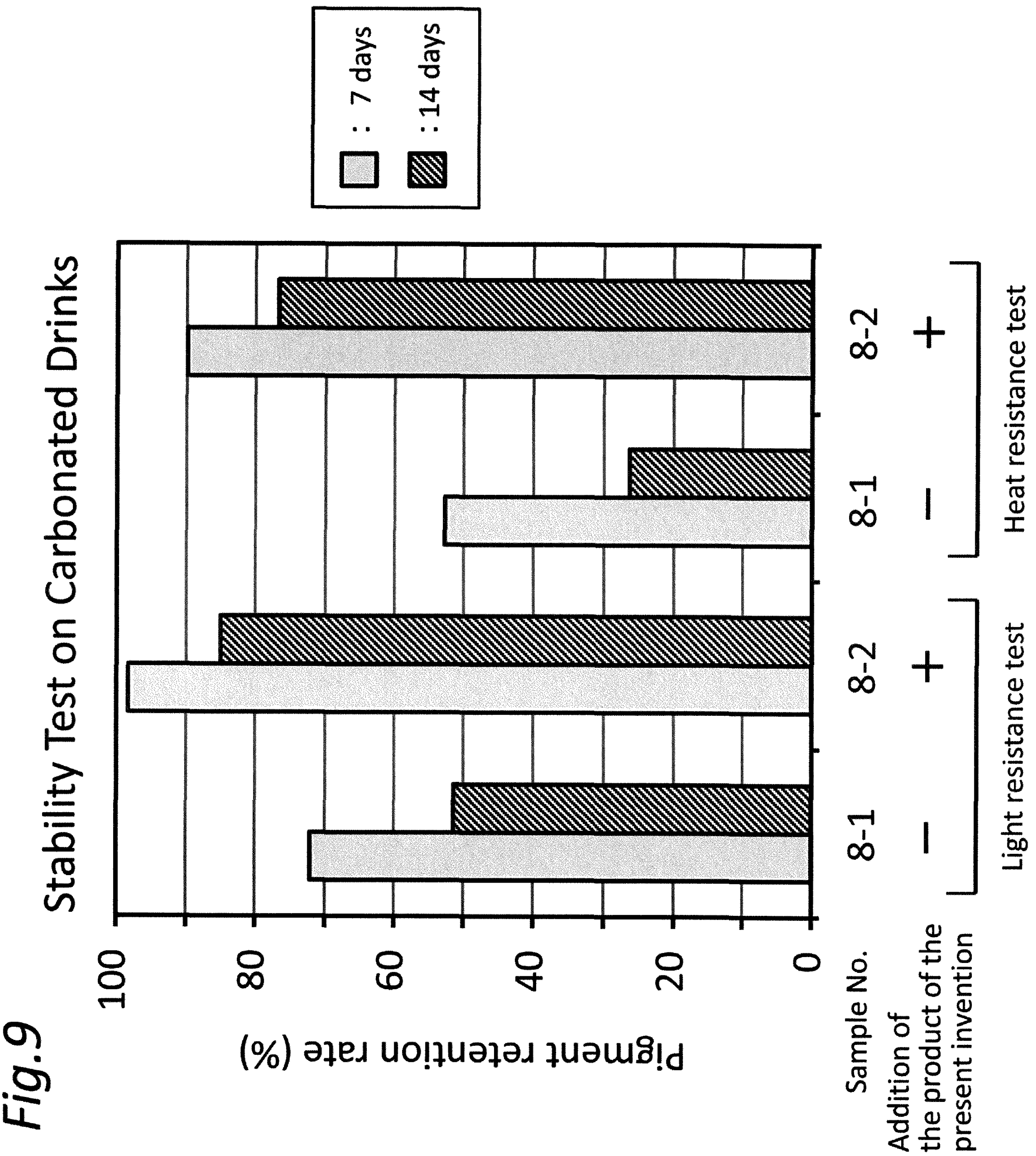
Stability Test on Carbonated Drinks
Pigment retention rate (%)
100
80
60
40
20
0
: 7 days
: 14 days
Sample No.
8-1
8-2
8-1
8-2
Addition of the product of the present invention
−
+
−
+
Light resistance test
Heat resistance test

Fig.10

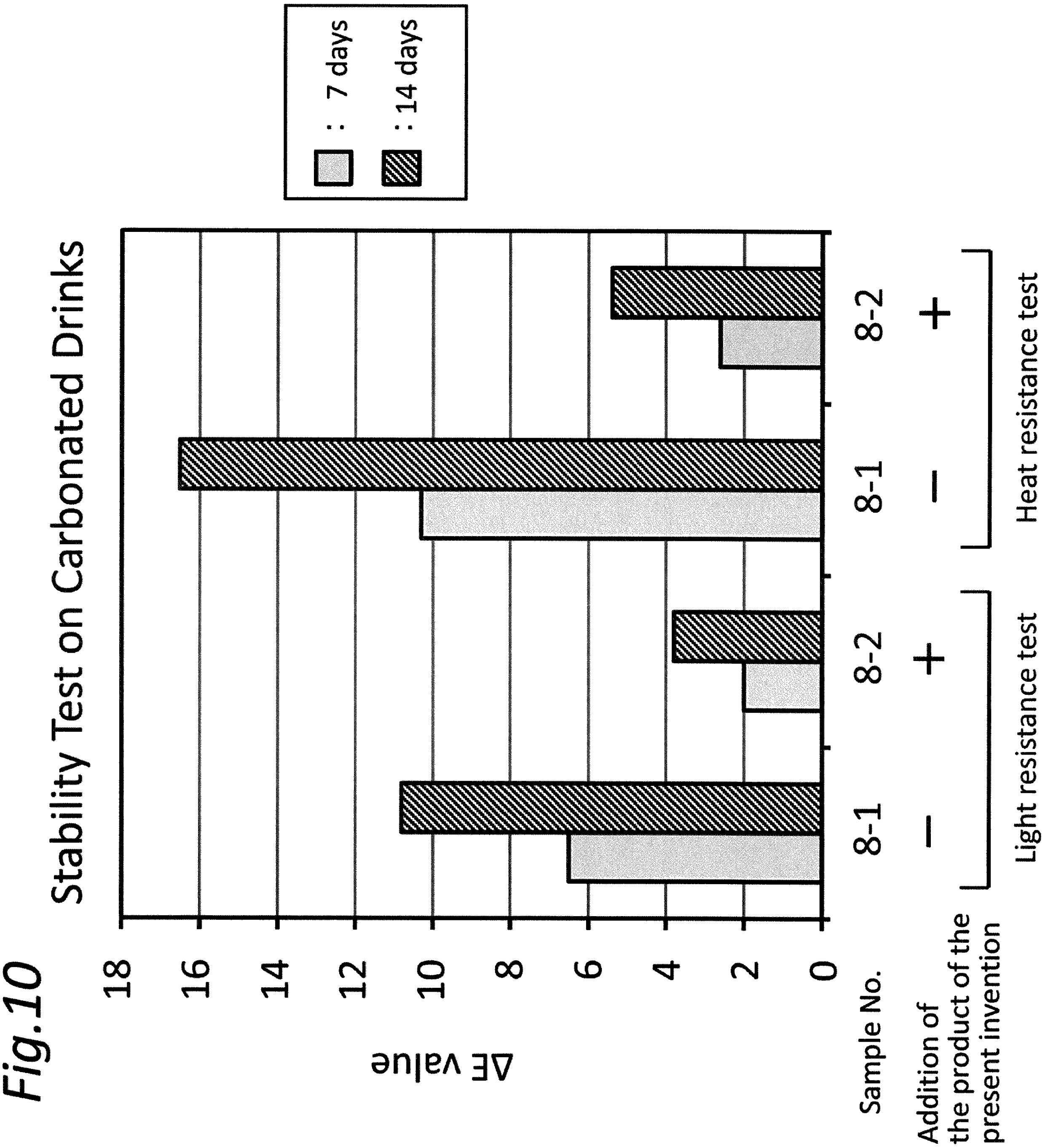
Stability Test on Carbonated Drinks
ΔE value
18
16
14
12
10
8
6
4
2
0
: 7 days
: 14 days
Sample No.
8-1
8-2
8-1
8-2
Addition of the product of the present invention
−
+
−
+
Light resistance test
Heat resistance test

*Fig.11*

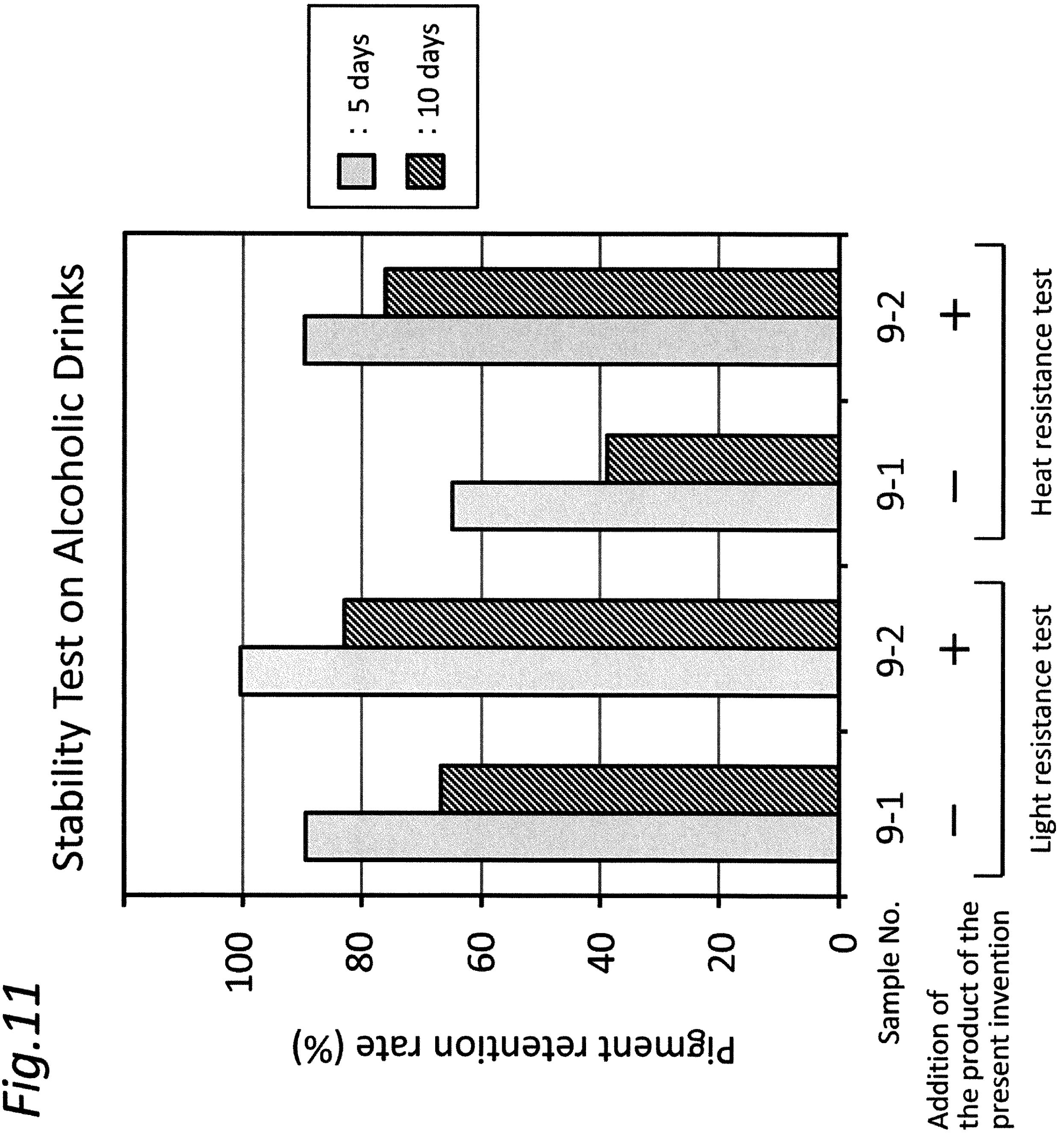
Stability Test on Alcoholic Drinks
Pigment retention rate (%)
100
80
60
40
20
0
: 5 days
: 10 days
Sample No.
9-1
9-2
9-1
9-2
Addition of the product of the present invention
−
+
−
+
Light resistance test
Heat resistance test

*Fig.12*

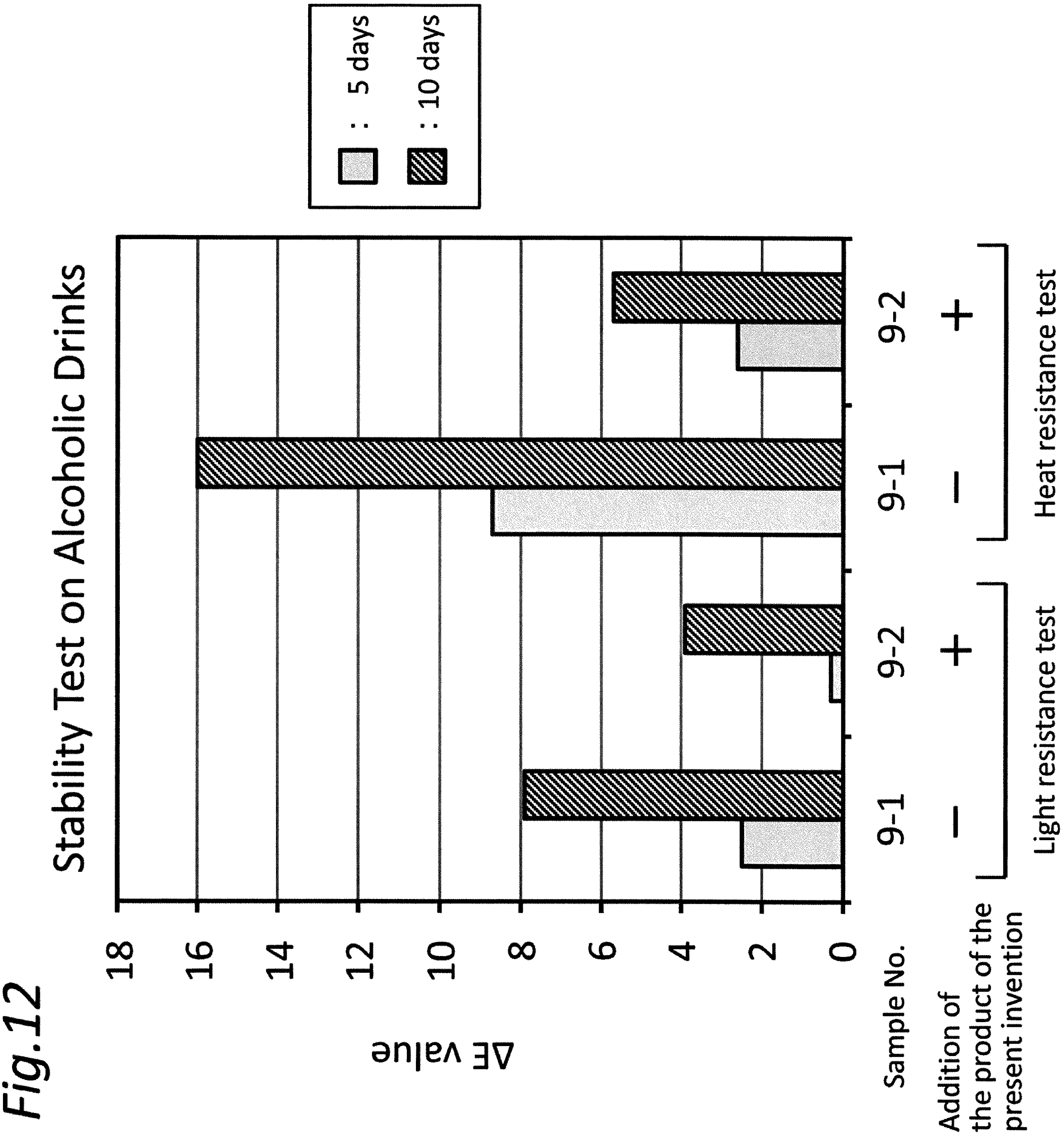
Stability Test on Alcoholic Drinks
ΔE value
18
16
14
12
10
8
6
4
2
0
: 5 days
: 10 days
Sample No.
9-1
9-2
9-1
9-2
Addition of the product of the present invention
−
+
−
+
Light resistance test
Heat resistance test

*Fig.13*

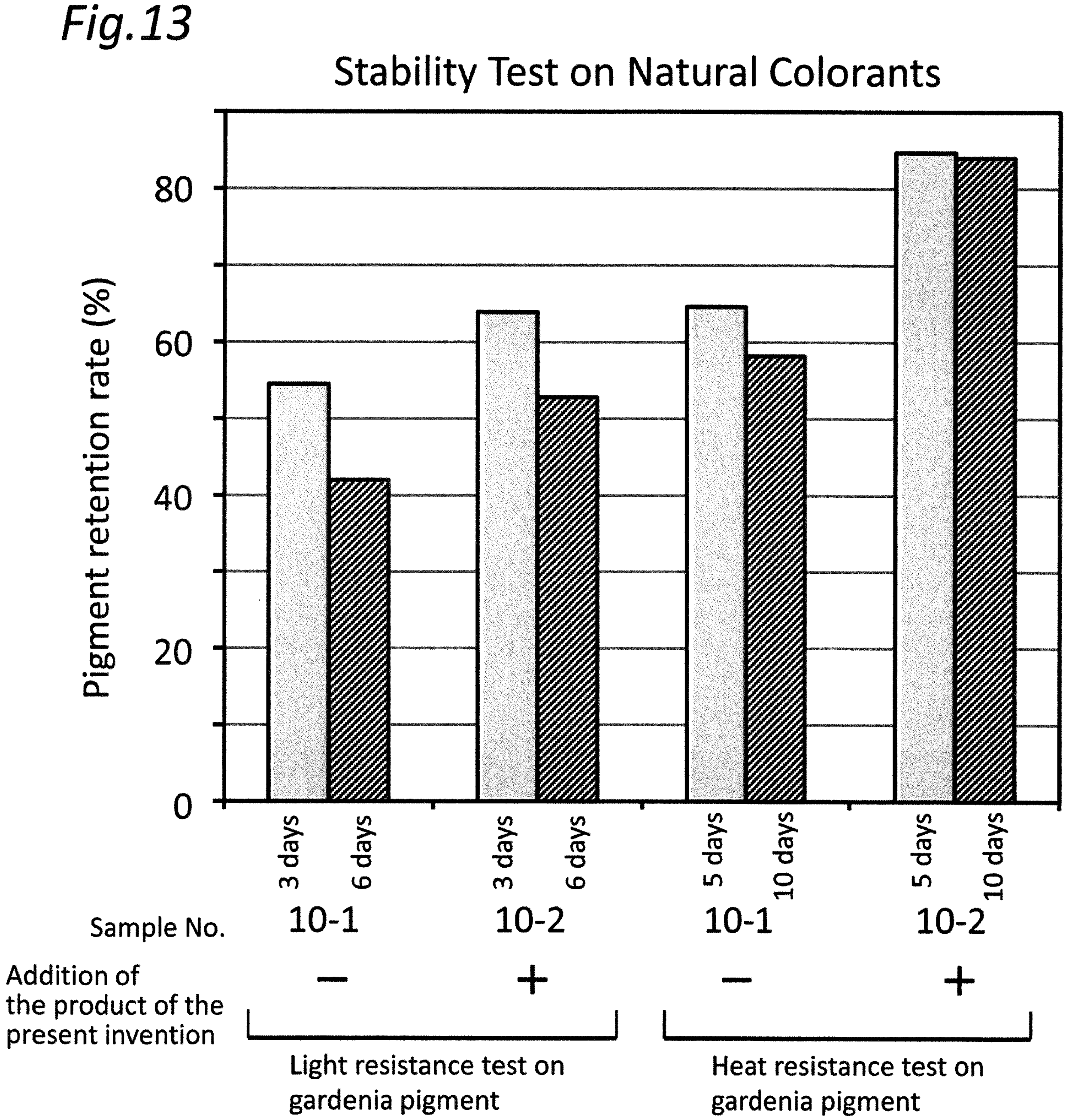
Stability Test on Natural Colorants
Pigment retention rate (%)
0
20
40
60
80
3 days
6 days
3 days
6 days
5 days
10 days
5 days
10 days
Sample No.
10-1
10-2
10-1
10-2
Addition of the product of the present invention
−
+
−
+
Light resistance test on gardenia pigment
Heat resistance test on gardenia pigment

*Fig.14*

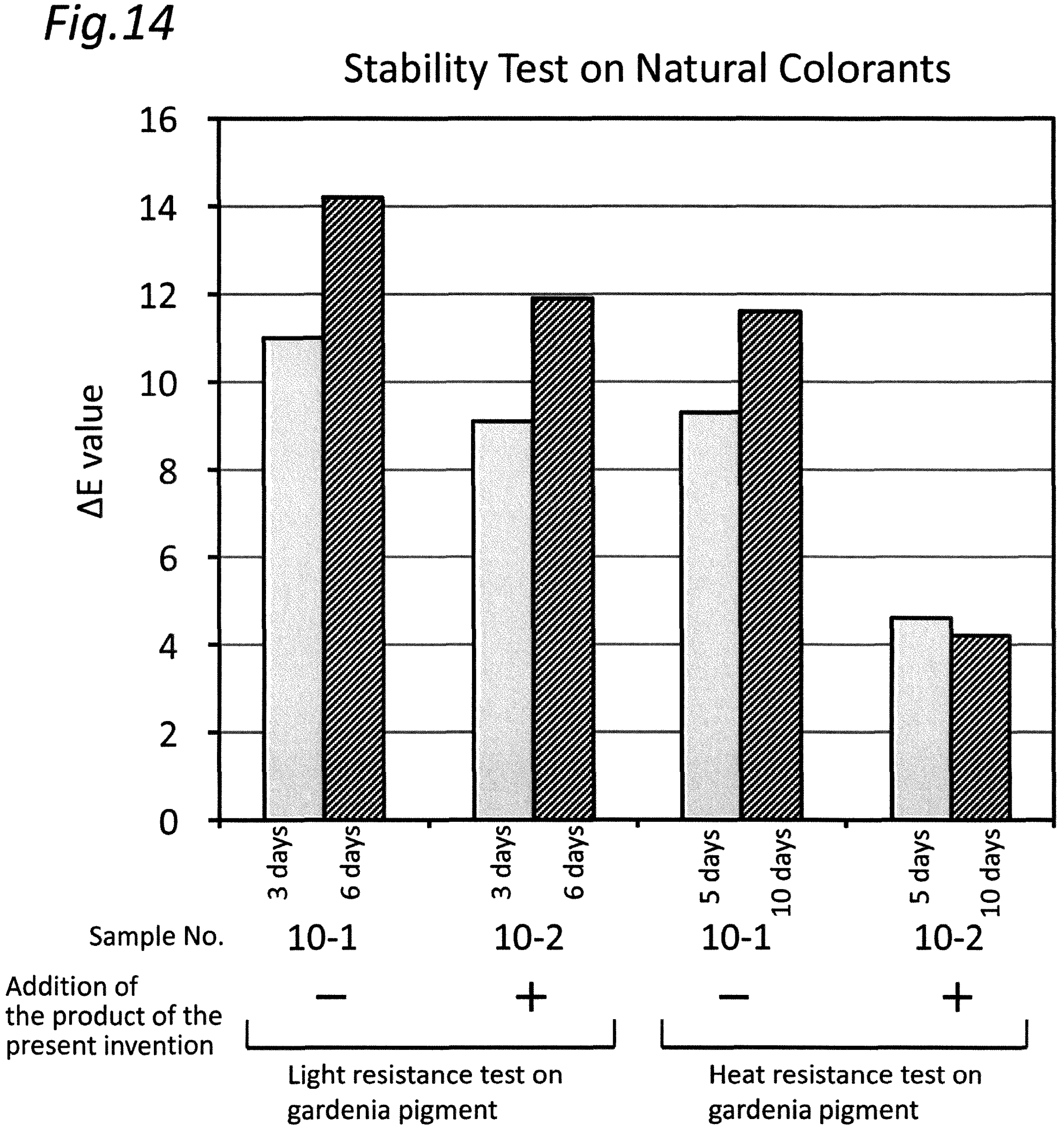
Stability Test on Natural Colorants
16
14
12
10
8
6
4
2
0
ΔE value
3 days
6 days
3 days
6 days
5 days
10 days
5 days
10 days
Sample No.
10-1
10-2
10-1
10-2
Addition of the product of the present invention
−
+
−
+
Light resistance test on gardenia pigment
Heat resistance test on gardenia pigment

*Fig.15*

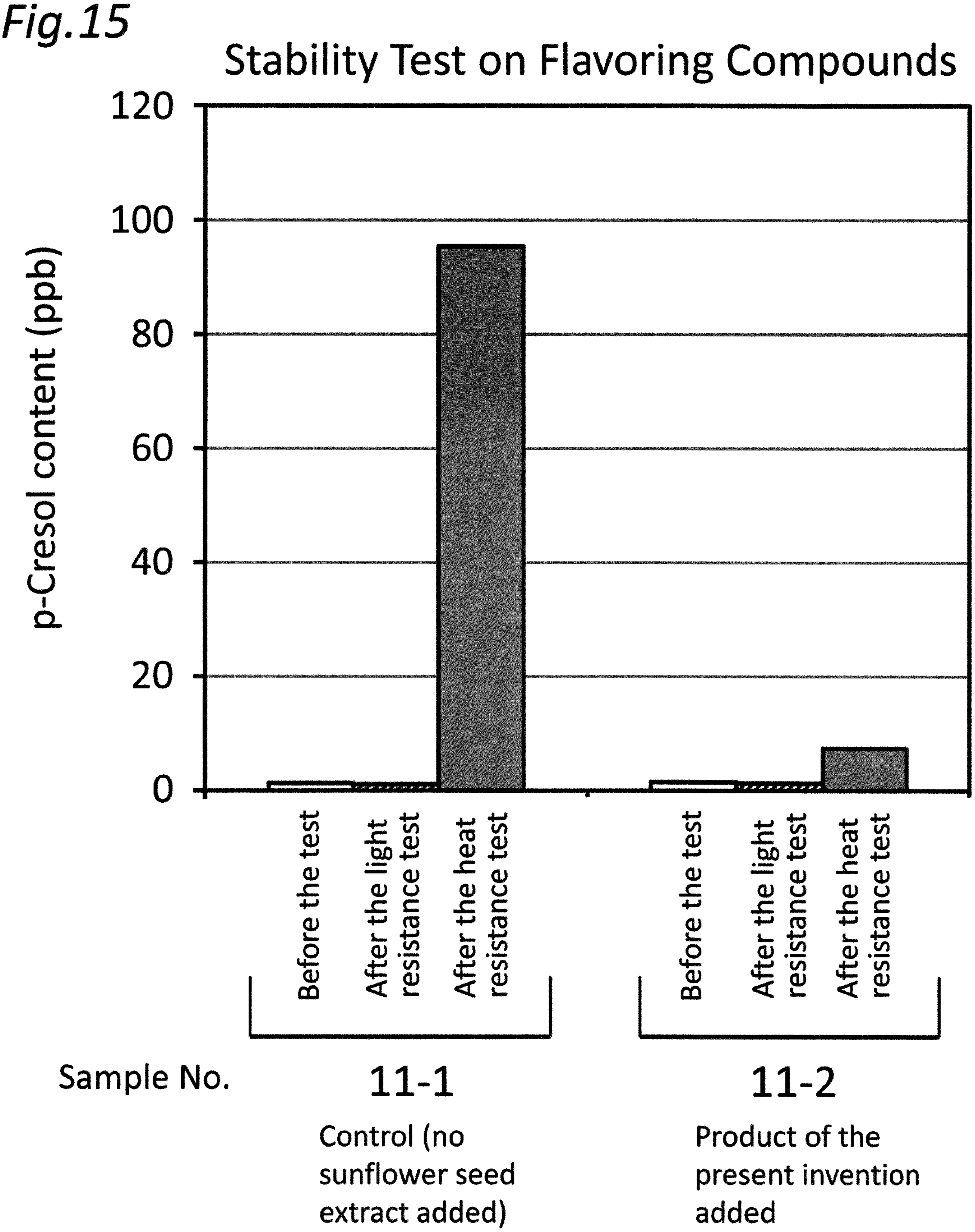
Stability Test on Flavoring Compounds
p-Cresol content (ppb)
120
100
80
60
40
20
0
Before the test
After the light resistance test
After the heat resistance test
Before the test
After the light resistance test
After the heat resistance test
Sample No.
11-1
Control (no sunflower seed extract added)
11-2
Product of the present invention added

*Fig.16*

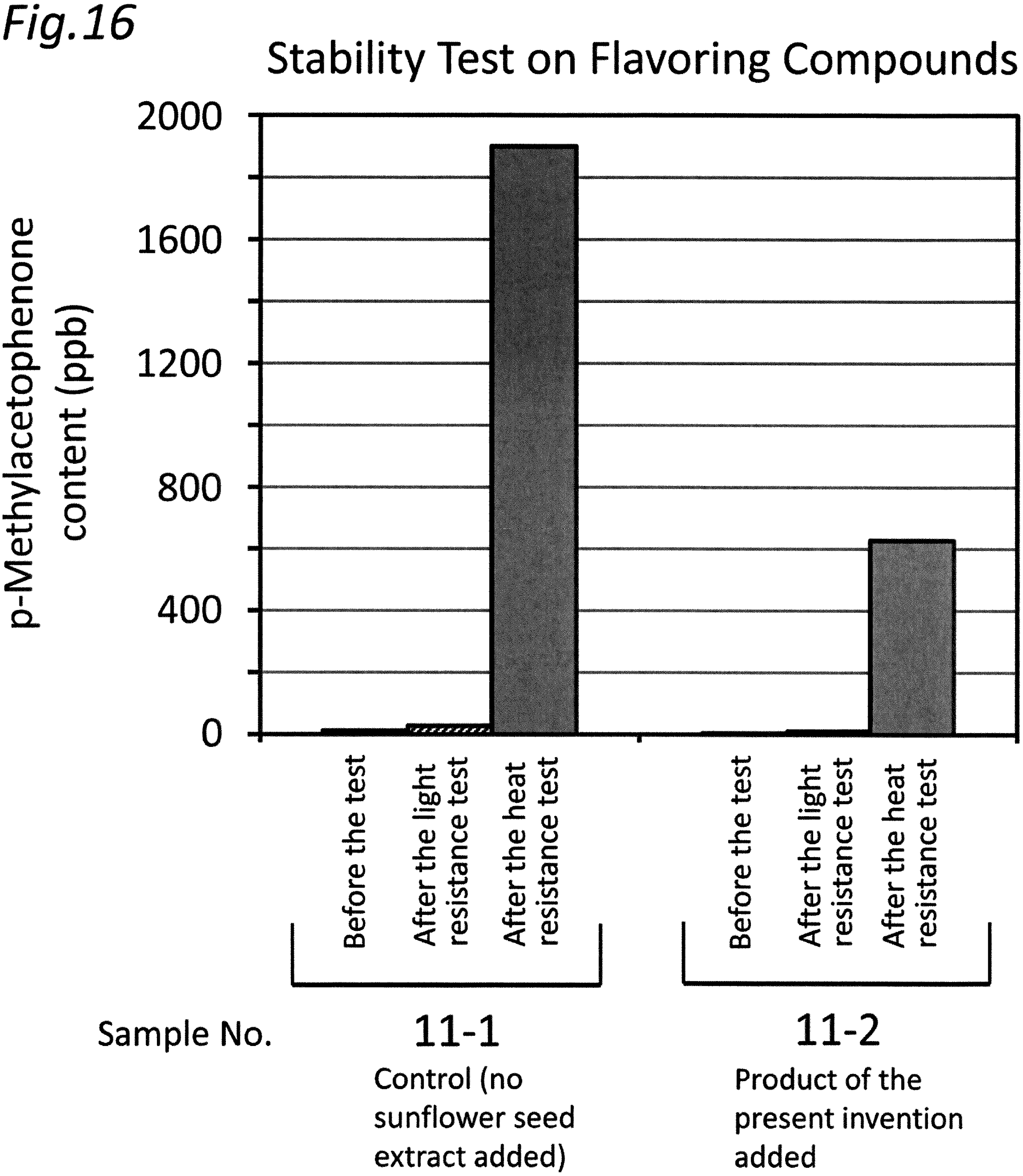
Stability Test on Flavoring Compounds
p-Methylacetophenone content (ppb)
2000
1600
1200
800
400
0
Before the test
After the light resistance test
After the heat resistance test
Before the test
After the light resistance test
After the heat resistance test
Sample No.
11-1
Control (no sunflower seed extract added)
11-2
Product of the present invention added

*Fig.17*

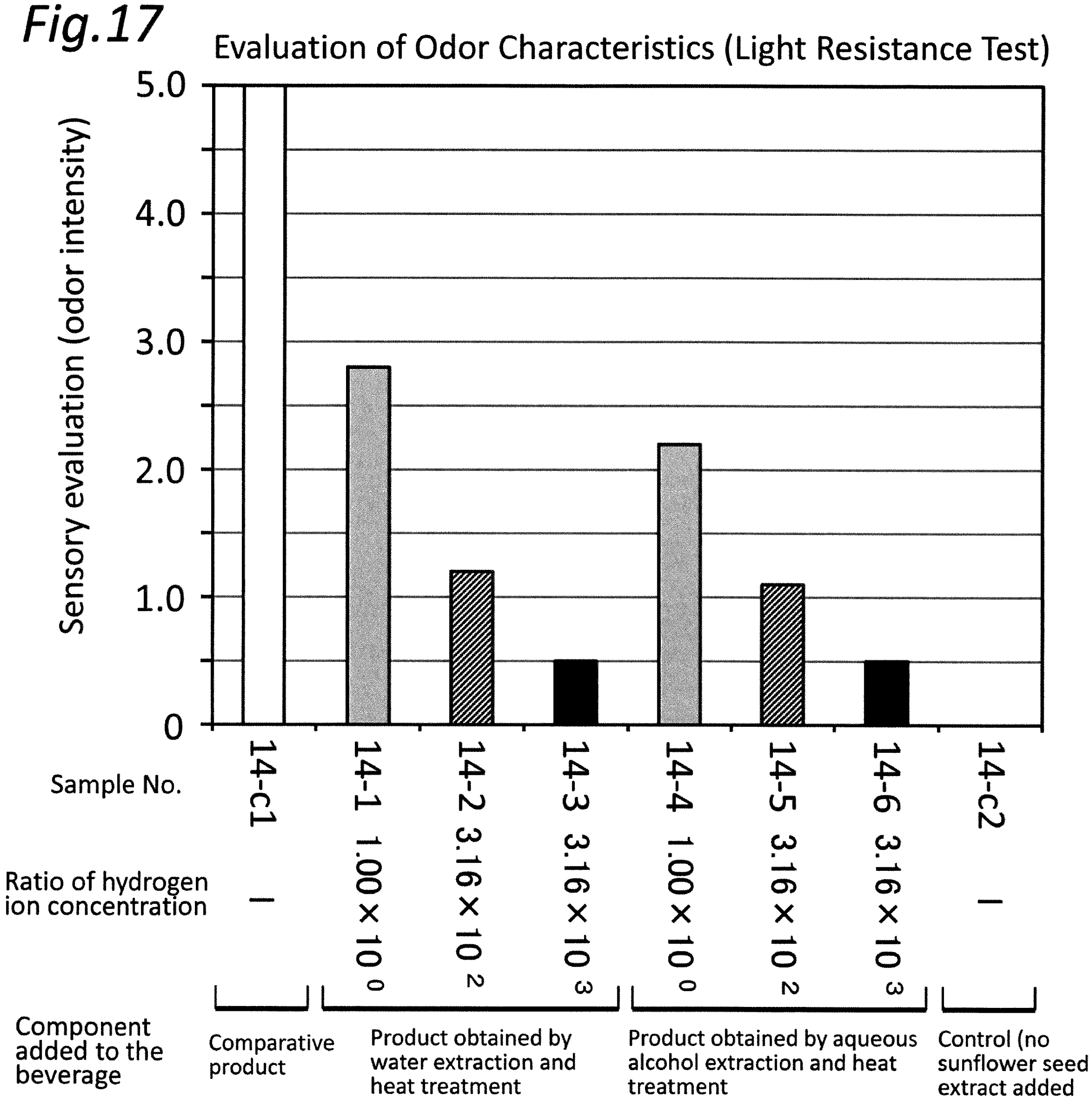
Evaluation of Odor Characteristics (Light Resistance Test)
Sensory evaluation (odor intensity)
5.0
4.0
3.0
2.0
1.0
0
Sample No.
Ratio of hydrogen ion concentration
14-c1 —
14-1 1.00 × 10 0
14-2 3.16 × 10 2
14-3 3.16 × 10 3
14-4 1.00 × 10 0
14-5 3.16 × 10 2
14-6 3.16 × 10 3
14-c2 —
Component added to the beverage
Comparative product
Product obtained by water extraction and heat treatment
Product obtained by aqueous alcohol extraction and heat treatment
Control (no sunflower seed extract added

*Fig.18*

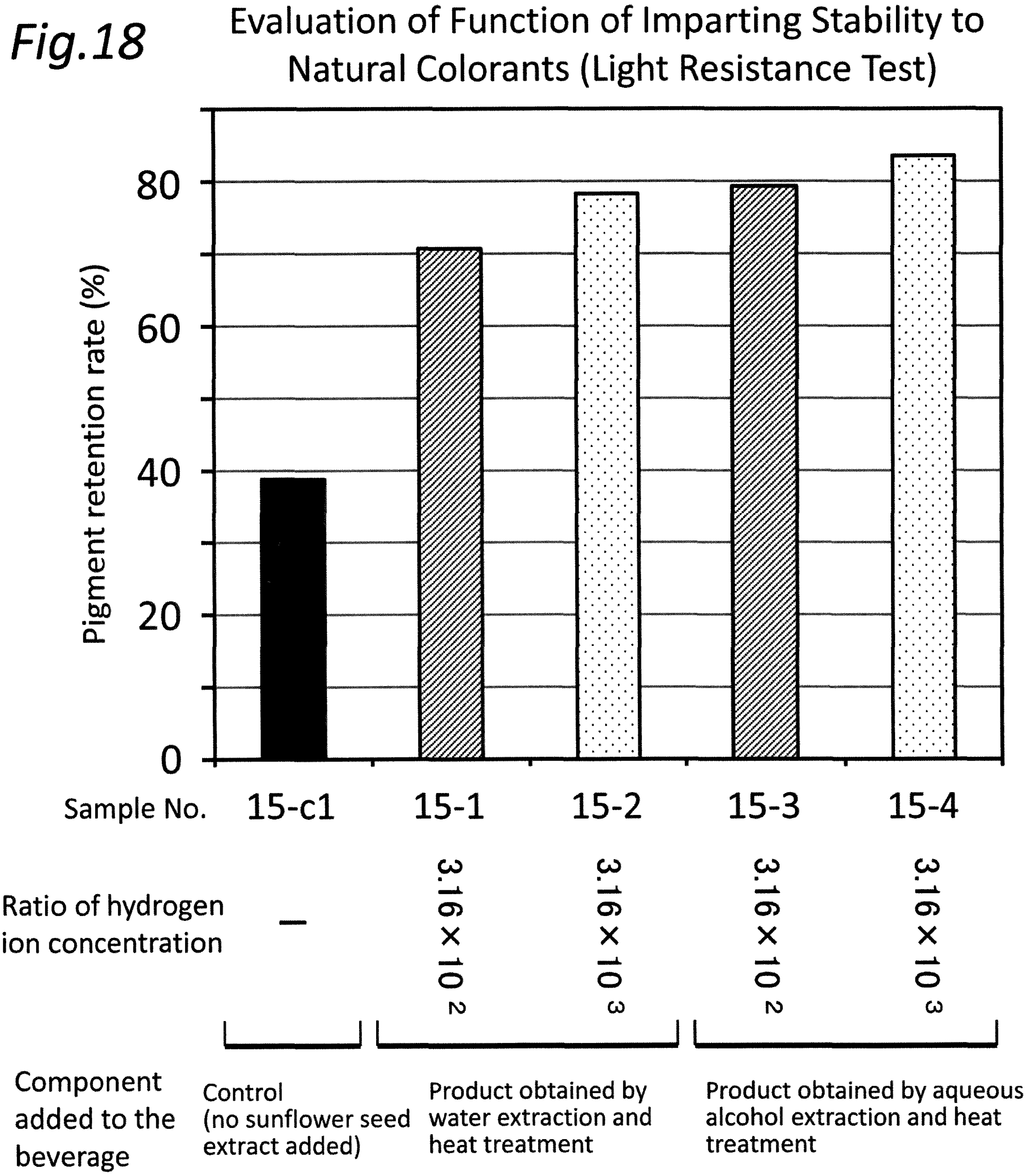
Evaluation of Function of Imparting Stability to Natural Colorants (Light Resistance Test)
Pigment retention rate (%)
80
60
40
20
0
Sample No.
15-c1
15-1
15-2
15-3
15-4
Ratio of hydrogen ion concentration
–
3.16×10 2
3.16×10 3
3.16×10 2
3.16×10 3
Component added to the beverage
Control (no sunflower seed extract added)
Product obtained by water extraction and heat treatment
Product obtained by aqueous alcohol extraction and heat treatment

**INTERNATIONAL SEARCH REPORT**

International application No.
PCT/JP2016/077559

A. CLASSIFICATION OF SUBJECT MATTER
*A23L33/105*(2016.01)i, *A23L5/41*(2016.01)i, *A61K8/97*(2006.01)i, *A61K36/28*(2006.01)i, *A61Q13/00*(2006.01)i, *C09B67/00*(2006.01)i, *C11B5/00*(2006.01)i, *C11B9/00*(2006.01)i, *C11B11/00*(2006.01)i, *C12N5/04*(2006.01)n

According to International Patent Classification (IPC) or to both national classification and IPC

B. FIELDS SEARCHED

Minimum documentation searched (classification system followed by classification symbols)
A23L33/105, A23L5/41, A61K8/97, A61K36/28, A61Q13/00, C09B67/00, C11B5/00, C11B9/00, C11B11/00, C12N5/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
CAplus/FSTA/FROSTI/WPIDS(STN), JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X<br><br><br>Y<br>A | JP 05-137536 A (Dainippon Ink and Chemicals, Inc.),<br>01 June 1993 (01.06.1993),<br>claims 1, 2; paragraphs [0001], [0007];<br>referential example 2<br>(Family: none) | 1-8,10,11,<br>13,14,17,18,<br>20<br>13-20<br>9,12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 November 2016 (25.11.16) | 06 December 2016 (06.12.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | <br><br>Telephone No. |

Form PCT/ISA/210 (second sheet) (January 2015)

**INTERNATIONAL SEARCH REPORT**

International application No.
PCT/JP2016/077559

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br>A | WO 2014/060244 A1 (Indena S.p.A.), 24 April 2014 (24.04.2014), claim 1; page 5, line 12 to page 6, line 11; examples<br>& JP 2015-535216 A<br>claim 1; paragraphs [0021] to [0025]<br>& US 2015/0258155 A1 & EP 2908663 A<br>& IT MI20121749 A & AU 2013331918 A<br>& CA 2888305 A & CN 104717892 A<br>& KR 10-2015-0068959 A & IL 238272 A<br>& HK 1211439 A & IT MI20121749 A1 | 1-7,11,17,20<br>13-20<br>8-10,12 |
| Y | JP 07-132073 A (San-Ei Gen F.F.I., Inc.), 23 May 1995 (23.05.1995), claims 1 to 5; paragraph [0002]<br>(Family: none) | 15-17,19,20 |
| A | JP 2000-063827 A (Dainippon Ink and Chemicals, Inc.), 29 February 2000 (29.02.2000), paragraphs [0019] to [0023]<br>(Family: none) | 1-20 |
| A | ZHU Mengmeng et al., Determination of Volatile Compounds of Chinese Traditional Aromatic Sunflower Seeds (Helianthus annulus L.), International Journal of Food Engineering, 2015.02, Vol.11, No.1, pp.85-95 | 1-20 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2015183988 A
- JP 3247990 B
- JP 2015051932 A
- JP 2015091223 A
- JP 4185317 B


### Non-patent literature cited in the description


- Specifications and Standards for Food Additives. Ministry of Health, Labour and Welfare of Japan